(19) Europäisches Patentamt
European Patent Office
Office européen des brevets

(11) EP 2 901 973 B1

# (12) EUROPEAN PATENT SPECIFICATION

(45) Date of publication and mention of the grant of the patent:
**18.04.2018 Bulletin 2018/16**

(21) Application number: **13840239.1**

(22) Date of filing: **12.09.2013**

(51) Int Cl.:
***A61F 13/15*** (2006.01) ***A61F 13/511*** (2006.01)

(86) International application number:
**PCT/JP2013/074720**

(87) International publication number:
**WO 2014/050595 (03.04.2014 Gazette 2014/14)**

## (54) ABSORBENT ARTICLE

SAUGFÄHIGER ARTIKEL

ARTICLE ABSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2012 JP 2012218871**

(43) Date of publication of application:
**05.08.2015 Bulletin 2015/32**

(73) Proprietor: **Unicharm Corporation**
**Ehime 799-0111 (JP)**

(72) Inventors:
- **NODA, Yuki**
**Kanonji-shi**
**Kagawa 769-1602 (JP)**
- **TAMURA, Tatsuya**
**Kanonji-shi**
**Kagawa 769-1602 (JP)**
- **HASHINO, Akira**
**Kanonji-shi**
**Kagawa 769-1602 (JP)**

(74) Representative: **Dolleymores**
**9 Rickmansworth Road**
**Watford, Hertfordshire WD18 0JU (GB)**

(56) References cited:
**WO-A1-01/24754**
**WO-A1-2010/128906**
**JP-A- 2004 141 619**
**JP-A- 2007 190 170**
**JP-A- 2008 125 605**
**JP-A- 2011 510 801**
**US-A- 5 662 633**
**US-A- 5 733 274**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 901 973 B1

## Description

### Technical Field

The present invention relates to an absorbent article, such as a sanitary napkin, panty liner, incontinence pad or incontinence liner.

### Background Art

The known prior art includes absorbent articles provided with a diffusion sheet over the entire surface of the skin side of the absorbent body, for absorption of menstrual blood excreted into the top sheet over a wide range of the absorbent body (PTLs 1 and 2, for example). In this absorbent article, menstrual blood excreted into the top sheet diffuses in the planar direction by the diffusion sheet, migrating smoothly into the absorbent body.

### Citation List

### Patent Literature

PTL 1 Japanese Patent Publication No. 4266685
PTL 2 Japanese Patent Publication No. 2810772

### Summary of Invention

### Technical Problem

When the wearer exchanges the absorbent article, absorbed menstrual blood can sometimes be seen in the absorbent article. When this occurs, and menstrual blood absorbed into the absorbent article diffuses non-uniformly in the planar direction, the wearer may feel uneasy regarding the performance of the absorbent article. In particular, in the case of an absorbent article provided with a diffusion sheet over the entire surface of the skin side of the absorbent body, diffusion of menstrual blood is high in the planar direction, and this can increase the non-uniformity with which menstrual blood absorbed into the absorbent article diffuses in the planar direction.

It is an object of the present invention to provide an absorbent article that can give the wearer a feeling of assurance regarding the performance of the absorbent article, when menstrual blood absorbed into the absorbent article has become visible when the absorbent article is exchanged.

### Solution to Problem

In order to solve the aforementioned problems, the invention employs the following construction.

(1) The absorbent article according to the invention of claim 1 comprises a liquid-permeable top sheet provided on the skin side, a liquid-impermeable back sheet provided on the clothing side, a liquid-retaining absorbent body provided between the top sheet and the back sheet, a first sheet provided between the top sheet and the absorbent body and provided in the region in which the absorbent body is provided, and a second sheet provided between the first sheet and the absorbent body, wherein the first sheet has a liquid-permeable as well as a liquid-retaining property, the second sheet has a liquid-permeable property but no liquid-retaining property, and conceals the redness of menstrual blood absorbed into the absorbent body, the absorbent article has a domed section that protrudes in a thickness direction of the absorbent article, the domed section comprises a cushion section between the first sheet and the second sheet, the cushion section has a maximum thickness of 3 to 30 mm, the cushion section contains multiple fibers, and the intersections between the multiple fibers are heat-fused, and the domed section contains a blood slipping agent having a kinematic viscosity of 0.01 to 80 $mm^2/s$ at 40°C, a water holding percentage of 0.01 to 4.0 mass%, and a weight-average molecular weight of less than 1,000.
(2) The invention of claim 2 is an absorbent article according to claim 1 wherein the region in which the first sheet is provided includes the excretory opening contact region.
(3) The invention of claim 3 is an absorbent article according to claim 1 or 2 wherein the first sheet contains cellulose-based fibers at 50 mass% or greater.
(4) The invention of claim 4 is an absorbent article according to any one of claims 1 to 3, wherein the second sheet

contains thermoplastic chemical fibers at 50 mass% or greater.

(5) The invention of claim 5 is an absorbent article according to any one of claims 1 to 4, wherein the ratio of the area of the first sheet with respect to the area of the absorbent body is 10% to 90%.

(6) The invention of claim 6 is an absorbent article according to any one of claims 1 to 5, wherein the region in which the second sheet is provided includes the region in which the first sheet is provided, the ratio of the area of the second sheet with respect to the area of the first sheet is 110% or greater, and the ratio of the area of the second sheet with respect to the area of the absorbent body is 150% or less.

(7) The invention of claim 7 is an absorbent article according to claim 6 wherein the blood slipping agent also has an IOB of 0.00 to 0.60.

(8) The invention of claim 8 is an absorbent article according to claim 6 or 7 wherein the blood slipping agent is selected from the group consisting of the following items (i)-(iii), and any combination thereof:

(i) a hydrocarbon;
(ii) a compound having (ii-1) a hydrocarbon moiety, and (ii-2) one or more, same or different groups selected from the group consisting of carbonyl group (-CO-) and oxy group (-0-) inserted between a C-C single bond of the hydrocarbon moiety; and
(iii) a compound having (iii-1) a hydrocarbon moiety, (iii-2) one or more, same or different groups selected from the group consisting of carbonyl group (-CO-) and oxy group (-0-) inserted between a C-C single bond of the hydrocarbon moiety, and (iii-3) one or more, same or different groups selected from the group consisting of carboxyl group (-COOH) and hydroxyl group (-OH) substituting a hydrogen of the hydrocarbon moiety;

with the proviso that when 2 or more oxy groups are inserted in the compound of (ii) or (iii), the oxy groups are not adjacent.

(9) The invention of claim 9 is an absorbent article according to any one of claims 6 to 8, wherein the blood slipping agent is selected from the group consisting of the following items (i')-(iii'), and any combination thereof:

(i') a hydrocarbon;
(ii') a compound having (ii'-1) a hydrocarbon moiety, and (ii'-2) one or more, same or different bonds selected from the group consisting of carbonyl bond (-CO-), ester bond (-C00-), carbonate bond (-OCOO-), and ether bond (-0-) inserted between a C-C single bond of the hydrocarbon moiety; and
(iii') a compound having (iii'-1) a hydrocarbon moiety, (iii'-2) one or more, same or different bonds selected from the group consisting of carbonyl bond (-CO-), ester bond (-COO-), carbonate bond (-OCOO-), and ether bond (-0-) inserted between a C-C single bond of the hydrocarbon moiety, and (iii'-3) one or more, same or different groups selected from the group consisting of carboxyl group (-COOH) and hydroxyl group (-OH) substituting a hydrogen on the hydrocarbon moiety;

with the proviso that when 2 or more same or different bonds are inserted in the compound of (ii') or (iii'), the bonds are not adjacent.

(10) The invention of claim 10 is an absorbent article according to any one of claims 6 to 9, wherein the blood slipping agent is selected from the group consisting of the following items (A)-(F), and any combination thereof:

(A) an ester of (A1) a compound having a chain hydrocarbon moiety and 2-4 hydroxyl groups substituting hydrogens on the chain hydrocarbon moiety, and (A2) a compound having a chain hydrocarbon moiety and 1 carboxyl group substituting a hydrogen on the chain hydrocarbon moiety;
(B) an ether of (B1) a compound having a chain hydrocarbon moiety and 2-4 hydroxyl groups substituting hydrogens on the chain hydrocarbon moiety and (B2) a compound having a chain hydrocarbon moiety and 1 hydroxyl group substituting a hydrogen on the chain hydrocarbon moiety;
(C) an ester of (C1) a carboxylic acid, hydroxy acid, alkoxy acid or oxoacid comprising a chain hydrocarbon moiety and 2-4 carboxyl groups substituting hydrogens on the chain hydrocarbon moiety and (C2) a compound having a chain hydrocarbon moiety and 1 hydroxyl group substituting a hydrogen on the chain hydrocarbon moiety;
(D) a compound having a chain hydrocarbon moiety and one bond selected from the group consisting of an ether bond (-0-), carbonyl bond (-CO-), ester bond (-COO-) and carbonate bond (-OCOO-) inserted between a C-C single bond of the chain hydrocarbon moiety;
(E) a polyoxy $C_3$-$C_6$ alkylene glycol, or alkyl ester or alkyl ether thereof; and
(F) a chain hydrocarbon.

(11) The invention of claim 11 is an absorbent article according to any one of claims 6 to 10, wherein the blood

slipping agent is selected from the group consisting of ($a_1$) an ester of a chain hydrocarbon tetraol and at least one fatty acid, ($a_2$) an ester of a chain hydrocarbon triol and at least one fatty acid, ($a_3$) an ester of a chain hydrocarbon diol and at least one fatty acid, ($b_1$) an ether of a chain hydrocarbon tetraol and at least one aliphatic monohydric alcohol, ($b_2$) an ether of a chain hydrocarbon triol and at least one aliphatic monohydric alcohol, ($b_3$) an ether of a chain hydrocarbon diol and at least one aliphatic monohydric alcohol, ($c_1$) an ester of a chain hydrocarbon tetracarboxylic acid, hydroxy acid, alkoxy acid or oxoacid with 4 carboxyl groups, and at least one aliphatic monohydric alcohol, ($c_2$) an ester of a chain hydrocarbon tricarboxylic acid, hydroxy acid, alkoxy acid or oxoacid with 3 carboxyl groups, and at least one aliphatic monohydric alcohol, ($c_3$) an ester of a chain hydrocarbon dicarboxylic acid, hydroxy acid, alkoxy acid or oxoacid with 2 carboxyl groups, and at least one aliphatic monohydric alcohol, ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, ($d_4$) a dialkyl carbonate, ($e_1$) a polyoxy $C_3$-$C_6$ alkylene glycol, ($e_2$) an ester of a polyoxy $C_3$-$C_6$ alkylene glycol and at least one fatty acid, ($e_3$) an ether of a polyoxy $C_3$-$C_6$ alkylene glycol and at least one aliphatic monohydric alcohol, and ($f_1$) a chain alkane, and any combination thereof.

(12) The invention of claim 12 is an absorbent article according to any one of claims 6 to 11, wherein the blood slipping agent has a vapor pressure of 0.00 to 0.01 Pa at 1 atmosphere, 40°C.

Advantageous Effects of Invention

According to the invention it is possible to give the wearer a feeling of assurance regarding the performance of the absorbent article, when menstrual blood absorbed into the absorbent article has become visible when the absorbent article is exchanged.

Brief Description of Drawing

Fig. 1 is a plan view of an absorbent article according to a reference example of the invention.
Fig. 2 is a schematic cross-sectional view showing a cross-section of Fig. 1 along cross-section A-A.
Fig. 3 is a diagram illustrating the state where the absorbent article of the reference example of the invention has absorbed menstrual blood in the top sheet, diffusion sheet, cover sheet and absorbent body, after menstrual blood excreted from the wearer has been absorbed.
Fig. 4 is a plan view of an absorbent article according to the reference example of the invention, after menstrual blood excreted by the wearer has been absorbed.
Fig. 5 is a perspective view showing an absorbent article according to an embodiment of the invention.
Fig. 6 is a plan view showing an absorbent article according to the embodiment of the invention.
Fig. 7 is a schematic cross-sectional view of Fig. 6 along cross-section B-B.
Fig. 8 is an electron micrograph of the skin contact surface of a top sheet in a sanitary napkin wherein the top sheet comprises tri-C2L oil fatty acid glycerides.
Fig. 9 is a pair of photomicrographs of menstrual blood containing and not containing a blood slipping agent.
Fig. 10 is a diagram illustrating a method of measuring surface tension.

Description of Embodiments

- Reference example -

An absorbent article according to a reference example of the invention will now be explained with reference to the accompanying drawings. However, the invention is not limited to the examples depicted in the drawings. The absorbent article according to the reference example of the invention is a sanitary napkin.

Fig. 1 is plan view showing an absorbent article according to a reference example of the invention, and Fig. 2 is a schematic cross-sectional view showing a cross-section of Fig. 1 along cross-section A-A. The absorbent article 1 comprises a liquid-permeable top sheet 2 provided on the skin side, a liquid-impermeable back sheet 3 provided on the clothing side, a liquid-retaining absorbent body 4 provided between the top sheet 2 and the back sheet 3, a diffusion sheet 5 provided between the top sheet 2 and the absorbent body 4, and a cover sheet 6 provided between the diffusion sheet 5 and the absorbent body 4.

The absorbent article 1 further comprises a pair of side sheets 7 provided over both sides in the widthwise direction of the top sheet 2. The absorbent article 1 further has a body section 11 and a pair of wing sections 12 extending from the body section 11 in the widthwise direction. The wing sections 12 are each constructed from a side sheet 7 and a back sheet 3. Pressure-sensitive adhesive sections 8 are provided on the clothing sides of the body section 11 and

the wing section 12.

In Fig. 1, the X direction is the widthwise direction of the absorbent article 1, and the Y direction is the lengthwise direction of the absorbent article.

The shape of the body section 11 is not particularly restricted so long as it is a shape suited to the female body and the shape of shorts, such as roughly rectangular, roughly elliptical or roughly hourglass-shaped. The dimensions of extension in the lengthwise direction of the outer shape of the body section 11 are preferably 100 to 500 mm and more preferably 150 to 350 mm. Also, the dimensions of extension in the widthwise direction of the outer shape of the body section 8 are preferably 30 to 200 mm and more preferably 40 to 180 mm.

The top sheet 2 causes menstrual blood discharged by the wearer to migrate into the diffusion sheet 5 or cover sheet 6 provided under it. The top sheet 2 holds the absorbent body 4 in a manner with the absorbent body 4 held between it and the back sheet 3. All or a portion of the top sheet 2 is liquid-permeable, and the liquid-permeable areas of the top sheet 2 may be formed of a liquid-permeable nonwoven fabric or woven fabric, a resin film with a plurality of liquid-permeable holes formed therein, or a net-like sheet with a plurality of mesh holes.

The material used for the nonwoven fabric or woven fabric in the top sheet 2 may be either natural fibers or chemical fibers. Examples of natural fibers include cellulose, such as ground pulp and cotton. Examples of chemical fibers include regenerated cellulose, such as rayon and fibril rayon, semi-synthetic cellulose, such as acetate and triacetate, thermoplastic hydrophobic chemical fibers, and hydrophilicized thermoplastic hydrophobic chemical fibers. Thermoplastic hydrophobic chemical fibers include monofilaments of polyethylene (PE), polypropylene (PP) and polyethylene terephthalate (PET), fibers obtained by graft polymerization of PE and PP, and composite fibers with a core-sheath structure or the like.

Fabrication of a nonwoven fabric to be used in the top sheet 2 may be accomplished by web forming, with either a dry method (for example, a carding method, spunbond method, meltblown method or airlaid method) or wet method, or with a combination of a dry method and a wet method. The web bonding method for fabrication of a nonwoven fabric to be used in the top sheet 2 may be thermal bonding, needle punching, chemical bonding or the like, with no particular restriction to these methods. Spunlace formed into a sheet by a hydroentangling method may also be used in the top sheet 2. There may also be used for the top sheet 2 a nonwoven fabric having concavoconvexities on the skin side, such as a nonwoven fabric having heat-shrinkable fibers or the like for shrinking on the lower layer side to form concavoconvexities on the upper layer side, or a nonwoven fabric in which concavoconvexities are formed by applying air during web formation. Forming concavoconvexities on the skin side in this manner can reduce the contact area between the top sheet 2 and the skin.

As fibers in the nonwoven fabric for the top sheet 2 there may be used core-sheath type fibers wherein the melting point of the core component is higher than that of the sheath component, eccentric core-sheath type fibers, or side-by-side type composite fibers wherein the melting points of the left and right components differ. In addition, hollow type fibers or flat fibers, or irregularly shaped fibers, such as Y-shaped fibers or C-shaped fibers, solid crimped fibers, such as latent crimped or developed crimped fibers, or split fibers that have been split by a physical load, such as a water stream, heat or embossing, may be combined in a nonwoven fabric to be used for the top sheet 2.

In consideration of uptake of fluids and feel on the skin, the size of the fibers of the nonwoven fabric used for the top sheet 2 is preferably 1.1 to 8.8 dtex.

When hydrophobic synthetic fibers are used in the top sheet 2, in consideration of uptake of fluids and rewet-back by the top sheet 2, the hydrophobic synthetic fibers may be mixed with a hydrophilic agent, water-repellent agent or the like, or the hydrophobic synthetic fibers may be coated with a hydrophilic agent, water-repellent agent or the like. The hydrophobic synthetic fibers may also be imparted with hydrophilicity by corona treatment or plasma treatment.

When a resin film or net-like sheet is to be used as the top sheet 2, the resin film or net-like sheet can be formed from polypropylene (PP), polyethylene (PE), polyethylene terephthalate (PET), or the like.

The back sheet 3 prevents menstrual blood that has been absorbed into the absorbent body 4 from leaking to the outside. As the back sheet there may be used a liquid-impermeable film composed mainly of polyethylene (PE) and polypropylene (PP), an air-permeable resin film, a composite film comprising an air-permeable resin film bonded to a spunbond or spunlace nonwoven fabric, or a spunbond/melt blowing/spunbond (SMS) nonwoven fabric comprising a highly water-resistant meltblown nonwoven fabric sandwiched between high-strength spunbond nonwoven fabrics. In order to soften the absorbent article 1 so as not to impair the feel during wearing of the absorbent article 1A, it is preferred to use a resin film with a basis weight of 15 to 30 g/$m^2$, composed mainly of a low-density polyethylene (LDPE) resin, for example, as the back sheet 3.

The absorbent body 4 has the function of absorbing and retaining menstrual blood. The absorbent body 4 preferably has high bulk, is resistant to deformation and has low chemical irritation. The absorbent body 4 used may be, for example, an absorbent body comprising hydrophilic fibers and a super-absorbent polymer (SAP), or a mixture comprising fluffy pulp or an airlaid nonwoven fabric and a super-absorbent polymer.

Hydrophilic fibers for an absorbent body 4 include cellulose, such as ground pulp and cotton, regenerated cellulose, such as rayon or fibril rayon, semi-synthetic cellulose, such as acetate and triacetate, particulate polymers,

filamentous polymers, thermoplastic hydrophobic chemical fibers, hydrophilicized thermoplastic hydrophobic chemical fibers, and mixtures of the foregoing. Cellulose foam and synthetic resin continuous foam may also be used in the absorbent body 4. Also, a foam or sheeted material may be pulverized and then molded into the absorbent body shape for use as the absorbent body 4. Preferably, ground pulp is used as the hydrophilic fibers for the absorbent body 4, in consideration of reducing cost and facilitating molding.

As super-absorbent polymers (SAP) for such an absorbent body 4 there are commonly used particulate polymers, such as sodium acrylate copolymer which exhibits absorptivity and hygroscopicity. In order to impart other functions to the polymer, silver, copper, zinc, silica, active carbon, an aluminosilicate compound, zeolite or the like may also be added to the polymer. This can impart functions, such as deodorant, antibacterial or heat-absorbing effects to the polymer.

The diffusion sheet 5 has a liquid-permeable property. This allows the diffusion sheet 5 to cause menstrual blood excreted into the top sheet 2 to rapidly migrate into the cover sheet 6, described hereunder. The diffusion sheet 5 also has a liquid-retaining property. As a result, a portion of the menstrual blood remains in the diffusion sheet 5, and the diffusion sheet is colored red. The diffusion sheet 5 that has been colored red is visible to the wearer through the top sheet 2, allowing confirmation that menstrual blood has been absorbed into the absorbent body 4.

The diffusion sheet 5 is preferably provided in the region in which the absorbent body 4 is provided. Thus, when the absorbent article is exchanged, the diffusion sheet 5 that has been colored red beyond the region in which the absorbent body 4 is formed is visible to the wearer, and can therefore prevent uneasiness regarding leakage of menstrual blood from the absorbent article 1.

The region in which the diffusion sheet 5 is provided preferably includes the excretory opening contact region. This can prevent excreted menstrual blood from migrating to the cover sheet 6 described below without passing through the diffusion sheet 5, either resulting in no red coloration of the diffusion sheet 5 or only partial red coloration of the diffusion sheet 5. The excretory opening contact region is the region corresponding to the excretory opening for menstrual blood of the wearer in the absorbent article 1.

The ratio of the area of the diffusion sheet 5 with respect to the area of the absorbent body 4 is preferably about 10% to about 90%, more preferably about 15% to about 70% and even more preferably about 20% to about 50%. If the ratio of the area of the diffusion sheet 5 with respect to the area of the absorbent body 4 is smaller than about 10%, the diffusion sheet 5 will be too small and it may be difficult to see the red-colored diffusion sheet 5. If the ratio of the area of the diffusion sheet 5 with respect to the area of the absorbent body 4 is greater than about 90%, menstrual blood may not diffuse throughout the entire diffusion sheet 5. This may cause diffusion of menstrual blood in the planar direction of the diffusion sheet 5 to be non-uniform.

The length of the diffusion sheet 5 in the lengthwise direction is preferably about 30 to about 200 mm, more preferably about 40 to about 170 mm and even more preferably about 50 to about 100 mm. Also, the length of the diffusion sheet 5 in the widthwise direction is preferably about 10 to about 100 mm, more preferably about 20 to about 70 mm and even more preferably about 25 to about 50 mm. When the length of the diffusion sheet 5 in the lengthwise direction is smaller than about 30 mm and the length of the diffusion sheet 5 in the widthwise direction is smaller than about 10 mm, the diffusion sheet 5 may become too small and it may be difficult for the red-colored diffusion sheet 5 to be visible. Also, if the length of the diffusion sheet 5 in the lengthwise direction is greater than about 200 mm, and the length of the diffusion sheet 5 in the widthwise direction is greater than about 100 mm, menstrual blood may not diffuse throughout the entire diffusion sheet 5. This may cause diffusion of menstrual blood in the planar direction of the diffusion sheet 5 to be non-uniform.

The diffusion sheet 5 preferably includes cellulose-based fibers. The proportion of cellulose-based fibers in the diffusion sheet 5 is preferably about 50-100 mass% and more preferably about 70-100 mass% based on the mass of the diffusion sheet 5. If the proportion of cellulose-based fibers in the diffusion sheet 5 is less than about 50 mass%, the liquid permeability of the diffusion sheet 5 may be poor, the liquid-retaining property of the diffusion sheet 5 may be low and coloration of the diffusion sheet 5 by menstrual blood may be light.

Cellulose-based fibers used in the diffusion sheet 5 may be ground pulp, cotton, regenerated cellulose (for example, rayon and fibril rayon), semi-synthetic cellulose (for example, acetate and triacetate), and combinations thereof. For reasons of cost reduction and satisfactory moldability, the cellulose-based fibers used in the diffusion sheet 5 are preferably ground pulp.

The method of web formation used for production of the diffusion sheet 5 may be a dry method (for example, a carding method, spunbond method, meltblown method, airlaid method or TOW fiber opening method), a wet method, or a combination thereof. The web bonding method used for production of the diffusion sheet 5 may be a thermal bonding, needle punching, chemical bonding, stitch bond or water jet punch method. For reasons of cost reduction and satisfactory diffusion performance, the diffusion sheet 5 is preferably produced by a wet method, and is a tissue composed mainly of ground pulp.

The cover sheet 6 masks the redness of menstrual blood absorbed by the absorbent body 4. This can minimize uneasy feeling of the wearer regarding the performance of the absorbent article 1, since menstrual blood absorbed into the absorbent body 4 is not conspicuous even when diffusion of the menstrual blood absorbed into the absorbent body

4 is non-uniform in the planar direction.

The cover sheet 6 also has a liquid-permeable property. This allows the cover sheet 6 to cause menstrual blood excreted into the top sheet 2 to rapidly migrate into the absorbent body 4. The cover sheet 6 does not have a liquid-retaining property. This can minimize residue of menstrual blood in the cover sheet 6, and reduce visibility of menstrual blood-reddened sections by the wearer in portions other than the diffusion sheet 5 mentioned above.

The cover sheet 6 contains thermoplastic chemical fibers at about 50-100 mass% and more preferably about 70-100 mass% with respect to the mass of the cover sheet 6. If the proportion of thermoplastic chemical fibers in the cover sheet 6 is lower than about 50 mass%, the liquid permeable property of the cover sheet 6 may be too low and the liquid-retaining property may be excessively high.

The starting material for the thermoplastic chemical fibers used in the cover sheet 6 may be polyethylene (PE), polypropylene (PP), polyethylene terephthalate (PET), or a graft polymer of PE and PP. The type of thermoplastic chemical fibers to be used for the cover sheet 6 may be monofilaments, composite fibers, heat-shrinkable fibers, heat-extendable fibers, irregularly shaped fibers, solid crimped fibers, split fibers or the like. Composite fibers to be used in the cover sheet 6 may be core-sheath composite fibers wherein the melting point of the core component is higher than the melting point of the sheath component (for example, eccentric core-sheath composite fibers), or side-by-side composite fibers wherein the two components have different melting points. Irregularly shaped fibers to be used in the cover sheet 6 may be hollow, flat, Y-type or C-type irregularly shaped fibers. Solid crimped fibers to be used in the cover sheet 6 may have latent crimping or developed crimping. Split fibers to be used in the cover sheet 6 may be split fibers that have been split by a physical load, such as a water stream, heat or embossing.

In order to facilitate migration of menstrual blood into the cover sheet 6, the thermoplastic chemical fibers of the cover sheet 6 may be fibers containing a hydrophilic agent or water-repellent agent, or fibers coated with a hydrophilic agent or water-repellent agent. The thermoplastic chemical fibers of the cover sheet 6 may also be fibers that have been hydrophilized by hydrophilicizing treatment, such as corona treatment or plasma treatment.

In order to increase the concealing property of the cover sheet 6, the cover sheet 6 may be subjected to whitening treatment. For example, the thermoplastic chemical fibers of the cover sheet 6 may contain an inorganic filler, such as titanium oxide, barium sulfate or calcium carbonate. Also, when the thermoplastic chemical fibers of the cover sheet 6 are core-sheath type composite fibers, the inorganic filler may be added only to the core or only to the sheath.

Furthermore, the bulk of the cover sheet 6 may be increased and the distance between the surface on the skin side of the top sheet 2 and the surface on the skin side of the absorbent body 4 may be increased, to increase the concealing property of the cover sheet 6.

In order to make the bulk of the cover sheet 6 resistant to collapse even after the cover sheet 6 has absorbed menstrual blood, and to maintain the whiteness of the cover sheet 6 even after the cover sheet 6 has absorbed menstrual blood, the fibers used in the cover sheet 6 are preferably composite fibers rather than monofilaments, and more preferably they are core-sheath composite fibers comprising polyethylene in the sheaths.

The size of the fibers used in the cover sheet 6 are preferably about 1.1 to about 8.8 dtex in order to facilitate permeation of menstrual blood into the cover sheet 6 and to increase whitening of the cover sheet 6.

The method of web formation used for production of the cover sheet 6 may be a dry method (for example, a carding method, spunbond method, meltblown method, airlaid method or TOW fiber opening method), a wet method, or a combination thereof. The web bonding method used for production of the cover sheet 6 may be a thermal bonding, needle punching, chemical bonding, stitch bond or water jet punch method. In order to facilitate permeation of menstrual blood into the cover sheet 6, and to increase whitening of the cover sheet 6, the nonwoven fabric used for the cover sheet 6 is preferably an air-through nonwoven fabric produced by a carding method.

The region in which the cover sheet 6 is provided preferably includes the region in which the diffusion sheet 5 is provided. Thus, the redness of menstrual blood absorbed into the absorbent body 4 is not visible surrounding the diffusion sheet 5, thus allowing increased visibility of the cover sheet 6 that has been colored red by menstrual blood.

The ratio of the area of the cover sheet 6 with respect to the area of the diffusion sheet 5 is preferably about 110% or greater, more preferably about 130% or greater and even more preferably 150% or greater. If the ratio of the area of the cover sheet 6 with respect to the area of the diffusion sheet 5 is smaller than about 110%, the area of the cover sheet 6 surrounding the diffusion sheet 5 will be small, such that the area of the non-reddened portion surrounding the cover sheet 6 that has been colored red by menstrual blood will also be small, and visibility of the cover sheet 6 that has been colored red with menstrual blood will be poor.

Furthermore, the ratio of the area of the cover sheet 6 with respect to the area of the absorbent body 4 is not particularly restricted so long as the redness of menstrual blood absorbed into the absorbent body 4 can be concealed, but it is preferably about 150% or less, more preferably about 100% or less, and even more preferably 100%.

The side sheet 7 prevents menstrual blood from leaking through the surface and/or interior of the top sheet 2 to the outside of the absorbent article 1 in the widthwise direction. The side sheet 7 preferably has hydrophobicity and water-repellency. A spunbond nonwoven fabric or SMS nonwoven fabric, for example, is used for the side sheet 7. In addition, since the side sheet 7 contacts with the skin of the wearer, an air-through nonwoven fabric that can reduce

rubbing irritation on the skin is preferably used as the side sheet 7. The side sheet 7 is not essential, however, in the absorbent article 1.

The top sheet 2, back sheet 3, absorbent body 4, diffusion sheet 5, cover sheet 6 and side sheet 7 are preferably bonded together to prevent interlayer separation between them. Their bonding may be accomplished, for example, by embossing, ultrasonic waves, with a hot-melt adhesive, or by a combination of the foregoing. The top sheet 2 and back sheet 3 are bonded at seal sections (not shown) formed by embossing, for example, at the both ends in the lengthwise direction of the body section 11. The back sheet 3 and side sheet 7 are bonded at seal sections (not shown) formed by embossing, for example, at both sides in the lengthwise direction of the wing sections 12. The top sheet 2 and diffusion sheet 5 are bonded by a hot-melt adhesive, for example. The top sheet 2 and side sheet 7 are bonded by a hot-melt adhesive, for example, on both sides in the widthwise direction of the body section 11.

The wing sections 12 are provided in the absorbent article 1 to stably anchor the absorbent article 1 to underwear. After the wing sections 12 have been folded on the outer side of the underwear, the absorbent article is attached to the crotch region of the underwear through the pressure-sensitive adhesive section 8 to allow the absorbent article 1 to be stably anchored to the underwear. The shapes of the wing sections 12 are roughly rectangular.

The pressure-sensitive adhesive section 8 on the clothing side of the back sheet 3 anchors the body section 11 to the inside of the crotch region of the underwear, and the pressure-sensitive adhesive section 8 on the clothing side of the wing sections 12 anchors the wing sections 12 to the outside of the crotch region of the underwear. The pressure-sensitive adhesive used to form the pressure-sensitive adhesive section 8 is preferably, for example, one composed mainly of a styrene-based polymer, tackifier or plasticizer. Styrene-based polymers include styrene-ethylene-butylene-styrene block copolymer, styrene-butylene polymer, styrene-butylene-styrene block copolymer and styrene-isobutylene-styrene copolymer, any of which may be used alone or as polymer blends of two or more. Styrene-ethylene-butylene-styrene block copolymer is preferred as the pressure-sensitive adhesive for the pressure-sensitive adhesive sections 8 from the viewpoint of satisfactory thermostability.

An organic compound that is solid at ordinary temperature is preferably used as the tackifier and plasticizer. A tackifier may be, for example, a C5 petroleum resin, C9 petroleum resin, dicyclopentadiene-based petroleum resin, rosin-based petroleum resin, polyterpene resin, terpenephenol resin or the like, and a plasticizer may be, for example, a monomer plasticizer, such as tricresyl phosphate, dibutyl phthalate or dioctyl phthalate, or a polymer plasticizer, such as a vinyl polymer or polyester.

The appearance of menstrual blood that has been absorbed into the absorbent article 1 will now be explained with reference to Fig. 3 and Fig. 4. Fig. 3 is a diagram illustrating the state where an absorbent article 1 has absorbed menstrual blood in the top sheet 2, diffusion sheet 5, cover sheet 6 and absorbent body 4, after menstrual blood excreted from the wearer has been absorbed. Fig. 4 is a plan view of an absorbent article 1 after absorption of menstrual blood that has been excreted by a wearer.

After the absorbent article 1 has absorbed menstrual blood, no menstrual blood resides in the top sheet 2, as shown in Fig. 3. However, faint vestiges 21 remain in the region where the top sheet 2 has absorbed menstrual blood. On the other hand, menstrual blood resides in the diffusion sheet 5 and the diffusion sheet 5 is colored red. Since the area of the diffusion sheet 5 is small, menstrual blood diffuses throughout the entire diffusion sheet 5 and the entire diffusion sheet 5 is colored red.

While menstrual blood does not reside in the cover sheet 6, faint vestiges 61 remain in the region where the cover sheet 6 has absorbed menstrual blood. On the other hand, menstrual blood resides in the absorbent body 4, and a red-colored menstrual blood diffusion region 41 is visible. Because the area of the absorbent body 4 is large, a portion of the absorbent body 4 is colored red. Thus, diffusion of menstrual blood absorbed into the absorbent body 4 is non-uniform in the planar direction.

As shown in Fig. 4, when the wearer views the absorbent article 1 from the outer side, the wearer can see a vestige 21 of absorbed menstrual blood in the top sheet 2, the diffusion sheet 5 that has been colored red by menstrual blood, and the menstrual blood diffusion region 41 of the absorbent body 4. Since the vestige 61 of absorbed menstrual blood on the cover sheet 6 (see Fig. 3) is faint, it is concealed by the top sheet 2 and cannot be seen. Also, the menstrual blood 41 diffused through the absorbent body 3 is only faintly seen because the redness is concealed by the cover sheet 6.

The surface on the skin side of the absorbent body 3 is separated from the surface on the skin side of the top sheet 2 by the bulk of the cover sheet 6, and therefore the menstrual blood 41 that has diffused through the absorbent body 4 can be seen separate from the surface on the skin side of the absorbent article 1. Also, as mentioned above, the menstrual blood 41 that has diffused through the absorbent body 3 can be faintly seen through the cover sheet 6, and therefore the menstrual blood 41 that has diffused through the absorbent body 4 can be seen further separated from the surface on the skin side of the absorbent article 1. Consequently, the menstrual blood 41 that has diffused through the absorbent body 4 is even less conspicuous.

As explained above, the vestige 21 of menstrual blood absorbed in the top sheet 2 is faint and is therefore inconspicuous. Furthermore, the vestige 61 of absorbed menstrual blood on the cover sheet 6 cannot be seen as it is concealed by the top sheet 2. In addition, the menstrual blood 41 that has diffused through the absorbent body 4 is

inconspicuous due to the cover sheet 6. Consequently, menstrual blood absorbed into the absorbent article 1 is visible only in the diffusion sheet 5. Incidentally, since the entire diffusion sheet 5 is colored red, there is no visible unevenness of diffusion of the absorbed menstrual blood in the planar direction of the absorbent article 1. Therefore, the absorbent article 1 can give the wearer a feeling of assurance regarding the performance of the absorbent article 1, when menstrual blood absorbed into the absorbent article 1 has become visible to the wearer when the absorbent article 1 is exchanged.

Furthermore, the wearer sometimes judges the performance of the absorbent article based on the difference in area between the area of diffusion of menstrual blood in the absorbent article 1 when the absorbent article 1 is viewed from the skin side (hereunder referred to as "menstrual blood diffusion area") and the area of diffusion of menstrual blood in the absorbent article 1 when the absorbent article 1 is viewed from the clothing side (hereunder referred to as "absorbent body diffusion area"). Specifically, a larger absorbent body diffusion area compared to the menstrual blood diffusion area results in absorption at the sections where the menstrual blood excreted into the top sheet 2 is separated further from the skin, and therefore the wearer may judge that the burden on the skin is reduced and that the absorbent article can absorb a large amount of menstrual blood. The absorbent article 1 according to one embodiment of the invention has a small menstrual blood diffusion area, and therefore a larger absorbent body diffusion area compared to the menstrual blood diffusion area. Thus, since the wearer judges that the burden on the skin is reduced and a large amount of menstrual blood has been absorbed when using the absorbent article 1, the absorbent article 1 can give the wearer a feeling of assurance regarding performance in this respect as well.

- Embodiment -

An absorbent article according to an embodiment of the invention will now be explained with reference to the accompanying drawings. However, the invention is not limited to the examples depicted in the drawings. The absorbent article according to the embodiment of the invention is a sanitary napkin.

Fig. 5 is a perspective view showing an absorbent article 1A according to the embodiment of the invention. The absorbent article 1A is a sanitary napkin. In the absorbent article 1A, the right side corresponds to the front of the wearer while the left side corresponds to the back of the wearer, and the absorbent article 1A has a shape with essentially longitudinal symmetry and bilateral symmetry.

The absorbent article 1A comprises a liquid-permeable top sheet 2A, a liquid-impermeable back sheet (not shown), and an absorbent body (not shown) between the liquid-permeable top sheet 2A and the liquid-impermeable back sheet. The absorbent article 1A has compressed sections 13A.

The absorbent article 1A has a domed section 14A that protrudes in the thickness direction of the absorbent article 1A in the excretory opening contact region. In the absorbent article 1A, the top sheet 2A has a plurality of ridge-furrow structures extending in the lengthwise direction on the skin contact surface, the borders between the ridges and furrows being illustrated as solid lines for convenience in Fig. 5. In the absorbent article 1A, the wide regions surrounded by two solid lines are the ridges and the narrow regions surrounded by two solid lines are the furrows, and in the absorbent article 1A, a plurality of ridges and a plurality of furrows are alternately arranged in the widthwise direction of the absorbent article 1A. Also, the absorbent article 1A has a perimeter section 15A surrounding the domed section 14A.

As used herein, "domed section" refers to a section including the top sheet 2A and the cushion section described hereunder, and since it generally has the maximum thickness of the absorbent article 1A, its thickness decreases toward the outer peripheral section.

As used herein, "center section" refers to the section that includes the part of the domed section 14A at the point where the thickness is at the maximum. The thickness is the thickness from the clothing contact surface of the back sheet. Also, the "center section", as it relates to the domed section 14A, is the region of preferably between 0 and about 50%, more preferably between 0 and about 40%, and even more preferably between 0 and about 30%, of the distance from the point of maximum thickness to the outer edge of the domed section 14A.

Fig. 6 is a front view of the absorbent article 1A shown in Fig. 5, as observed from the skin contact surface side of the top sheet 1A. In the absorbent article 1A shown in Fig. 6, the upper end corresponds to the front of the wearer, and the lower end corresponds to the back of the wearer. The absorbent article 1A comprises a liquid-permeable top sheet 2A, a liquid-impermeable back sheet (not shown), and an absorbent body 4A between the liquid-permeable top sheet 2A and the liquid-impermeable back sheet. The absorbent article 1A has a domed section 14A that protrudes in the thickness direction of the absorbent article 1A at the excretory opening and especially in the region that contacts with the labia minora. The domed section 14A includes part of the top sheet 2A, and a cushion section 16A situated between the top sheet 2A and the absorbent body 4A. A perimeter section 15A and compressed sections 13A surrounding the domed section 14A are also shown in Fig. 6.

Also, as shown in Fig. 6, a diffusion sheet 5A is provided in the section of the domed section 14A where the cushion section 16A is provided, and a cover sheet 6A is provided in the section where the absorbent body 4A is provided. The diffusion sheet 5A and cover sheet 6A of the absorbent article 1A of the embodiment are similar to the diffusion sheet 5 and cover sheet 6 of the absorbent article 1 of the reference example, and therefore explanation of the diffusion

sheet 5A and cover sheet 6A of the absorbent article 1A of the embodiment will be omitted here.

Similar to the absorbent article 1 of the reference example, when menstrual blood is absorbed by the absorbent article 1A of the embodiment, redness can be seen only throughout the diffusion sheet 5A, and therefore only the entire domed section 14A appears to be colored red. Therefore, the wearer sees uniform diffusion of absorbed menstrual blood in the planar direction of the absorbent article 1A. As a result, with the absorbent article 1A of the embodiment as well, the absorbent article 1A can give the wearer a feeling of assurance regarding the performance when menstrual blood absorbed into the absorbent article 1A has become visible to the wearer during exchange.

Fig. 7 is a schematic cross-sectional view of Fig. 6 along cross-section B-B. In the absorbent article 1A shown in Fig. 7, a liquid-impermeable back sheet 3A, an absorbent body 4A, a cover sheet 6A and a liquid-permeable top sheet 2A are layered in that order from the bottom, and a cushion section 16A and diffusion sheet 5A are disposed between the absorbent body 4A and the liquid-permeable top sheet 2A, in the excretory opening contact region. Therefore, the cushion section 16A is disposed between the diffusion sheet 5A and the cover sheet 6A.

Also, the domed section 14A includes part of the top sheet 2A and the cushion section 16A, and the absorbent article 1A has compressed sections 13A formed by compressing the top sheet 2A and absorbent body 4A near the outer edge of the cushion section 16A, and more specifically, outside the cushion section 16A. The absorbent article 1A has a plurality of ridge-furrow structures on the skin contact surface. The density of the cushion section 16A at the outer peripheral section 142A, in the domed section 14A of the absorbent article 1A, is higher than the density of the cushion section 16A at the center section 141A. An elastic member 17A is also shown in Fig. 7. A pressure-sensitive adhesive section 8A is applied onto the clothing contact surface of the liquid-impermeable back sheet 3A, and during use, the wearer fixes the pressure-sensitive adhesive section 8A to clothing for use of the absorbent article 1A.

In the absorbent article 1A, the material of the cushion section 16A is not particularly restricted and may be made of a material, such as, for example, a fiber-containing or non-fiber-containing material. Examples of fiber-containing materials include those that contain natural fibers, chemical fibers or both, and preferably a plurality of the fiber intersections are heat-fused. Heat-fusing the fiber intersections can provide excellent shape stability of the cushion section 16A, and therefore the domed section 14A, even after menstrual blood has been absorbed. The fiber-containing material of the cushion section 16A preferably comprises about 50 to about 100 mass% and more preferably about 70 to about 100 mass% of thermoplastic chemical fibers, in order to accomplish heat fusion between the fibers.

The starting material for the thermoplastic chemical fibers of the cushion section 16A may be polyethylene (PE), polypropylene (PP), polyethylene terephthalate (PET), PE and PP graft polymer, or the like, and the thermoplastic chemical fibers may be single filaments or composite fibers, such as core-sheath fibers, heat-shrinkable fibers or heat-extendable fibers. In consideration of facilitating uptake of menstrual blood and inhibiting rewet-back, the thermoplastic chemical fibers may be kneaded with or coated with a hydrophilic agent, water-repellent agent or the like. Corona treatment or plasma treatment of the thermoplastic chemical fibers can render the thermoplastic chemical fibers hydrophilic.

In order to increase the whiteness of the cushion section 16A, an inorganic filler, such as titanium oxide, barium sulfate or calcium carbonate may be added to the fibers. When the fibers composing the cushion section 16A are core-sheath fibers, the inorganic filler may be added to the core and/or sheath. When the cushion section 16A is a fiber-containing material, the fibers composing the fiber-containing material are preferably composite fibers rather than single filaments, and more preferably core-sheath fibers comprising polyethylene in the sheath, from the viewpoint of compression recoverability.

The composite fibers may be core-sheath fibers wherein the melting point of the core component is higher than the melting point of the sheath component, eccentric core-sheath fibers, or side-by-side fibers wherein the two components have different melting points. Also, when the cushion section 16A is a fiber-containing material, the cushion section 16A may comprise irregularly shaped fibers, such as hollow fibers, flat fibers, Y-shaped fibers or C-shaped fibers, solid crimped fibers, such as latent crimped or developed crimped fibers, or split fibers that have been split by a physical load, such as a water stream, heat, embossing or the like. When the cushion section 16A is a fiber-containing material, the fibers composing the cushion section 16A preferably have a size of about 1.1 to about 8.8 dtex, from the viewpoint of facilitating uptake of menstrual blood and improved feel on the skin.

Examples of natural fibers include cellulose, such as ground pulp or cotton, regenerated cellulose, such as rayon or fibril rayon, and semi-synthetic cellulose, such as acetate or triacetate, with ground pulp being preferred from the viewpoint of low cost and easier shaping. If the cushion section 16A contains cellulose, it will be possible to inhibit leakage when it is difficult to apply body pressure to the domed section 14A, due to sleeping posture, etc. If the cushion section 16A is formed of 100% thermoplastic chemical fibers, menstrual blood will flow on the outer surface of the top sheet 2A, often resulting in leakage.

When the cushion section 16A is a fiber-containing material, the cushion section 16A can be formed by a web forming method, for example, in a dry system (carding method, spunbond method, meltblown method, airlaid method or TOW) or a wet system, or a combination thereof. The cushion section 16A may be formed by a web bonding method, such as, for example, thermal bonding, needle punching, chemical bonding, hydroentangling or the like.

Examples of non-fiber-containing materials include elastomers, and continuous foam materials, such as foamed polyethylene. Specific examples of elastomers to be used in the cushion section 16A include polyester-based, urethane-based, olefin-based, styrene-based and polyamide-based thermoplastic elastomers, and low-density polyethylene or ethylene-$\alpha$-olefin copolymer using metallocene catalysts, as well as combinations of the foregoing. These polyester-based elastomers include those comprising an aromatic polyester in the hard segment and an amorphous polyether or aliphatic polyester in the soft segment. A "continuous foam material" is an open foam material having open foam cells. A closed foam material without open foam cells will result in poor permeation of menstrual blood through the interior of the cushion section, and inferior liquid permeability.

Examples of urethane-based elastomers include polyurethanes comprising polyesters, low molecular glycol, methylene bisphenyl isocyanate or the like as thermoplastic elastomers. Examples of olefin-based elastomers include random copolymers of ethylene and $\alpha$-olefin, and random copolymers of ethylene, $\alpha$-olefins and dienes. Examples of styrene-based elastomers include block copolymers, such as SEBS, SIS, SEPS and SBS. These polyamide-based elastomers may include nylon as the hard segment, and a polyester or polyol as the soft segment.

The non-fiber-containing material may include high-density polyethylene, low-density polyethylene, linear low-density polyethylene or the like for shaping stability. The non-fiber-containing material may also include an antiblocking agent, ultraviolet absorber, thickening/branching agent, delustering agent, coloring agent, or different types of modifiers. In consideration of facilitating uptake of menstrual blood and inhibiting rewet-back, the non-fiber-containing material may be kneaded with or coated with a hydrophilic agent, water-repellent agent or the like. Corona treatment or plasma treatment of the non-fiber-containing material can render the non-fiber-containing material hydrophilic.

If the absorbent article 1A has a domed section 14A that protrudes in the thickness direction of the absorbent article 1A at the excretory opening contact region, the domed section 14A includes a portion of the top sheet 2A and a cushion section 16A disposed between the top sheet 2A and the absorbent body 4A, the domed section 14A has a center section 141A and a peripheral section 142A surrounding the center section 141A, and the density of the cushion section 16A in the peripheral section 142A is higher than the density of the cushion section 16A in the center section 141A, then the absorbent article 1A will have an effect in which the domed section 14A will fit with the excretory opening of the wearer and especially the labia minora of the wearer, helping to prevent leakage, both when it is dry before absorption of menstrual blood and when it is wet after absorption of menstrual blood. The reason why the absorbent article 1A exhibits this effect is believed to be as follows.

The labia minora of an adult female does not have a uniform shape and it undergoes little shape deformation when the body is moved, especially compared to the labia majora, though with differences from individual to individual. Also, deformation of the labia majora during body movement varies greatly depending on the body type of the woman, the labia majora tending to deform much more easily during body movement in slightly overweight women.

In the absorbent article 1A, a domed section is provided in the excretory opening contact region, and the density of the cushion section 16A forming the domed section 14A differs between the outer peripheral section 142A of the domed section 14A and the center section 141A, the density of the cushion section 16A at the outer peripheral section 142A being higher than the density of the cushion section 16A at the center section 141A. Thus, the cushion section 16A that has relatively low density at the center section 141A of the domed section 14A has low rigidity, and when it contacts with the labia minora, it deforms along the shape of the labia minora allowing it to embed the labia minora. However, the cushion section 16A that has relatively high density at the outer peripheral section 142A of the domed section 14A has high rigidity, and it can continue to contact with the border between the labia minora and the labia majora, or with the labia majora, during periods of dryness before absorption of menstrual blood. Also, the cushion section 16A that has relatively high density at the outer peripheral section 142A of the domed section 14A has high compression recoverability, and therefore it can continue to contact with the complex-shaped excretory opening even during periods of wetness after menstrual blood has been absorbed.

As used herein, "excretory opening contact region" refers to the region bordering the excretory opening of the wearer, and "excretory opening" refers primarily to the labia minora, though not precluding that the outer peripheral section of the domed section borders with the labia majora.

Also, since the center section 141A of the domed section 14A is easily compressed, it deforms with depressions along the shape of the labia minora of the wearer during use, embedding the labia minora, so that the top sheet 2A closely contacts with the vaginal opening, and it is possible to prevent excessive spread of menstrual blood on the top sheet 2A. Also, since the top sheet 2A closely contacts with the vaginal opening, the distance between the vaginal opening and the absorbent body is minimal during use, and when the cushion section 16A is composed mainly of thermoplastic chemical fibers, menstrual blood passes through the top sheet 2A and cushion section 16A without diffusing in the planar directions during initial absorption of menstrual blood, and can rapidly migrate into the absorbent body 4A. Once a passage is formed for menstrual blood, the region becomes hydrophilicized, and therefore during a second or later absorption of menstrual blood, the menstrual blood still passes through the top sheet 2A and cushion section 16A and can rapidly migrate into the absorbent body 4A.

Furthermore, even in cases where body pressure is applied and the bulk of the cushion section 16A is temporarily

reduced, such that a large amount of menstrual blood pools in the cushion section 16A with the reduced bulk, the bulk of the cushion section 16A rapidly recovers when the body pressure is weakened, starting with the outer peripheral section 142A of the domed section 14A, allowing menstrual blood to rapidly migrate into the absorbent body 4A.

It is a feature of the absorbent article 1A that the density of the cushion section 16A at the outer peripheral section 142A of the domed section 14A is higher than the density of the cushion section 16A at the center section 141A of the domed section 14A, but this feature is achieved, for example, by having compressed sections 13A formed by compressing at least the top sheet 2A and absorbent body 4A, near the outer edge of the cushion section 16A. By compressing at least the top sheet 2A and the absorbent body 4A in such a manner that the outer edge of the cushion section 16A is compressed by tensile force of the compressed top sheet 2A and absorbent body 4A, creating a reduced thickness there, it is possible to increase the density of the cushion section 16A at the outer peripheral section 142A of the domed section 14A above the density of the cushion section 16A at the center section 141A of the domed section 14A. Also, since the degree of compression of the cushion section 16A is low at the center section 141A of the domed section 14A and the thickness is not reduced as much as the outer peripheral section 142A, the density of the cushion section 16A does not easily increase.

Specific examples of cases where compressed sections 13A are provided near the outer edge of the cushion section 16A, formed by compressing at least the top sheet 2A and the absorbent body 4A, include (i) an example in which compressed sections 13A are formed by compressing the top sheet 2A and absorbent body 4A on the outside of the cushion section 16A, and (ii) an example in which compressed sections 13A are formed by compressing the top sheet 2A, cushion section 16A and absorbent body 4A on the outer edge of the cushion section 16A.

As absorbent articles 1A there may be mentioned (i) an absorbent article 1A that is formed by compressing the outer edge of the cushion section 16A, and then layering the liquid-impermeable back sheet 3A, absorbent body 4A, cover sheet 6A, cushion section 16A, diffusion sheet 5A and top sheet 2A in that order, (ii) an absorbent article 1A that is formed by layering the cushion section 16A provided with the diffusion sheet 5A on the absorbent body 4A provided with the cover sheet 6A, compressing the outer edge of the cushion section 16A and the absorbent body together, and then layering the liquid-impermeable back sheet 3A, compressed members and liquid-permeable top sheet 2A in that order, and (iii) an absorbent article 1A that is formed by layering the cushion section 16A provided with the diffusion sheet 5A on the liquid-permeable top sheet 2A, compressing the outer edge of the cushion section 16A and the top sheet 2A together, and then layering the liquid-impermeable back sheet 3A, absorbent body 4A, cover sheet 6A and compressed members in that order.

With compressed sections 13A formed by compressing at least the top sheet 2A and absorbent body 4A near the outer edge of the cushion section 16A, it is possible during periods of wetness after menstrual blood has been absorbed, for example, to minimize detachment of the top sheet 2A from the absorbent body 4A and to maintain the height of the density of the cushion section 16A at the outer peripheral section 142A of the domed section 14A.

As used herein, the phrase "near the outer edge", as it relates to the cushion section 16A, is a concept including not only the outer edge of the cushion section 16A, but also an area inside the outer edge of the cushion section 16A and an area outside the outer edge of the cushion section 16A. Also, "near" means a range of preferably ±15%, more preferably ±10% and even more preferably ±5%, of the distance from the center of the cushion section 16A to the outer edge of the cushion section 16A, in the planar direction of the absorbent article 1A. The "center" of the cushion section 16A is the center in the lengthwise direction and the widthwise direction of the absorbent article 1A.

The absorbent article 1A has compressed sections 13A formed by continuously compressing the top sheet 2A and absorbent body 4A on the outside of the cushion section 16A, but the compressed sections may be present that are formed by intermittently compressing the top sheet 2A and absorbent body 4A on the outside of the cushion section 16A. Alternatively, compressed sections may be present that are formed by continuously or intermittently compressing the top sheet 2A, cushion section 16A and absorbent body 4A at the edge of the cushion section 16A. Compressed sections 13A may also be present that are formed by continuously or intermittently compressing only the cushion section 16A at the edge of the cushion section 16A.

The shape of the cushion section 16A before it is incorporated into the absorbent article 1A is not particularly restricted so long as an absorbent article 1A is formed in which the density of the cushion section 16A at the outer peripheral section 141A of the domed section 14A is higher than the density of the cushion section 16A at the center section 141A of the domed section 14A after the cushion section 16A has been incorporated, and for example, the projected form in the thickness direction of the absorbent article may have a shape similar to the labia minora, such as roughly circular, roughly elliptical, roughly rounded rectangular, or a figure surrounded by two arcs.

The cushion section 16A may also have a constant thickness in the thickness direction of the absorbent article 1A, or it may have a thickness that increases toward the outer edges from the center, or it may have a thickness that decreases toward the outer edges form the center. When the thickness of the cushion section 16A decreases from the center toward the outer edges, in cases where the absorbent body 4A and top sheet 2A have been compressed, it will often be difficult for the density of the cushion section 16A at the outer peripheral section 142A of the domed section 14A to be increased above the density of the cushion section 16A at the center section 141A of the domed section 14A,

and therefore it is preferred for the cushion section 16A to have a constant thickness, or for the thickness to increase from the center toward the outer edges. The cushion section 16A may also have a different basis weight at different locations.

The density of the cushion section 16A at the center section 141A of the domed section 14A is preferably about 0.001 to 0.1 g/cm$^3$, more preferably about 0.005 to about 0.08 g/cm$^3$, and even more preferably about 0.01 to about 0.05 g/cm$^3$. If the density is less than about 0.001 g/cm$^3$, the compression recoverability will tend to be inadequate during periods of wetness after menstrual blood has been absorbed, and if the density is greater than about 0.1 g/cm$^3$, it will tend to deform along the labia minora of the wearer, resulting in more difficult fitting to the labia minora.

The density of the cushion section 16A at the center section 141A and outer peripheral section 142A of the domed section 14A can be measured in the following manner.

(1) A two-dimensional laser displacement gauge is used to measure the thickness t (cm) of the cushion section at the measuring location. An example of such a two-dimensional laser displacement gauge is the LJ-G Series high precision two-dimensional laser displacement gauge (Model: LJ-G030) by Keyence Corp. The thickness of the cushion section 16A at the measuring location can be calculated by subtracting the thickness of the absorbent article 1A in the region other than the cushion section 16A, i.e. at the perimeter section 142A surrounding the domed section 14A, from the thickness of the absorbent article 1A at the measuring location.
(2) The cushion section 16A is removed from the absorbent article 1A and its basis weight b (g/m$^2$) is measured. When the basis weight of the cushion section 16A differs depending on the location, an approximately 15 mm × 15 mm sample is taken centered on the measuring location, and its basis weight b (g/m$^2$) is measured.
(3) The density d (g/cm$^3$) is calculated by the following formula:

$$d = b/(10{,}000 \times t)$$

As indicated by this formula, if the basis weight of the cushion section 16A is constant, the ratio of the density at locations other than the cushion section 16A can be compared by the thickness alone. That is, when the basis weight of the cushion section 16A is constant, a lower thickness of the cushion section 16A represents a higher density of the cushion section 16A.

In the absorbent article 1A, the density of the cushion section 16A at the outer peripheral section 142A of the domed section 14A is higher than the density of the cushion section 16A at the center section 141A of the domed section 14A, and preferably it is about 1.1 to about 5.0 times higher than the density of the cushion section 16A at the center section 141A of the domed section 14A, more preferably about 1.2 to about 4.0 times higher than the density of the cushion section 16A at the center section 141A of the domed section 14A, and even more preferably about 1.5 to about 3.0 times higher than the density of the cushion section 16A at the center section 141A of the domed section 14A. If the proportion is less than about 1.1 times higher, the compression recoverability of the cushion section 16A at the outer peripheral section 142A of the domed section 14A will tend to be inadequate during periods of wetness, and if the proportion is greater than about 5 times higher, the rigidity of the cushion section 16A at the outer peripheral section 142A of the domed section 14A will be increased, tending to leave the wearer with a feeling of the presence of a foreign object.

In the absorbent article 1A, the maximum thickness of the cushion section 16A is preferably about 3 to about 30 mm, more preferably about 4 to about 20 mm, and even more preferably about 5 to about 10 mm. If the maximum thickness of the cushion section 16A is within this range, it will fit the excretory opening of the wearer, and especially the labia minora, during wearing, thus helping to reduce leakage. As used herein, the term "maximum thickness" as it relates to the cushion section 16A and the domed section 14A, is the thickness at the thickest section of the cushion section 16A and domed section 14A.

The cushion section 16A has a length of preferably about 30 to about 300 mm, more preferably about 40 to about 250 mm and even more preferably about 50 to about 100 mm in the lengthwise direction of the absorbent article 1A, and a length of preferably about 10 to about 100 mm, more preferably about 20 to about 70 mm and even more preferably about 25 to about 50 mm in the widthwise direction of the absorbent article 1A. If the size of the cushion section 16A is within this range, the domed section 14A will fit the excretory opening of the wearer, and especially the labia minora, during wearing, thus allowing leakage to be minimized. If the size of the cushion section 16A is smaller than this range, the domed section 14A will fail to fit the excretory opening of the wearer, and especially the labia minora, tending to result in leakage, while if the size of the cushion section 16A is larger than this range, an uncomfortable feeling will tend to be noticed during wearing, or a gap will tend to form between the labia minora, resulting in easier leakage.

The cushion section 16A in the absorbent article of the present disclosure has a basis weight of preferably about 50 to about 1,000 g/m$^2$, more preferably about 100 to 500 g/m$^2$, and even more preferably about 150 to about

300 g/m$^2$. If the basis weight is within this range, the domed section 14A will not collapse even during periods of wetness after menstrual blood has been absorbed, and the domed section 14A will fit the excretory opening of the wearer, and especially the labia minora, thus reducing leakage.

The cushion section 14A preferably retains at least about 50% of its maximum thickness, more preferably it retains at least about 60% of its maximum thickness and even more preferably it retains at least about 70% of its maximum thickness, after absorption of 2 g of horse EDTA blood, compared to before horse EDTA blood absorption. If this range of the maximum thickness is retained, the domed section 14A including the cushion section 16A will be resistant to collapse even during periods of wetness after menstrual blood has been absorbed, and the domed section 14A will fit the excretory opening of the wearer, and especially the labia minora, thus reducing leakage.

The reason for dropping 2 g of horse EDTA blood is that the amount of menstrual blood excreted at once by a human is considered to be approximately 2 g. In order to absorb the horse EDTA blood into the cushion section, 2 g of horse EDTA blood is dropped onto the entire cushion section using a pipette, but when the cushion section comprises a water-repellent material, so that the horse EDTA blood is poorly taken up into the cushion section, the horse EDTA blood may be absorbed into the cushion section by applying pressure to the cushion section. The maximum thickness of the cushion section after absorption of 2 g of horse EDTA blood can be measured at 1 minute after all of the horse EDTA blood has been absorbed. The maximum thickness of the cushion section can also be measured using the aforementioned two-dimensional laser displacement gauge. The EDTA blood is described below.

In the absorbent article 1A, as shown in Fig. 5, the absorbent article 1A has a curved structure in which the domed section 14A curves inward. If the absorbent article 1A has a curved structure, the absorbent article 1A will presumably fit by curving with the body of the wearer, thus further helping to limit leakage of absorbed menstrual blood. The curved structure may be formed, for example, by passing an elastic member 17A (see Fig. 7), such as a rubber thread, expanding film or the like through both sides in the lengthwise direction of the absorbent article 1A, and applying tensile force to both sides in the lengthwise direction of the absorbent article.

The liquid-permeable top sheet 2A has a plurality of ridges and a plurality of furrows on the skin contact surface, extending in the lengthwise direction of the absorbent article 1A (throughout the present specification, the top sheet 2A with a plurality of ridges and a plurality of furrows extending in the lengthwise direction of the absorbent article will sometimes be referred to simply as "top sheet 2A with a ridge-furrow structure"). The top sheet 2A can be produced by the method described in Japanese Unexamined Patent Publication No. 2008-025078, Japanese Unexamined Patent Publication No. 2008-025079, or elsewhere.

The top sheet 2A with a ridge-furrow structure may be produced by the method described in Japanese Unexamined Patent Publication No. 2011-226010, Japanese Unexamined Patent Publication No. 2011-226011, or elsewhere. The top sheet 2A with a ridge-furrow structure can be formed by passing the top sheet to be treated through the gap between a pair of gear rolls with rotational axis lines that are perpendicular to the machine direction, and rotating while a plurality of teeth situated on the peripheral surfaces of each of the gear rolls are mutually engaged, and subjecting it to fluid treatment.

Specifically, the draw ratio of the gear rolls is preferably about 105% or greater, more preferably about 105% to about 500%, even more preferably about 120% to 300%, and even more preferably about 130% to about 200%. If the draw ratio is less than about 105%, the stretchability of the top sheet 2A may be inadequate and the cushion section 16A will more easily collapse during production of the absorbent article 1A, while if the draw ratio is greater than about 500%, the top sheet 2A will tend to tear during production of the absorbent article 1A.

The term "draw ratio" refers to the value calculated by the following formula:

$$\text{Draw ratio}\ (\%) = 100 \times \left[ \frac{\sqrt{P^2 + 4D^2}}{P} - 1 \right]$$

wherein P is the gear pitch and D is the gear tooth cutting depth.

The liquid-permeable top sheet 2A may also have a plurality of slits. If the liquid-permeable top sheet 2A has a plurality of slits running through the top sheet 2A, it will be possible to prevent widening of the slits and excessive collapse of the cushion section 16A during production of the absorbent article 1A. The top sheet 2A with a plurality of slits can be formed by passing the top sheet 2A through a slit roll having longitudinal slits arranged in a zigzag pattern.

The top sheet 2A with a plurality of slits can be produced as described in Japanese Patent Public Inspection No. 2002-528174, for example. The liquid-permeable top sheet 2A may also have a plurality of pinhole sections.

A top sheet 2A having a ridge-furrow structure, slits and open pinhole sections can prevent excessive collapse of the cushion section 16A during production of the absorbent article, due to change of the shape of the ridge-furrow structure of the top sheet 2A, and opening and closing of the slits and open pinhole sections. From this viewpoint, the ridge-furrow structure, slits and open pinhole sections of the top sheet 2A are preferably present at least at the section

bordering the cushion section 16A, i.e. the section composing the domed section 14A, but they may also be present over the entire top sheet 2A.

The absorbent article 1A may have any desired shape, such as rectangular, elliptical or gourd-shaped, and it may also have a flap to prevent slipping of clothing, such as shorts. Since the absorbent article 1A fits with the excretory opening of the wearer and especially the labia minora, helping to prevent leakage, it can be reduced in size, and the absorbent article of the present disclosure may have a length of preferably about 100 to about 500 mm, more preferably about 120 to about 350 mm and even more preferably about 150 to about 250 mm in the lengthwise direction, and a length of preferably about 40 to about 200 mm, more preferably about 45 to about 180 mm and even more preferably about 50 to 100 mm in the widthwise direction.

In the absorbent article 1A, the absorbent body 4A has a length of preferably about 80 to about 350 mm, more preferably about 100 to 300 mm and even more preferably about 120 to 250 mm in the lengthwise direction. This is because if a highly-rigid absorbent body 4A has an excessive size in the absorbent article 1A, the domed section 14A will not be able to easily contact the excretory opening, and especially the labia minora. For example, the gluteal region is a region that undergoes very large change during periods when the wearer walks or sits, and when the absorbent body 4A slips at sections bordering the gluteal region, the absorbent body 4A at the sections bordering the excretory opening, and especially the labia minora, is pulled with it and tends to also slip.

In the absorbent article 1A of the present disclosure, the absorbent body 4A has a length of preferably about 30 to about 100 mm, more preferably about 35 to about 80 mm and even more preferably about 40 to about 70 mm in the widthwise direction. This is because if the width of the absorbent body 4A is excessively greater than the width between the thighs of the wearer, deformation of the absorbent body 4A may lead to diffusion and/or transfer of menstrual blood to other regions, and leakage of the absorbed menstrual blood.

The domed section 14A also contains the blood slipping agent described below.

[Blood slipping agent]

For the absorbent article of the present disclosure, the liquid-permeable top sheet contains, at least in the projections, a blood slipping agent having a kinematic viscosity of about 0.01 to about 80 $mm^2/s$ at 40°C, a water holding percentage of about 0.05 to about 4.0 mass%, and a weight-average molecular weight of less than about 1,000.

The blood slipping agent has, at 40°C, a kinematic viscosity of about 0 to about 80 $mm^2/s$, preferably a kinematic viscosity of about 1 to about 70 $mm^2/s$, more preferably a kinematic viscosity of about 3 to about 60 $mm^2/s$, even more preferably a kinematic viscosity of about 5 to about 50 $mm^2/s$, and yet more preferably a kinematic viscosity of about 7 to about 45 $mm^2/s$.

The kinematic viscosity tends to be higher with a) a larger molecular weight of the blood slipping agent, b) a higher percentage of polar groups, such as carbonyl bonds (-CO-), ether bonds (-O-), carboxyl groups (-COOH) and hydroxyl groups (-OH), and c) a larger IOB.

In order to have a kinematic viscosity of about 0 to about 80 $mm^2/s$ at 40°C, the melting point of the blood slipping agent is preferably no higher than 45°C. This is because the kinematic viscosity will tend to be higher if the blood slipping agent contains crystals at 40°C.

As used herein, the "40°C kinematic viscosity" may be referred to simply as "kinematic viscosity".

The significance of the kinematic viscosity of the blood slipping agent will be explained below, but a kinematic viscosity exceeding about 80 $mm^2/s$ will tend to result in high viscosity of the blood slipping agent, such that it will not as easily slide down from the projections to the recesses together with menstrual blood that has reached the skin contact surface of the top sheet, and subsequently migrate into the absorbent body.

The kinematic viscosity can be measured according to JIS K 2283:2000, "5. Kinematic Viscosity Test Method", using a Cannon-Fenske reverse-flow viscometer, at a testing temperature of 40°C.

The blood slipping agent has a water holding percentage of about 0.01 to about 4.0 mass%, preferably it has a water holding percentage of about 0.02 to about 3.5 mass%, more preferably it has a water holding percentage of about 0.03 to about 3.0 mass%, even more preferably it has a water holding percentage of about 0.04 to about 2.5 mass%, and yet more preferably it has a water holding percentage of about 0.05 to about 2.0 mass%.

As used herein, "water holding percentage" means the percentage (mass) of water that can be held by a substance, and it may be measured in the following manner.

(1) A 20 mL test tube, a rubber stopper, the substance to be measured and deionized water are allowed to stand for a day and a night in a thermostatic chamber at 40°C.

(2) Into the test tube in the thermostatic chamber there are charged 5.0 g of the substance to be measured and 5.0 g of deionized water.

(3) The mouth of the test tube is sealed with the rubber stopper in the thermostatic chamber, and the test tube is rotated once and allowed to stand for 5 minutes.

(4) A 3.0 g portion of the layer of the substance to be measured (usually the upper layer) is sampled into a glass dish with a diameter of 90 mm and a mass $W_0$ (g), in the thermostatic chamber.
(5) The dish is heated at 105°C for 3 hours in an oven to evaporate off the moisture, and the mass $W_1$ (g) of each dish is measured.
(6) The water holding percentage is calculated by the following formula.

$$\text{Water holding percentage (mass\%)} = 100 \times [W_0 \text{ (g)} - W_1 \text{ (g)}]/3.0 \text{ (g)}$$

The measurement is conducted three times, and the average value is recorded.

The significance of the water holding percentage of the blood slipping agent will be explained below, but a low water holding percentage will tend to lower the affinity between the blood slipping agent and menstrual blood, thus impeding its migration into the absorbent body together with menstrual blood that has reached the skin contact surface of the top sheet. If the water holding percentage is high, on the other hand, the affinity between menstrual blood and the blood modifying agent will become very high, similar to a surfactant, and absorbed menstrual blood will tend to remain on the skin contact surface of the top sheet, resulting in more red coloration of the skin contact surface of the top sheet.

The water holding percentage tends to be a larger value with a) a smaller molecular weight of the blood slipping agent, and b) a higher percentage of polar groups, such as carbonyl bonds (-CO-), ether bonds (-O-), carboxyl groups (-COOH) and hydroxyl groups (-OH). This is because the blood slipping agent has greater hydrophilicity. The water holding percentage will tend to have a larger value with a greater IOB, i.e with a higher inorganic value or with a lower organic value. This is also because the blood slipping agent has greater hydrophilicity.

The blood slipping agent has a weight-average molecular weight of less than about 1,000, and preferably a weight-average molecular weight of less than about 900. This is because, if the weight-average molecular weight is about 1,000 or higher, tack may result in the blood slipping agent itself, tending to create a feeling of unpleasantness for the wearer. If the weight-average molecular weight increases, the viscosity of the blood slipping agent will tend to increase, and it will therefore be difficult to lower the viscosity of the blood slipping agent by heating to a viscosity suitable for coating, and as a result, the blood slipping agent may need to be diluted with a solvent.

The blood slipping agent preferably has a weight-average molecular weight of about 100 or greater, and more preferably it has a weight-average molecular weight of about 200 or greater. This is because if the weight-average molecular weight is low, the vapor pressure of the blood slipping agent may be increased, gasification may occur during storage and the amount may be reduced, often leading to problems, such as odor during wear.

As used herein, "weight-average molecular weight" includes the concept of a polydisperse compound (for example, a compound produced by stepwise polymerization, an ester formed from a plurality of fatty acids and a plurality of aliphatic monohydric alcohols), and a simple compound (for example, an ester formed from one fatty acid and one aliphatic monohydric alcohol), and in a system comprising $N_i$ molecules with molecular weight $M_i$ (i = 1, or i = 1, 2 ...), it refers to $M_w$ determined by the following formula.

$$M_w = \Sigma N_i M_i^2 / \Sigma N_i M_i$$

The weight-average molecular weights used throughout the present specification are the values measured by gel permeation chromatography (GPC), based on polystyrene.

The GPC measuring conditions may be the following, for example.

Device: Lachrom Elite high-speed liquid chromatogram by Hitachi High-Technologies Corp.
Columns: SHODEX KF-801, KF-803 and KF-804, by Showa Denko K.K.
Eluent: THF
Flow rate: 1.0 mL/min
Driving volume: 100 μL
Detection: RI (differential refractometer)

The weight-average molecular weights listed in the examples of the present specification were measured under the conditions described below.

The blood slipping agent may have an IOB of about 0.00 to about 0.60.

The IOB (Inorganic Organic Balance) is an indicator of the hydrophilic-lipophilic balance, and as used herein,

it is the value calculated by the following formula by Oda et al.:

$$IOB = Inorganic\ value/organic\ value.$$

The inorganic value and the organic value are based on the organic paradigm described in "Organic compound predictions and organic paradigms" by Fujita A., Kagaku no Ryoiki (Journal of Japanese Chemistry), Vol.11, No.10 (1957) p.719-725.

The organic values and inorganic values of major groups, according to Fujita, are summarized in Table 1 below.

Table 1

| Group | Inorganic value | Organic value |
|---|---|---|
| -COOH | 150 | 0 |
| -OH | 100 | 0 |
| -O-CO-O- | 80 | 0 |
| -CO- | 65 | 0 |
| -COOR | 60 | 0 |
| -O- | 20 | 0 |
| Triple bond | 3 | 0 |
| Double bond | 2 | 0 |
| $CH_2$ | 0 | 20 |
| *iso* branching | 0 | -10 |
| *tert* branching | 0 | -20 |
| Light metal (salts) | ≥500 | 0 |
| Heavy metal (salts), amines, $NH_3$ salts | ≥400 | 0 |

For example, in the case of an ester of tetradecanoic acid which has 14 carbon atoms and dodecyl alcohol which has 12 carbon atoms, the organic value is 520 ($CH_2$, 20 × 26) and the inorganic value is 60 (-COOR, 60 × 1), and therefore IOB = 0.12.

The IOB of the blood slipping agent is preferably between about 0.00 and 0.60, more preferably between about 0.00 and 0.50, even more preferably between about 0.00 and 0.40 and most preferably between about 0.00 and 0.30. If the IOB is within this range, it will be easier to meet the aforementioned conditions for the water-holding capacity and kinematic viscosity.

The blood slipping agent preferably has a melting point of no higher than 45°C. If the blood slipping agent has a melting point of no higher than 45°C, the blood slipping agent will more easily exhibit a kinematic viscosity in the aforementioned range.

As used herein, the term "melting point" refers to the peak top temperature for the endothermic peak during conversion from solid to liquid, upon measurement with a differential scanning calorimetry analyzer at a temperature-elevating rate of 10°C/min. The melting point may be measured using a Model DSC-60 DSC measuring apparatus by Shimadzu Corp., for example.

If the blood slipping agent has a melting point of no higher than about 45°C, it may be either liquid or solid at room temperature (about 25°C), or in other words, the melting point may be either about 25°C or higher or below about 25°C, and for example, it may have a melting point of about -5°C or about -20°C.

The blood slipping agent does not have a lower limit for its melting point, but its vapor pressure is preferably low. The vapor pressure of the blood slipping agent is preferably between about 0 and about 200 Pa, more preferably between about 0 and about 100 Pa, more preferably between about 0 and about 10 Pa, even more preferably between about 0 and about 1 Pa and yet more preferably between about 0.0 and about 0.1 Pa, at 25°C (1 atmosphere).

Considering that the absorbent article of the present disclosure is to be used in contact with the human body, the vapor pressure is preferably between about 0 and about 700 Pa, more preferably between about 0 and about 100 Pa, more preferably between about 0 and about 10 Pa, even more preferably between about 0 and about 1 Pa and yet more preferably between about 0.0 and about 0.1 Pa, at 40°C (1 atmosphere). If the vapor pressure of the blood slipping

agent is high, gasification may occur during storage and the amount may be reduced, often creating problems, such as odor during wear.

The melting point of the blood slipping agent may be selected depending on the weather or duration of wear. For example, in regions with a mean atmospheric temperature of no higher than about 10°C, using a blood slipping agent with a melting point of no higher than about 10°C may help the blood slipping agent function after excretion of menstrual blood, even if it has been cooled by the ambient temperature.

Also, when the absorbent article is to be used for a prolonged period of time, the melting point of the blood slipping agent is preferably at the high end of the range of no higher than about 45°C. This is so that the blood slipping agent will not be easily affected by sweat or friction during wearing, and will not easily become biased even during prolonged wearing.

In the technical field, the skin contact surfaces of top sheets are coated with surfactants in order to alter the surface tension of menstrual blood and promote rapid absorption of menstrual blood. However, the top sheet coated with the surfactant has very high affinity for the hydrophilic components (blood plasma, etc.) in menstrual blood, and acts to attract them, tending to cause menstrual blood instead to remain on the top sheet. The blood slipping agent, unlike conventionally known surfactants, has low affinity with menstrual blood and therefore does not cause residue of menstrual blood on the top sheet and allows rapid migration into the absorbent body.

The blood slipping agent is preferably selected from the group consisting of the following items (i)-(iii), and any combination thereof:

(i) a hydrocarbon;
(ii) a compound having (ii-1) a hydrocarbon moiety, and (ii-2) one or more, same or different groups selected from the group consisting of carbonyl group (-CO-) and oxy group (-O-) inserted between a C-C single bond of the hydrocarbon moiety; and
(iii) a compound having (iii-1) a hydrocarbon moiety, (iii-2) one or more, same or different groups selected from the group consisting of carbonyl group (-CO-) and oxy group (-O-) inserted between a C-C single bond of the hydrocarbon moiety, and (iii-3) one or more, same or different groups selected from the group consisting of carboxyl group (-COOH) and hydroxyl group (-OH) substituting a hydrogen of the hydrocarbon moiety.

As used herein, "hydrocarbon" refers to a compound composed of carbon and hydrogen, and it may be a chain hydrocarbon, such as a paraffinic hydrocarbon (containing no double bond or triple bond, also referred to as "alkane"), an olefin-based hydrocarbon (containing one double bond, also referred to as "alkene"), an acetylene-based hydrocarbon (containing one triple bond, also referred to as "alkyne"), or a hydrocarbon or cyclic hydrocarbon comprising two or more bonds selected from the group consisting of double bonds or triple bonds, such as aromatic hydrocarbons and alicyclic hydrocarbons.

Preferred as such hydrocarbons are chain hydrocarbons and alicyclic hydrocarbons, with chain hydrocarbons being more preferred, paraffinic hydrocarbons, olefin-based hydrocarbons and hydrocarbons with two or more double bonds (containing no triple bond) being more preferred, and paraffinic hydrocarbons being even more preferred.

Chain hydrocarbons include straight-chain hydrocarbons and branched-chain hydrocarbons.

When two or more oxy groups (-O-) are inserted in the compounds of (ii) and (iii) above, the oxy groups (-O-) are not adjacent. Thus, compounds (ii) and (iii) do not include compounds with continuous oxy groups (i.e. peroxides).

In the compounds of (iii), compounds in which at least one hydrogen on the hydrocarbon moiety is substituted with a hydroxyl group (-OH) are preferred over compounds in which at least one hydrogen on the hydrocarbon moiety is substituted with a carboxyl group (-COOH). This is because the carboxyl groups bond with metals and the like in menstrual blood, increasing the water holding percentage of the blood slipping agent, which may sometimes exceed the prescribed range. The same is true from the viewpoint of the IOB as well. As shown in Table 1, the carboxyl groups bond with metals and the like in menstrual blood, drastically increasing the inorganic value from 150 to 400 or greater, and therefore a blood slipping agent with carboxyl groups can increase the IOB value to more than about 0.60 during use.

The blood slipping agent is more preferably selected from the group consisting of the following items (i')-(iii'), and any combination thereof:

(i') a hydrocarbon;
(ii') a compound having (ii'-1) a hydrocarbon moiety, and (ii'-2) one or more, same or different bonds selected from the group consisting of carbonyl bond (-CO-), ester bond (-COO-), carbonate bond (-OCOO-), and ether bond (-O-) inserted between a C-C single bond of the hydrocarbon moiety; and
(iii') a compound having (iii'-1) a hydrocarbon moiety, (iii'-2) one or more, same or different bonds selected from the group consisting of carbonyl bond (-CO-), ester bond (-COO-), carbonate bond (-OCOO-), and ether bond (-O-) inserted between a C-C single bond of the hydrocarbon moiety, and (iii'-3) one or more, same or different groups selected from the group consisting of carboxyl group (-COOH) and hydroxyl group (-OH) substituting a hydrogen

on the hydrocarbon moiety;

with the proviso that when 2 or more same or different bonds are inserted in the compound of (ii') or (iii'), the bonds are not adjacent.

When 2 or more identical or different bonds are inserted in a compound of (ii') or (iii'), that is, when 2 or more identical or different bonds selected from among carbonyl bonds (-CO-), ester bonds (-COO-), carbonate bonds (-OCOO-) and ether bonds (-O-) are inserted, the bonds are not adjacent to each other, and at least one carbon atom lies between each of the bonds.

The blood slipping agent more preferably has no more than about 1.8 carbonyl bonds (-CO-), no more than two ester bonds (-COO-), no more than about 1.5 carbonate bonds (-OCOO-), no more than about 6 ether bonds (-O-), no more than about 0.8 carboxyl groups (-COOH) and/or no more than about 1.2 hydroxyl groups (-OH), per 10 carbon atoms in the hydrocarbon moiety.

The blood slipping agent is even more preferably selected from the group consisting of the following items (A)-(F), and any combination thereof:

(A) an ester of (A1) a compound having a chain hydrocarbon moiety and 2-4 hydroxyl groups substituting hydrogens on the chain hydrocarbon moiety, and (A2) a compound having a chain hydrocarbon moiety and 1 carboxyl group substituting a hydrogen on the chain hydrocarbon moiety;
(B) an ether of (B1) a compound having a chain hydrocarbon moiety and 2-4 hydroxyl groups substituting hydrogens on the chain hydrocarbon moiety and (B2) a compound having a chain hydrocarbon moiety and 1 hydroxyl group substituting a hydrogen on the chain hydrocarbon moiety;
(C) an ester of (C1) a carboxylic acid, hydroxy acid, alkoxy acid or oxoacid comprising a chain hydrocarbon moiety and 2-4 carboxyl groups substituting hydrogens on the chain hydrocarbon moiety and (C2) a compound having a chain hydrocarbon moiety and 1 hydroxyl group substituting a hydrogen on the chain hydrocarbon moiety;
(D) a compound having a chain hydrocarbon moiety and one bond selected from the group consisting of an ether bond (-O-), carbonyl bond (-CO-), ester bond (-COO-) and carbonate bond (-OCOO-) inserted between a C-C single bond of the chain hydrocarbon moiety;
(E) a polyoxy $C_3$-$C_6$ alkylene glycol, or alkyl ester or alkyl ether thereof; and
(F) a chain hydrocarbon.

The blood slipping agent according to (A) to (F) will now be explained in detail.

[(A) Ester of (A1) a compound having a chain hydrocarbon moiety and 2-4 hydroxyl groups substituting hydrogens on the chain hydrocarbon moiety, and (A2) a compound having a chain hydrocarbon moiety and 1 carboxyl group substituting a hydrogen on the chain hydrocarbon moiety]

The (A) ester of (A1) a compound having a chain hydrocarbon moiety and 2-4 hydroxyl groups substituting hydrogens on the chain hydrocarbon moiety, and (A2) a compound having a chain hydrocarbon moiety and 1 carboxyl group substituting a hydrogen on the chain hydrocarbon moiety (hereunder also referred to as "compound (A)") does not need to have all of the hydroxyl groups esterified, so long as it has the aforementioned kinematic viscosity, water holding percentage and weight-average molecular weight.

Examples for the (A1) compound having a chain hydrocarbon moiety and 2-4 hydroxyl groups substituting at hydrogens of the chain hydrocarbon moiety (hereunder also referred to as "compound (A1)") include chain hydrocarbon tetraols, such as alkanetetraols including pentaerythritol, chain hydrocarbon triols, such as alkanetriols including glycerin, and chain hydrocarbon diols, such as alkanediols including glycols.

Compounds for the (A2) compound having a chain hydrocarbon moiety and one carboxyl group substituting at a hydrogen of the chain hydrocarbon moiety include compounds in which one hydrogen on the hydrocarbon is substituted with one carboxyl group (-COOH), such as fatty acids.

Examples for compound (A) include ($a_1$) an ester of a chain hydrocarbon tetraol and at least one fatty acid, ($a_2$) an ester of a chain hydrocarbon triol and at least one fatty acid, and ($a_3$) an ester of a chain hydrocarbon diol and at least one fatty acids.

[($a_1$) Ester of a chain hydrocarbon tetraol and at least one fatty acid]

Examples of esters of a chain hydrocarbon tetraol and at least one fatty acid include tetraesters of pentaerythritols and fatty acids, represented by the following formula (1):

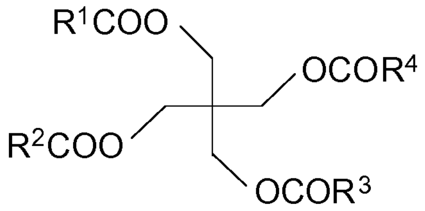

(1)

triesters of pentaerythritol and fatty acids, represented by the following formula (2):

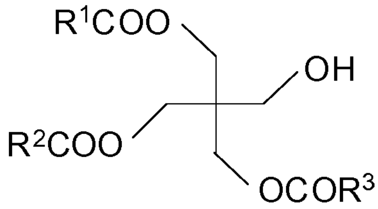

(2)

diesters of pentaerythritol and fatty acids, represented by the following formula (3):

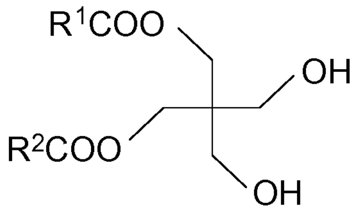

(3)

and monoesters of pentaerythritol and fatty acids, represented by the following formula (4).

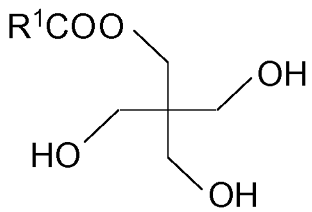

(4)

(In the formulas, $R^1$ to $R^4$ each represent a chain hydrocarbon.)

The fatty acids composing the esters of pentaerythritol and fatty acids ($R^1COOH$, $R^2COOH$, $R^3COOH$, and $R^4COOH$) are not particularly restricted so long as the pentaerythritol and fatty acid esters satisfy the conditions for the kinematic viscosity, water holding percentage and weight-average molecular weight, and for example, there may be mentioned saturated fatty acids, such as a $C_2$-$C_{30}$ saturated fatty acids, including acetic acid ($C_2$) ($C_2$ representing the number of carbons, corresponding to the number of carbons of $R^1C$, $R^2C$, $R^3C$ or $R^4C$, same hereunder), propanoic acid ($C_3$), butanoic acid ($C_4$) and its isomers, such as 2-methylpropanoic acid ($C_4$), pentanoic acid ($C_5$) and its isomers, such as 2-methylbutanoic acid ($C_5$) and 2,2-dimethylpropanoic acid ($C_5$), hexanoic acid ($C_6$), heptanoic acid ($C_7$), octanoic acid ($C_8$) and its isomers, such as 2-ethylhexanoic acid ($C_8$), nonanoic acid (Cg), decanoic acid ($C_{10}$), dodecanoic acid ($C_{12}$), tetradecanoic acid ($C_{14}$), hexadecanoic acid ($C_{16}$), heptadecanoic acid ($C_{17}$), octadecanoic acid ($C_{18}$), eicosanoic acid ($C_{20}$), docosanoic acid ($C_{22}$), tetracosanoic acid ($C_{24}$), hexacosanoic acid ($C_{26}$), octacosanoic acid ($C_{28}$) and triacontanoic acid ($C_{30}$), as well as isomers of the foregoing that have not been mentioned.

The fatty acid may also be an unsaturated fatty acid. Examples of unsaturated fatty acids include $C_3$-$C_{20}$ unsaturated fatty acids, such as monounsaturated fatty acids including crotonic acid ($C_4$), myristoleic acid ($C_{14}$), palmitoleic acid ($C_{16}$), oleic acid ($C_{18}$), elaidic acid ($C_{18}$), vaccenic acid ($C_{18}$), gadoleic acid ($C_{20}$) and eicosenoic acid ($C_{20}$), di-unsaturated fatty acids including linolic acid ($C_{18}$) and eicosadienoic acid ($C_{20}$), tri-unsaturated fatty acids including linolenic acids, such as $\alpha$-linolenic acid ($C_{18}$) and $\gamma$-linolenic acid ($C_{18}$), pinolenic acid ($C_{18}$), eleostearic acids, such as $\alpha$-eleostearic acid ($C_{18}$) and $\beta$-eleostearic acid ($C_{18}$), Mead acid ($C_{20}$), dihomo-$\gamma$-linolenic acid ($C_{20}$) and eicosatrienoic acid ($C_{20}$), tetra-unsaturated fatty acids including stearidonic acid ($C_{20}$), arachidonic acid ($C_{20}$) and eicosatetraenoic acid ($C_{20}$), penta-unsaturated fatty acids including bosseopentaenoic acid ($C_{18}$) and eicosapentaenoic acid ($C_{20}$), and

partial hydrogen adducts of the foregoing.

Considering the potential for degradation by oxidation and the like, the ester of pentaerythritol and a fatty acid is preferably an ester of pentaerythritol and a fatty acid derived from a saturated fatty acid, or in other words, an ester of pentaerythritol and a saturated fatty acid.

Also, in order to lower the water holding percentage value, the ester of pentaerythritol and a fatty acid is preferably a diester, triester or tetraester, more preferably a triester or tetraester, and most preferably a tetraester.

From the viewpoint of the IOB being from about 0.00 to about 0.60, in a tetraester of pentaerythritol and a fatty acid, the total number of carbons of the fatty acid composing the tetraester of the pentaerythritol and fatty acid, i.e. the total number of carbons of the $R^1C$, $R^2C$, $R^3C$ and $R^4C$ portions in formula (1), is preferably about 15 (the IOB is 0.60 when the total number of carbon atoms is 15).

Examples of tetraesters of pentaerythritol and fatty acids include tetraesters of pentaerythritol with hexanoic acid ($C_6$), heptanoic acid ($C_7$), octanoic acid ($C_8$), such as 2-ethylhexanoic acid ($C_8$), nonanoic acid ($C_9$), decanoic acid ($C_{10}$) and/or dodecanoic acid ($C_{12}$).

From the viewpoint of the IOB being from about 0.00 to about 0.60, in a triester of pentaerythritol and a fatty acid, the total number of carbons of the fatty acid composing the triester of the pentaerythritol and fatty acid, i.e. the total number of carbons of the $R^1C$, $R^2C$ and $R^3C$ portions in formula (2), is preferably about 19 or greater (the IOB is 0.58 when the number of carbon atoms is 19).

From the viewpoint of the IOB being from about 0.00 to about 0.60, in a diester of pentaerythritol and a fatty acid, the total number of carbons of the fatty acid composing the diester of the pentaerythritol and fatty acid, i.e. the total number of carbons of the $R^1C$ and $R^2C$ portion in formula (3), is preferably about 22 or greater (the IOB is 0.59 when the number of carbon atoms is 22).

From the viewpoint of the IOB being from about 0.00 to about 0.60, in a monoester of pentaerythritol and a fatty acid, the total number of carbons of the fatty acid composing the monoester of the pentaerythritol and fatty acid, i.e. the number of carbons of the $R^1C$ portion in formula (4), is preferably about 25 or greater (the IOB is 0.60 when the number of carbon atoms is 25).

The effects of double bonds, triple bonds, iso-branches and tert-branches are not considered in this calculation of the IOB (same hereunder).

Commercial products which are esters of pentaerythritol and fatty acids include UNISTAR H-408BRS and H-2408BRS-22 (mixed product) (both products of NOF Corp.) .

[($a_2$) Ester of a chain hydrocarbon triol and at least one fatty acid]

Examples of esters of a chain hydrocarbon triol and at least one fatty acid include triesters of glycerin and fatty acids, represented by formula (5):

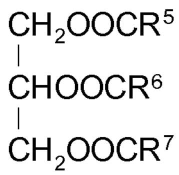

(5)

diesters of glycerin and fatty acids, represented by the following formula (6):

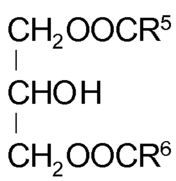

or

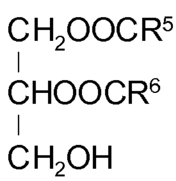

(6)

and monoesters of glycerin and fatty acids, represented by the following formula (7):

$$\begin{array}{l} CH_2OOCR^5 \\ | \\ CHOH \\ | \\ CH_2OH \end{array} \quad \text{or} \quad \begin{array}{l} CH_2OH \\ | \\ CHOOCR^5 \\ | \\ CH_2OH \end{array} \qquad (7)$$

wherein $R^5$-$R^7$ each represent a chain hydrocarbon.

The fatty acid composing the ester of glycerin and a fatty acid ($R^5COOH$, $R^6COOH$ and $R^7COOH$) is not particularly restricted so long as the ester of glycerin and a fatty acid satisfies the conditions for the kinematic viscosity, water holding percentage and weight-average molecular weight, and for example, there may be mentioned the fatty acids mentioned for the "($a_1$) Ester of chain hydrocarbon tetraol and at least one fatty acid", namely saturated fatty acids and unsaturated fatty acids, and in consideration of the potential for degradation by oxidation and the like, the ester is preferably a glycerin and fatty acid ester derived from a saturated fatty acid, or in other words, an ester of glycerin and a saturated fatty acid.

Also, from the viewpoint of lowering the water holding percentage value, the ester of glycerin and a fatty acid is preferably a diester or triester, and more preferably a triester.

A triester of glycerin and a fatty acid is also known as a triglyceride, and examples include triesters of glycerin and octanoic acid ($C_8$), triesters of glycerin and decanoic acid ($C_{10}$), triesters of glycerin and dodecanoic acid ($C_{12}$), triesters of glycerin and 2 or more different fatty acids, and mixtures of the foregoing.

Examples of triesters of glycerin and 2 or more fatty acids include triesters of glycerin with octanoic acid ($C_8$) and decanoic acid ($C_{10}$), triesters of glycerin with octanoic acid ($C_8$), decanoic acid ($C_{10}$) and dodecanoic acid ($C_{12}$), and triesters of glycerin with octanoic acid ($C_8$), decanoic acid ($C_{10}$), dodecanoic acid ($C_{12}$), tetradecanoic acid ($C_{14}$), hexadecanoic acid ($C_{16}$) and octadecanoic acid ($C_{18}$).

Considered from the viewpoint of obtaining a melting point of no higher than about 45°C, the triester of glycerin and a fatty acid preferably has a total number of carbon atoms in the fatty acid composing the triester of glycerin and a fatty acid, i.e. a total number of carbons in the $R^5C$, $R^6C$ and $R^7C$ portions in formula (5), of about 40 or less.

From the viewpoint of the IOB being from about 0.00 to about 0.60, in a triester of glycerin and a fatty acid, the total number of carbons of the fatty acid composing the triester of the glycerin and fatty acid, i.e. the total number of carbons of the $R^5C$, $R^6C$ and $R^7C$ portions in formula (5), is preferably about 12 or greater (the IOB is 0.60 when the total number of carbon atoms is 12).

Triesters of glycerin and fatty acids, being aliphatic and therefore potential constituent components of the human body, are preferred from the viewpoint of safety.

Commercial products of triesters of glycerin and fatty acids include tri-coconut fatty acid glycerides, NA36, PANACET 800, PANACET 800B and PANACET 810S, and tri-C2L oil fatty acid glycerides and tri-CL oil fatty acid glycerides (all products of NOF Corp.).

A diester of glycerin and a fatty acid is also known as a diglyceride, and examples include diesters of glycerin and decanoic acid ($C_{10}$), diesters of glycerin and dodecanoic acid ($C_{12}$), diesters of glycerin and hexadecanoic acid ($C_{16}$), diesters of glycerin and 2 or more different fatty acids, and mixtures of the foregoing.

From the viewpoint of the IOB being from about 0.00 to about 0.60, in a diester of glycerin and a fatty acid, the total number of carbons of the fatty acid composing the diester of the glycerin and fatty acid, i.e. the total number of carbons of the $R^5C$ and $R^6C$ portions in formula (6), is preferably about 16 or greater (the IOB is 0.58 when the total number of carbon atoms is 16).

Monoesters of glycerin and fatty acids are also known as monoglycerides, and examples include glycerin and octadecanoic acid ($C_{18}$) monoester, and glycerin and docosanoic acid ($C_{22}$) monoester.

From the viewpoint of the IOB being from about 0.00 to about 0.60, in a monoester of glycerin and a fatty acid, the total number of carbons of the fatty acid composing the monoester of the glycerin and fatty acid, i.e. the number of carbons of the $R^5C$ portion in formula (7), is preferably about 19 or greater (the IOB is 0.59 when the number of carbon atoms is 19).

[($a_3$) Ester of a chain hydrocarbon diol and at least one fatty acid]

Examples of esters of a chain hydrocarbon diol and at least one fatty acid include monoesters and diesters of fatty acids with $C_2$-$C_6$ chain hydrocarbon diols, such as $C_2$-$C_6$ glycols, including ethylene glycol, propylene glycol, butylene glycol, pentylene glycol and hexylene glycol.

Specifically, examples of esters of a chain hydrocarbon diol and at least one fatty acid include diesters of $C_2$-$C_6$ glycols and fatty acids, represented by the following formula (8):

$R^8COOC_kH_{2k}OCOR^9$ (8)

wherein k represents an integer of 2 to 6, and $R^8$ and $R^9$ each represent a chain hydrocarbon, and monoesters of $C_2$-$C_6$ glycols and fatty acids, represented by the following formula (9):

$R^8COOC_kH_{2k}OH$ (9)

wherein k represents an integer of 2 to 6, and $R^8$ is a chain hydrocarbon.

The fatty acid to be esterified in an ester of a $C_2$-$C_6$ glycol and a fatty acid (corresponding to $R^8COOH$ and $R^9COOH$ in formula (8) and formula (9)) is not particularly restricted so long as the ester of the $C_2$-$C_6$ glycol and fatty acid satisfies the conditions for the kinematic viscosity, water holding percentage and weight-average molecular weight, and for example, there may be mentioned the fatty acids mentioned above for the "($a_1$) Ester of chain hydrocarbon tetraol and at least one fatty acid", namely saturated fatty acids and unsaturated fatty acids, and in consideration of the potential for degradation by oxidation and the like, it is preferably a saturated fatty acid.

From the viewpoint of the IOB being from about 0.00 to about 0.60, in a diester of butylene glycol represented by formula (8) (k = 4) and a fatty acid, the total number of carbons of the $R^8C$ and $R^9C$ portions is preferably about 6 or greater (the IOB is 0.60 when the total number of carbon atoms is 6).

From the viewpoint of the IOB being from about 0.00 to about 0.60, in a monoester of ethylene glycol represented by formula (9) (k = 2) and a fatty acid, the number of carbons of the $R^8C$ portion is preferably about 12 or greater (the IOB is 0.57 when the number of carbon atoms is 12).

Considering the potential for degradation by oxidation and the like, the ester of the $C_2$-$C_6$ glycol and fatty acid is preferably a $C_2$-$C_6$ glycol and fatty acid ester derived from a saturated fatty acid, or in other words, an ester of a $C_2$-$C_6$ glycol and a saturated fatty acid.

Also, from the viewpoint of lowering the water holding percentage value, the ester of the $C_2$-$C_6$ glycol and fatty acid is preferably a glycol and fatty acid ester derived from a glycol with a greater number of carbons, such as an ester of a glycol and a fatty acid derived from butylene glycol, pentylene glycol or hexylene glycol.

Also, from the viewpoint of lowering the water holding percentage value, the ester of a $C_2$-$C_6$ glycol and fatty acid is preferably a diester.

Examples of commercial products of esters of $C_2$-$C_6$ glycols and fatty acids include COMPOL BL and COMPOL BS (both products of NOF Corp.).

[(B) Ether of (B1) a compound having a chain hydrocarbon moiety and 2-4 hydroxyl groups substituting hydrogens on the chain hydrocarbon moiety and (B2) a compound having a chain hydrocarbon moiety and 1 hydroxyl group substituting a hydrogen on the chain hydrocarbon moiety]

The (B) ether of (B1) a compound having a chain hydrocarbon moiety and 2-4 hydroxyl groups substituting hydrogens on the chain hydrocarbon moiety and (B2) a compound having a chain hydrocarbon moiety and 1 hydroxyl group substituting a hydrogen on the chain hydrocarbon moiety (hereunder also referred to as "compound (B)") does not need to have all of the hydroxyl groups etherified, so long as it has the aforementioned kinematic viscosity, water holding percentage and weight-average molecular weight.

The (B1) compound having a chain hydrocarbon moiety and 2-4 hydroxyl groups substituting at hydrogens of the chain hydrocarbon moiety (hereunder also referred to as "compound (B1)"), may be pentaerythritol, glycerin or glycol, for example, mentioned as compound (A1) for "compound (A)".

The (B2) compound having a chain hydrocarbon moiety and one hydroxyl group substituting at a hydrogen of the chain hydrocarbon moiety (hereunder also referred to as "compound (B2)") may be, for example, a compound in which one hydrogen of the hydrocarbon is substituted with one hydroxyl group (-OH), such as an aliphatic monohydric alcohol, which may be a saturated aliphatic monohydric alcohol or an unsaturated aliphatic monohydric alcohol.

Examples of saturated aliphatic monohydric alcohols include $C_1$-$C_{20}$ saturated aliphatic monohydric alcohols, such as methyl alcohol ($C_1$) ($C_1$ representing the number of carbon atoms, same hereunder), ethyl alcohol ($C_2$), propyl alcohol ($C_3$) and its isomers, including isopropyl alcohol ($C_3$), butyl alcohol ($C_4$) and its isomers, including sec-butyl alcohol ($C_4$) and tert-butyl alcohol ($C_4$), pentyl alcohol ($C_5$), hexyl alcohol ($C_6$), heptyl alcohol ($C_7$), octyl alcohol ($C_8$) and its isomers, including 2-ethylhexyl alcohol ($C_8$), nonyl alcohol ($C_9$), decyl alcohol ($C_{10}$), dodecyl alcohol ($C_{12}$), tetradecyl alcohol ($C_{14}$), hexadecyl alcohol ($C_{16}$), heptadecyl alcohol ($C_{17}$), octadecyl alcohol ($C_{18}$) and eicosyl alcohol ($C_{20}$), as well as their isomers other than those mentioned.

Unsaturated aliphatic monohydric alcohols include those wherein one C-C single bond of a saturated aliphatic monohydric alcohol mentioned above is replaced with a C=C double bond, such as oleyl alcohol, and for example, such alcohols are commercially available by New Japan Chemical Co., Ltd. as the RIKACOL Series and UNJECOL Series.

Examples for compound (B) include ($b_1$) ethers of a chain hydrocarbon tetraol and at least one aliphatic monohydric alcohol, such as monoethers, diethers, triethers and tetraethers, preferably diethers, triethers and tetraethers, more preferably triethers and tetraethers and even more preferably tetraethers, ($b_2$) ethers of a chain hydrocarbon triol and at least one aliphatic monohydric alcohol, such as monoethers, diethers and triethers, preferably diethers and triethers and more preferably triethers, and ($b_3$) ethers of a chain hydrocarbon diol and at least one aliphatic monohydric alcohol, such as monoethers and diethers, and preferably diethers.

Examples of ethers of a chain hydrocarbon tetraol and at least one aliphatic monohydric alcohol include tetraethers, triethers, diethers and monoethers of pentaerythritol and aliphatic monohydric alcohols, represented by the following formulas (10) to (13):

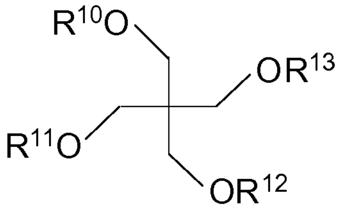 (10)

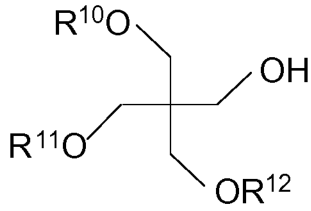 (11)

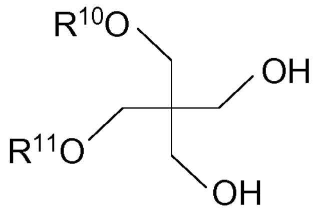 (12)

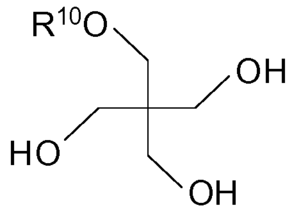 (13)

wherein $R^{10}$-$R^{13}$ each represent a chain hydrocarbon.

Examples of ethers of chain hydrocarbon triol and at least one aliphatic monohydric alcohol include triethers, diethers and monoethers of glycerin and aliphatic monohydric alcohols, represented by the following formulas (14) to (16):

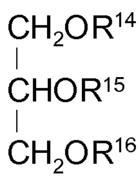 (14)

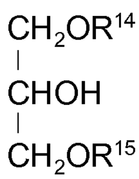 or 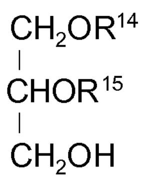 (15)

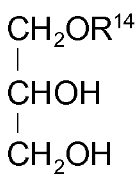 or 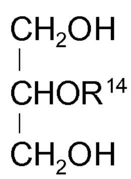 (16)

wherein $R^{14}$ to $R^{16}$ each represent a chain hydrocarbon.

Ethers of a chain hydrocarbon diol and at least one aliphatic monohydric alcohol include diethers of $C_2$-$C_6$ glycols and aliphatic monohydric alcohols, represented by the following formula (17):

$$R^{17}OC_nH_{2n}OR^{18} \qquad (17)$$

wherein n is an integer of 2 to 6, and $R^{17}$ and $R^{18}$ are each a chain hydrocarbon,

and monoethers of $C_2$-$C_6$ glycols and aliphatic monohydric alcohols, represented by the following formula (18):

$$R^{17}OC_nH_{2n}OH \qquad (18)$$

wherein n is an integer of 2 to 6, and $R^{17}$ is a chain hydrocarbon.

From the viewpoint of the IOB being between about 0.00 and about 0.60, in a tetraether of pentaerythritol and an aliphatic monohydric alcohol, the total number of carbon atoms of the aliphatic monohydric alcohol composing the tetraether of pentaerythritol and the aliphatic monohydric alcohol, i.e. the total number of carbon atoms of the $R^{10}$, $R^{11}$, $R^{12}$ and $R^{13}$ portions in formula (10), is preferably about 4 or greater (the IOB is 0.44 when the total number of carbon atoms is 4).

From the viewpoint of the IOB being between about 0.00 and about 0.60, in a triether of pentaerythritol and an aliphatic monohydric alcohol, the total number of carbon atoms of the aliphatic monohydric alcohol composing the triether of pentaerythritol and the aliphatic monohydric alcohol, i.e. the total number of carbon atoms of the $R^{10}$, $R^{11}$ and $R^{12}$ portions in formula (11), is preferably about 9 or greater (the IOB is 0.57 when the total number of carbon atoms is 9).

From the viewpoint of the IOB being between about 0.00 and about 0.60, in a diether of pentaerythritol and an aliphatic monohydric alcohol, the total number of carbon atoms of the aliphatic monohydric alcohol composing the diether of pentaerythritol and the aliphatic monohydric alcohol, i.e. the total number of carbon atoms of the $R^{10}$ and $R^{11}$ portions in formula (12), is preferably about 15 or greater (the IOB is 0.60 when the total number of carbon atoms is 15).

From the viewpoint of the IOB being between about 0.00 and about 0.60, in a monoether of pentaerythritol and an aliphatic monohydric alcohol, the number of carbon atoms of the aliphatic monohydric alcohol composing the monoether of pentaerythritol and the aliphatic monohydric alcohol, i.e. the number of carbon atoms of the $R^{10}$ portion in formula (13), is preferably about 22 or greater (the IOB is 0.59 when the number of carbon atoms is 22).

From the viewpoint of the IOB being between about 0.00 and about 0.60, in a triether of glycerin and an aliphatic monohydric alcohol, the total number of carbon atoms of the aliphatic monohydric alcohol composing the triether of glycerin and the aliphatic monohydric alcohol, i.e. the total number of carbon atoms of the $R^{14}$, $R^{15}$ and $R^{16}$ portions in formula (14), is preferably about 3 or greater (the IOB is 0.50 when the total number of carbon atoms is 3).

From the viewpoint of the IOB being between about 0.00 and about 0.60, in a diether of glycerin and an aliphatic monohydric alcohol, the total number of carbon atoms of the aliphatic monohydric alcohol composing the diether of glycerin and the aliphatic monohydric alcohol, i.e. the total number of carbon atoms of the $R^{14}$ and $R^{15}$ portions in formula (15), is preferably about 9 or greater (the IOB is 0.58 when the total number of carbon atoms is 9).

From the viewpoint of the IOB being between about 0.00 and about 0.60, in a monoether of glycerin and an aliphatic monohydric alcohol, the number of carbon atoms of the aliphatic monohydric alcohol composing the monoether of glycerin and the aliphatic monohydric alcohol, i.e. the number of carbon atoms of the $R^{14}$ portion in formula (16), is preferably 16 or greater (the IOB is 0.58 when the number of carbon atoms is 16).

From the viewpoint of the IOB being from about 0.00 to about 0.60, in a diether of butylene glycol represented by formula (17) (n = 4) and an aliphatic monohydric alcohol, the total number of carbon atoms of the $R^{17}$ and $R^{18}$ portions is preferably about 2 or greater (the IOB is 0.33 when the total number of carbon atoms is 2) .

From the viewpoint of the IOB being from about 0.00 to about 0.60, in a monoether of ethylene glycol represented by formula (18) (n = 2) and an aliphatic monohydric alcohol, the number of carbon atoms of the $R^{17}$ portion is preferably about 8 or greater (the IOB is 0.60 when the number of carbon atoms is 8).

Compound (B) can be produced by dehydrating condensation of compound (B1) and compound (B2) in the presence of an acid catalyst.

[(C) Ester of (C1) a carboxylic acid, hydroxy acid, alkoxy acid or oxoacid comprising a chain hydrocarbon moiety and 2-4 carboxyl groups substituting hydrogens on the chain hydrocarbon moiety and (C2) a compound having a chain hydrocarbon moiety and 1 hydroxyl group substituting a hydrogen on the chain hydrocarbon moiety]

The (C) ester of (C1) a carboxylic acid, hydroxy acid, alkoxy acid or oxoacid comprising a chain hydrocarbon moiety and 2-4 carboxyl groups substituting hydrogens on the chain hydrocarbon moiety and (C2) a compound having a chain hydrocarbon moiety and 1 hydroxyl group substituting a hydrogen on the chain hydrocarbon moiety (hereunder also referred to as "compound (C)") does not need to have all of the carboxyl groups esterified so long as it has the aforementioned kinematic viscosity, water holding percentage and weight-average molecular weight.

Examples for the (C1) carboxylic acid, hydroxy acid, alkoxy acid or oxoacid including a chain hydrocarbon moiety and 2-4 carboxyl groups substituting hydrogens of the chain hydrocarbon moiety (hereunder also referred to as "compound (C1)") include chain hydrocarbon carboxylic acids with 2-4 carboxyl groups, for example, chain hydrocarbon dicarboxylic acids, which include alkanedicarboxylic acids, such as ethanedioic acid, propanedioic acid, butanedioic acid, pentanedioic acid, hexanedioic acid, heptanedioic acid, octanedioic acid, nonanedioic acid and decanedioic acid, chain hydrocarbon tricarboxylic acids, which include alkanetricarboxylic acids, such as propanetrioic acid, butanetrioic

acid, pentanetrioic acid, hexanetrioic acid, heptanetrioic acid, octanetrioic acid, nonanetrioic acid and decanetrioic acid, and chain hydrocarbon tetracarboxylic acids, which include alkanetetracarboxylic acids, such as butanetetraoic acid, pentanetetraoic acid, hexanetetraoic acid, heptanetetraoic acid, octanetetraoic acid, nonanetetraoic acid and decanetetraoic acid.

Also, compound (C1) includes chain hydrocarbon hydroxy acids with 2-4 carboxyl groups, for example, chain hydrocarbon alkoxy acids with 2-4 carboxyl groups, such as malic acid, tartaric acid, citric acid and isocitric acid, and O-acetylcitric acid or chain hydrocarbon oxoacids with 2-4 carboxyl groups.

The (C2) compound with a chain hydrocarbon moiety and one hydroxyl group substituting at a hydrogen of the chain hydrocarbon moiety may be any of those mentioned for "compound (B)", such as an aliphatic monohydric alcohol.

Compound (C) may be ($c_1$) an ester, for example a monoester, diester, triester or tetraester, preferably a diester, triester or tetraester, more preferably a triester or tetraester and even more preferably a tetraester, of a chain hydrocarbon tetracarboxylic acid, hydroxy acid, alkoxy acid or oxoacid with 4 carboxyl groups, and at least one aliphatic monohydric alcohol, ($c_2$) an ester, for example, a monoester, diester or triester, preferably a diester or triester and more preferably a triester, of a chain hydrocarbon tricarboxylic acid, hydroxy acid, alkoxy acid or oxoacid with 3 carboxyl groups, and at least one aliphatic monohydric alcohol, or ($c_3$) an ester, for example, a monoester or diester, and preferably a diester, of a chain hydrocarbon dicarboxylic acid, hydroxy acid, alkoxy acid or oxoacid with 2 carboxyl groups, and at least one aliphatic monohydric alcohol.

Examples for compound (C) include dioctyl adipate and tributyl O-acetylcitrate, of which commercially available products exist.

[(D) Compound having a chain hydrocarbon moiety and one bond selected from the group consisting of an ether bond (-O-), carbonyl bond (-CO-), ester bond (-COO-) and carbonate bond (-OCOO-) inserted between a C-C single bond of the chain hydrocarbon moiety]

The (D) compound having a chain hydrocarbon moiety and one bond selected from the group consisting of an ether bond (-O-), carbonyl bond (-CO-), ester bond (-COO-) and carbonate bond (-OCOO-) inserted between a C-C single bond of the chain hydrocarbon moiety (hereunder also referred to as "compound (D)") may be ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, or ($d_4$) a dialkyl carbonate.

[($d_1$) Ether of an aliphatic monohydric alcohol and an aliphatic monohydric alcohol]

Ethers of aliphatic monohydric alcohols and aliphatic monohydric alcohols include compounds having the following formula (19):

$$R^{19}OR^{20} \qquad (19)$$

wherein $R^{19}$ and $R^{20}$ each represent a chain hydrocarbon.

The aliphatic monohydric alcohol composing the ether (corresponding to $R^{19}OH$ and $R^{20}OH$ in formula (19)) is not particularly restricted so long as the ether satisfies the conditions for the kinematic viscosity, water holding percentage and weight-average molecular weight, and for example, it may be one of the aliphatic monohydric alcohols mentioned for "compound (B)".

[($d_2$) Dialkyl ketone]

The dialkyl ketone may be a compound of the following formula (20):

$$R^{21}COR^{22} \qquad (20)$$

wherein $R^{21}$ and $R^{22}$ are each an alkyl group.

The dialkyl ketone may be a commercially available product, or it may be obtained by a known method, such as by oxidation of a secondary alcohol with chromic acid or the like.

[($d_3$) Ester of a fatty acid and an aliphatic monohydric alcohol]

Examples of esters of fatty acids and aliphatic monohydric alcohols include compounds having the following formula (21):

$R^{23}COOR^{24}$ (21)

wherein $R^{23}$ and $R^{24}$ each represent a chain hydrocarbon.

Examples of fatty acids composing these esters (corresponding to $R^{23}COOH$ in formula (21)) include the fatty acids mentioned for the "($A_1$) esters of chain hydrocarbon tetraols and fatty acids", and specifically these include saturated fatty acids and unsaturated fatty acids, with saturated fatty acids being preferred in consideration of the potential for degradation by oxidation and the like. The aliphatic monohydric alcohol composing the ester (corresponding to $R^{24}OH$ in formula (21)) may be one of the aliphatic monohydric alcohols mentioned for "compound (B)".

Examples of esters of such fatty acids and aliphatic monohydric alcohols include esters of dodecanoic acid ($C_{12}$) and dodecyl alcohol ($C_{12}$) and esters of tetradecanoic acid ($C_{14}$) and dodecyl alcohol ($C_{12}$), and examples of commercial products of esters of such fatty acids and aliphatic monohydric alcohols include ELECTOL WE20 and ELECTOL WE40 (both products of NOF Corp.).

[($d_4$) Dialkyl carbonate]

The dialkyl carbonate may be a compound of the following formula (22):

$R^{25}OC(=O)OR^{26}$ (22)

wherein $R^{25}$ and $R^{26}$ are each an alkyl group.

The dialkyl carbonate may be a commercially available product, or it may be synthesized by reaction between phosgene and an alcohol, reaction between formic chloride and an alcohol or alcoholate, or reaction between silver carbonate and an alkyl iodide.

From the viewpoint of the water holding percentage and vapor pressure, the weight-average molecular weight is preferably about 100 or greater and more preferably about 200 or greater, for ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, and ($d_4$) a dialkyl carbonate.

If the total number of carbon atoms is about 8 in a ($d_2$) dialkyl ketone, the melting point will be approximately -50°C and the vapor pressure will be about 230 Pa at 20°C, in the case of 5-nonanone, for example.

[(E) Polyoxy $C_3$-$C_6$ alkylene glycol, or alkyl ester or alkyl ether thereof]

The (E) polyoxy $C_3$-$C_6$ alkylene glycol, or its alkyl ester or alkyl ether (hereunder also referred to as "compound (E)") may be ($e_1$) a polyoxy $C_3$-$C_6$ alkylene glycol, ($e_2$) an ester of a polyoxy $C_3$-$C_6$ alkylene glycol and at least one fatty acid, or ($e_3$) an ether of a polyoxy $C_3$-$C_6$ alkylene glycol and at least one aliphatic monohydric alcohol. These will now be explained.

[($e_1$) Polyoxy $C_3$-$C_6$ alkylene glycol]

The polyoxy $C_3$-$C_6$ alkylene glycol is i) a homopolymer having one backbone selected from the group consisting of oxy $C_3$-$C_6$ alkylene backbones, i.e. oxyethylene backbone, oxypropylene backbone, oxybutylene backbone, oxypentylene backbone and oxyhexylene backbone, and having hydroxy groups at both ends, ii) a block copolymer having a backbone of two or more selected from among the aforementioned group and having hydroxy groups at both ends, or iii) a random copolymer having a backbone of two or more selected from among the aforementioned group and having hydroxy groups at both ends.

A polyoxy $C_3$-$C_6$ alkylene glycol is represented by the following formula (23):

$HO-(C_mH_{2m}O)_n-H$ (23)

wherein m is an integer of 3-6.

The present inventors have found that with polypropylene glycol (corresponding to a homopolymer of formula (23) where m = 3), the condition for the water holding percentage is not satisfied when the weight-average molecular weight is less than about 1,000. Therefore, polypropylene glycol homopolymer is not included in the scope of the blood slipping agent described above, and propylene glycol should be included in the ($e_1$) polyoxy $C_3$-$C_6$ alkylene glycol only as a copolymer or random polymer with another glycol.

Incidentally, investigation by the present inventors suggests that with polyethylene glycol (corresponding to a homopolymer of formula (23) where m = 2), the condition for the kinematic viscosity and water holding percentage cannot be satisfied when the weight-average molecular weight is less than about 1,000.

From the viewpoint of the IOB being about 0.00 to about 0.60, when formula (23) is polybutylene glycol (a homopolymer where m = 4), for example, preferably n ≥ about 7 (when n = 7, the IOB is 0.57).
Examples of commercial products of poly $C_3$-$C_6$ alkylene glycols include UNIOL™ PB-500 and PB-700 (all products of NOF Corp.).

[($e_2$) Ester of a polyoxy $C_3$-$C_6$ alkylene glycol and at least one fatty acid]

The ester of a polyoxy $C_3$-$C_6$ alkylene glycol and at least one fatty acid may be one wherein one or both of the OH ends of a polyoxy $C_3$-$C_6$ alkylene glycol mentioned above under "($e_1$) Polyoxy $C_3$-$C_6$ alkylene glycol" are esterified by a fatty acid, i.e. a monoester or a diester.
Examples of fatty acids to be esterified in the ester of a polyoxy $C_3$-$C_6$ alkylene glycol and at least one fatty acid include the fatty acids mentioned above under "($a_1$) Ester of chain hydrocarbon tetraol and at least one fatty acid", and specifically these include saturated fatty acids and unsaturated fatty acids, with saturated fatty acids being preferred in consideration of the potential for degradation by oxidation and the like.

[($e_3$) Ether of a polyoxy $C_3$-$C_6$ alkylene glycol and at least one aliphatic monohydric alcohol]

The ether of a polyoxy $C_3$-$C_6$ alkylene glycol and at least one aliphatic monohydric alcohol may be one wherein one or both of the OH ends of a polyoxy $C_3$-$C_6$ alkylene glycol mentioned above under "($e_1$) Polyoxy $C_3$-$C_6$ alkylene glycol" are etherified by an aliphatic monohydric alcohol, i.e. a monoether or diether.
In an ether of a polyoxy $C_3$-$C_6$ alkylene glycol and at least one aliphatic monohydric alcohol, the aliphatic monohydric alcohol to be etherified may be an aliphatic monohydric alcohol among those mentioned for "compound (B) ".

[(F) Chain hydrocarbon]

Examples of chain hydrocarbons include ($f_1$) chain alkanes, such as straight-chain alkanes and branched chain alkanes. Straight-chain alkanes with melting points of no higher than about 45°C have up to about 22 carbon atoms, and at a vapor pressure of 1 atmosphere and about 0.01 Pa or less at 25°C, the number of carbon atoms is 13 or greater. Branched chain alkanes tend to have lower melting points than straight-chain alkanes, given the same number of carbon atoms. Branched chain alkanes may therefore include those with 22 and more carbon atoms, even with melting points of below about 45°C.
Examples of commercially available hydrocarbon products include PARLEAM 6 (NOF Corp.).
Instead of a blood slipping agent, there may be used a blood slipping agent-containing composition containing a blood slipping agent and at least one other component.
Such a blood slipping agent-containing composition will now be described.

[Blood slipping agent-containing composition]

The blood slipping agent-containing composition contains a blood slipping agent and at least one other component. The other component is not particularly restricted so long as it does not inhibit the effect of the present disclosure, and it may be any one commonly employed in absorbent articles of the art, and especially top sheets.
Examples for the other component(s) include silicone oils, silicones, silicone-based resins and the like.
Examples for the other component(s) also include antioxidants, such as BHT (2,6-di-t-butyl-p-cresol), BHA (butylated hydroxyanisole) and propyl gallate.
Further examples for the other component(s) include vitamins, such as natural vitamins and synthetic vitamins. Examples of vitamins include water-soluble vitamins, such as group B vitamins, including vitamin $B_1$, vitamin $B_2$, vitamin $B_3$, vitamin $B_5$, vitamin $B_6$, vitamin $B_7$, vitamin $B_9$ and vitamin $B_{12}$, and vitamin C.
Other examples of vitamins include fat-soluble vitamins, such as group A vitamins, group D vitamins, group E vitamins and group K vitamins.
The derivatives of these vitamins are also included.
Examples for the other component(s) include amino acids, such as alanine, arginine, lysine, histidine, proline and hydroxyproline, and peptides.
Other examples for the other component(s) include zeolite, such as natural zeolite, examples of which include analcite, chabazite, heulandite, natrolite, stilbite and thomosonite, and synthetic zeolite.
Still other examples for the other component(s) include cholesterol, hyaluronic acid, lecithin and ceramide.
Yet other examples for the other component(s) include drugs, such as skin astringents, anti-pimple medications, anti-wrinkle agents, anti-cellulite agents, skin whiteners, antimicrobial agents and antifungal agents.
Examples of skin astringents include zinc oxide, aluminum sulfate, tannic acid and the like, and oil-soluble skin

astringents, such as fat-soluble polyphenols. Fat-soluble polyphenols include natural fat-soluble polyphenols, such as barley extract, otogiriso extract, white deadnettle extract, chamomilla extract, burdock extract, salvia extract, linden extract, common lime extract, white birch extract, common horsetail extract, sage extract, salvia extract, walnut (*J. regia* L. var. *orientalis*) extract, hibiscus extract, loquat leaf extract, Miquel's linden extract, hop extract, common horse-chestnut extract and coix seed extract.

Examples of anti-pimple medications include salicylic acid, benzoyl peroxide, resorcinol, sulfur, erythromycin and zinc.

Examples of anti-wrinkle agents include lactic acid, salicylic acid, salicylic acid derivatives, glycolic acid, phytic acid, lipoic acid and lysophosphatidic acid.

Examples of anti-cellulite agents include xanthine compounds, such as aminophylline, caffeine, theophylline and theobromine.

Examples of skin whiteners include niacinamide, kojic acid, arbutin, glucosamine and its derivatives, phytosterol derivatives, and ascorbic acid and its derivatives, as well as mulberry extract and placenta extract.

Examples for the other component(s) also include anti-inflammatory components, pH regulators, antimicrobial agents, humectants, aromatics, pigments, dyes, pigments and plant extracts. Examples of anti-inflammatory components include naturally-derived anti-inflammatory drugs, such as peony, golden grass, otogiriso, chamomile, licorice, peach leaf, Japanese mugwort and perilla extract, and synthetic anti-inflammatory drugs, such as allantoin and dipotassium glycyrrhizinate.

Examples of pH regulators include those that keep the skin weakly acidic, such as malic acid, succinic acid, citric acid, tartaric acid and lactic acid.

Titanium oxide is an example of a pigment.

The blood slipping agent-containing composition contains the blood slipping agent and the one or more other components at preferably about 50 to about 99 mass% and about 1 to about 50 mass%, respectively, more preferably about 60 to about 99 mass% and about 1 to about 40 mass%, respectively, even more preferably about 70 to about 99 mass% and about 1 to about 30 mass%, respectively, yet more preferably about 80 to about 99 mass% and about 1 to about 20 mass%, respectively, even yet more preferably about 90 to 99 mass% and about 1 to about 10 mass%, respectively, and even yet more preferably about 95 to 99 mass% and about 1 to about 5 mass%, respectively. These ranges are from the viewpoint of the effect of the present disclosure.

The blood slipping agent-containing composition preferably contains a surfactant in not greater than the amount from hydrophilicizing treatment of the top sheet or second sheet. More specifically, the blood slipping agent-containing composition contains a surfactant in a basis weight range of preferably about 0.0 to about 1.0 g/$m^2$, more preferably about 0.0 to about 0.8 g/$m^2$, even more preferably about 0.1 to about 0.5 g/$m^2$, and yet more preferably about 0.1 to about 0.3 g/$m^2$.

This is because when the amount of surfactant is increased, menstrual blood will tend to be retained in the top sheet. The surfactant, incidentally, has no water holding percentage. This is because there is no layer of the substance to be measured due to its mixture with water.

The blood slipping agent-containing composition contains water in a basis weight range of preferably about 0.0 to about 1.0 g/$m^2$, more preferably about 0.0 to about 0.8 g/$m^2$, even more preferably about 0.1 to about 0.5 g/$m^2$, and yet more preferably about 0.1 to about 0.3 g/$m^2$.

Since water lowers the absorption performance of the absorbent article, the amount is preferably low.

Similar to the blood slipping agent, the blood slipping agent-containing composition, as a composition, has at 40°C, a kinematic viscosity of preferably about 0 to about 80 $mm^2$/s, more preferably a kinematic viscosity of about 1 to about 70 $mm^2$/s, even more preferably a kinematic viscosity of about 3 to about 60 $mm^2$/s, yet more preferably a kinematic viscosity of about 5 to about 50 $mm^2$/s, and even yet more preferably a kinematic viscosity of about 7 to about 45 $mm^2$/s.

If the kinematic viscosity of the blood slipping agent-containing composition exceeds 80 $mm^2$/s, the viscosity will increase, and the blood slipping agent composition may not slide down into the interior of the absorbent article as easily with menstrual blood that has reached the skin contact surface of the top sheet.

When the blood slipping agent-containing composition contains a component that is miscible with the blood slipping agent, as at least one other component, the other component preferably has a weight-average molecular weight of less than about 1,000, and more preferably a weight-average molecular weight of less than about 900. This is because if the weight-average molecular weight is about 1,000 or higher, tack may result in the blood slipping agent-containing composition itself, tending to create a feeling of unpleasantness for the wearer. If the weight-average molecular weight increases, the viscosity of the blood slipping agent-containing composition will tend to increase, and it will therefore be difficult to lower the viscosity of the blood slipping agent composition by heating to a viscosity suitable for coating, and as a result, the blood slipping agent may need to be diluted with a solvent.

The blood slipping agent-containing composition, as a composition, has a water holding percentage of about 0.01 to about 4.0 mass%, preferably it has a water holding percentage of about 0.02 to about 3.5 mass%, more preferably it has a water holding percentage of about 0.03 to about 3.0 mass%, even more preferably it has a water holding

percentage of about 0.04 to about 2.5 mass%, and yet more preferably it has a water holding percentage of about 0.05 to about 2.0 mass%.

A low water holding percentage value will tend to lower the affinity between the blood slipping agent composition and menstrual blood, thus inhibiting it from sliding down into the interior of the absorbent article with menstrual blood that has reached the skin contact surface of the top sheet.

When the blood slipping agent-containing composition contains solid matter, it is preferably removed by filtration for measurement of the kinematic viscosity and water holding percentage.

In this absorbent article, the top sheet comprises the blood slipping agent at a basis weight in the range of preferably between about 1 and about 30 g/$m^2$, more preferably between about 2 and about 20 g/$m^2$ and more preferably between about 3 and about 10 g/$m^2$. If the basis weight of the blood slipping agent is lower than about 1 g/$m^2$, the absorbed menstrual blood will tend to remain in the top sheet, while if the basis weight of the blood slipping agent is greater than 30 g/$m^2$, there will tend to be an increase in sticky feel during wear.

The basis weight of the blood slipping agent in the top sheet, referred to throughout the present specification, may be measured in the following manner.

(1) The region of the top sheet that is to be measured is cut out using a sharp blade, such as a cutter replacement blade, while minimizing any alteration in thickness, to obtain a sample.
(2) The area of the sample: SA ($m^2$) and the mass: $SM_0$ (g) are measured.
(3) The sample is stirred for at least 3 minutes in a solvent that can dissolve the blood slipping agent, such as ethanol or acetone, to dissolve the blood slipping agent in the solvent.
(4) The sample is filtered on mass-measured filter paper, and the sample is thoroughly rinsed with the solvent on the filter paper. The sample on the filter paper is dried in an oven at 60°C.
(5) The masses of the filter paper and sample are measured, and the mass of the filter paper is subtracted to calculate the dry sample mass: $SM_1$ (g).
(6) The basis weight BBS (g/$m^2$) of the blood slipping agent is calculated by the following formula.

$$BBS\ (g/m^2) = [SM_0\ (g) - SM_1\ (g)]/SA\ (m^2)$$

In order to minimize error, multiple samples are taken from multiple absorbent articles, without the total area of the sample exceeding 100 $cm^2$, conducting several repeated measurements and taking the average value.

When the material to be coated with the blood slipping agent, such as the top sheet, is a nonwoven fabric, woven fabric or porous film made of a synthetic resin, it is preferably subjected to hydrophilicizing treatment by coating the surface with a hydrophilic agent, or by combining it with a synthetic resin or a film. This is because, if the original material is hydrophilic, there will be lipophilic regions due to the blood slipping agent and hydrophilic regions due to the hydrophilic agent sparsely dispersed on the top sheet, which will facilitate sliding down of menstrual blood onto the projections and recesses of the top sheet, and its subsequent migration into the absorbent body.

The blood slipping agent or blood slipping agent-containing composition may, if desired, be applied as a coating solution containing a volatile solvent, such as an alcohol-based solvent, ester-based solvent or aromatic solvent. If the coating solution includes a volatile solvent, the viscosity of the coating solution containing the blood slipping agent or blood slipping agent-containing composition will be lowered, thereby allowing the application steps to be simplified, facilitating application and making heating during application unnecessary.

There are no particular restrictions on the method of applying the blood slipping agent or blood slipping agent-containing composition, or the coating solution containing it, and if necessary the blood slipping agent or blood slipping agent-containing composition or the coating solution containing it may be heated, and a coating applicator, for example a non-contact coater, such as a spiral coater, curtain coater, spray coater or dip coater, or a contact coater, may be used for application of the blood slipping agent or blood slipping agent-containing composition or the coating solution containing it. The coating applicator is preferably a non-contact coater, from the viewpoint of uniformly dispersing the droplet or particulate modifying agent throughout, and from the viewpoint of not causing damage in the material.

The blood slipping agent or blood slipping agent-containing composition, or the coating solution containing it, may be coated directly, if it is a liquid at room temperature, or it may be heated to lower the viscosity, and when it is a solid at room temperature, it may be heated to liquefaction and coated from a control seam HMA (Hot Melt Adhesive) gun. By increasing the air pressure of the control seam HMA gun, it is possible to apply the blood slipping agent or blood slipping agent-containing composition as fine particulates.

The coating amount of the blood modifying agent or blood slipping agent-containing composition can be adjusted, for example, by adjusting the discharged amount from a control seam HMA gun.

The blood slipping agent may be coated during production of the top sheet material, such as the nonwoven

fabric, or it may be coated in the manufacturing line for production of the absorbent article. From the viewpoint of minimizing equipment investment, the blood slipping agent is preferably coated in the manufacturing line for the absorbent article, and in order to prevent shedding of the blood slipping agent which may contaminate the line, the blood slipping agent is preferably coated during a step downstream from the manufacturing line, and specifically, immediately before encapsulation of the product in an individual package.

The blood slipping agent also has an effect as a lubricant. When the top sheet is a nonwoven fabric, therefore, the blood slipping agent can reduce friction between fibers, thereby improving the flexibility of the nonwoven fabric as a whole. When the top sheet is a resin film, the blood slipping agent can reduce friction between the top sheet and the skin.

According to a preferred embodiment of the absorbent article of the present disclosure, the absorbent article is one that is intended for absorption of blood, such as a sanitary napkin or panty liner.

An absorbent article of the present disclosure does not require components, such as emollients and immobilizing agents, unlike in an absorbent article containing a known skin care composition, lotion composition or the like, and the blood slipping agent alone may be applied to the top sheet.

The aforementioned reference example and embodiment may also be employed in combination. For example, in the top sheet 2 of the absorbent article 1 of the reference example, the blood slipping agent may be coated at least on the region in which the diffusion sheet 5 is provided.

The explanation above is merely an example, and the invention is in no way restricted by the described embodiment.

Examples

The present disclosure will now be explained in fuller detail by examples, with the understanding that it is not meant to be limited to the examples.

The blood slipping agents used for testing are listed below.

[($a_1$) Ester of a chain hydrocarbon tetraol and at least one fatty acid]

- UNISTAR H-408BRS, product of NOF Corp.

  Pentaerythritol tetra(2-ethylhexanoate), weight-average molecular weight: approximately 640

- UNISTAR H-2408BRS-22, product of NOF Corp.

  Mixture of pentaerythritol tetra(2-ethylhexanoate) and neopentylglycol di(2-ethylhexanoate) (58:42 as weight ratio), weight-average molecular weight: approximately 520

[($a_2$) Ester of a chain hydrocarbon triol and at least one fatty acid]

- Tri-C2L oil fatty acid glyceride, product of NOF Corp.

  Glycerin and fatty acid triester with $C_8$ fatty acid:$C_{10}$ fatty acid:$C_{12}$ fatty acid at a weight ratio of about 37:7:56, weight-average molecular weight: approximately 570

- Tri-CL oil fatty acid glyceride, product of NOF Corp.

  Glycerin and fatty acid triester with $C_8$ fatty acid:$C_{12}$ fatty acid at a weight ratio of about 44:56, weight-average molecular weight: approximately 570

- PANACET 810s, product of NOF Corp.

  Glycerin and fatty acid triester with $C_8$ fatty acid:$C_{10}$ fatty acid at a weight ratio of about 85:15, weight-average molecular weight: approximately 480

- PANACET 800, product of NOF Corp.

  Glycerin and fatty acid triester with octanoic acid ($C_8$) as the entire fatty acid portion, weight-average molecular weight: approximately 470

- PANACET 800B, product of NOF Corp.

  Glycerin and fatty acid triester with 2-ethylhexanoic acid ($C_8$) as the entire fatty acid portion, weight-average molecular weight: approximately 470

- NA36, product of NOF Corp.

  Glycerin and fatty acid triester with $C_{16}$ fatty acid:$C_{18}$ fatty acid:$C_{20}$ fatty acid (including both saturated fatty acids and unsaturated fatty acids) at a weight ratio of about 5:92:3, weight-average molecular weight: approximately 880

- Tri-coconut fatty acid glyceride, product of NOF Corp.

  Glycerin and fatty acid triester with $C_8$ fatty acid:$C_{10}$ fatty acid:$C_{12}$ fatty acid:$C_{14}$ fatty acid:$C_{16}$ fatty acid (including both saturated fatty acids and unsaturated fatty acids) at a weight ratio of about 4:8:60:25:3, weight-average molecular weight: 670

- Caprylic acid diglyceride, product of NOF Corp.

  Glycerin and fatty acid diester with octanoic acid as the fatty acid, weight-average molecular weight: approximately 340

[($a_3$) Ester of a chain hydrocarbon diol and at least one fatty acid]

- UNISTAR H-208BRS, product of NOF Corp.

  Neopentyl glycol di(2-ethylhexanoate), weight-average molecular weight: approximately 360

- COMPOL BL, product of NOF Corp.

  Dodecanoic acid ($C_{12}$) monoester of butylene glycol, weight-average molecular weight: approximately 270

- COMPOL BS, product of NOF Corp.

  Octadecanoic acid ($C_{18}$) monoester of butylene glycol, weight-average molecular weight: approximately 350

[($c_2$) Ester of a chain hydrocarbon tricarboxylic acid, hydroxy acid, alkoxy acid or oxoacid with 3 carboxyl groups, and at least one aliphatic monohydric alcohol]

- Tributyl O-acetylcitrate, product of Tokyo Kasei Kogyo Co., Ltd.

  Weight-average molecular weight: approximately 400

- Tributyl citrate, product of Tokyo Kasei Kogyo Co., Ltd.

  Weight-average molecular weight: approximately 360

[($c_3$) Ester of a chain hydrocarbon dicarboxylic acid, hydroxy acid, alkoxy acid or oxoacid with 2 carboxyl groups, and at least one aliphatic monohydric alcohol]

- Dioctyl adipate, product of Wako Pure Chemical Industries, Ltd.

    Weight-average molecular weight: approximately 380

[($d_3$) Esters of a fatty acid and aliphatic monohydric alcohol]

- ELECTOL WE20, product of NOF Corp.

    Ester of dodecanoic acid ($C_{12}$) and dodecyl alcohol ($C_{12}$), weight-average molecular weight: approximately 360

- ELECTOL WE40, product of NOF Corp.

    Ester of tetradecanoic acid ($C_{14}$) and dodecyl alcohol ($C_{12}$), weight-average molecular weight: approximately 390

[($e_1$) Polyoxy $C_3$-$C_6$ alkylene glycol]

- UNIOL PB500, product of NOF Corp.

    Polybutylene glycol, weight-average molecular weight: approximately 500,

- UNIOL PB700, product of NOF Corp.

    Polyoxybutylene polyoxypropylene glycol, weight-average molecular weight: approximately 700

[($f_1$) Chain alkane]

- PARLEAM 6, product of NOF Corp.

    Branched chain hydrocarbon, produced by copolymerization of liquid isoparaffin, isobutene and n-butene followed by hydrogen addition, polymerization degree: approximately 5-10, weight-average molecular weight: approximately 330

[Other materials]

- NA50, product of NOF Corp.

    Glycerin and fatty acid triester obtained by addition of hydrogen to NA36 for reduced proportion of double bonds from unsaturated fatty acid starting material, weight-average molecular weight: approximately 880

- (Caprylic acid/capric acid) monoglyceride, product of NOF Corp.

    Glycerin and fatty acid monoester, with octanoic acid ($C_8$) and decanoic acid ($C_{10}$) at a weight ratio of about 85:15, weight-average molecular weight: approximately 220

- Monomuls 90-L2 lauric acid monoglyceride, product of Cognis Japan

- Isopropyl citrate, product of Tokyo Kasei Kogyo Co., Ltd.
  Weight-average molecular weight: approximately 230
- Diisostearyl malate

  Weight-average molecular weight: approximately 640

- UNIOL PB1000R, product of NOF Corp.

  Polybutylene glycol, weight-average molecular weight: approximately 1,000,

- UNIOL D-250, product of NOF Corp.

  Polypropylene glycol, weight-average molecular weight: approximately 250

- UNIOL D-400, product of NOF Corp.

  Polypropylene glycol, weight-average molecular weight: approximately 400

- UNIOL D-700, product of NOF Corp.

  Polypropylene glycol, weight-average molecular weight: approximately 700

- UNIOL D-1000, product of NOF Corp.

  Polypropylene glycol, weight-average molecular weight: approximately 1,000

- UNIOL D-1200, product of NOF Corp.

  Polypropylene glycol, weight-average molecular weight: approximately 1,160

- UNIOL D-2000, product of NOF Corp.

  Polypropylene glycol, weight-average molecular weight: approximately 2,030

- UNIOL D-3000, product of NOF Corp.

  Polypropylene glycol, weight-average molecular weight: approximately 3,000

- UNIOL D-4000, product of NOF Corp.

  Polypropylene glycol, weight-average molecular weight: approximately 4,000

- PEG 1500, product of NOF Corp.

  Polyethylene glycol, weight-average molecular weight: approximately 1,500-1,600

- WILBRITE cp9, product of NOF Corp.

  Polybutylene glycol compound with OH groups at both ends esterified by hexadecanoic acid ($C_{16}$), weight-average molecular weight: approximately 1,150

- UNILUBE MS-70K, product of NOF Corp.

  Stearyl ether of polypropylene glycol, approximately 15 repeating units, weight-average molecular weight: approximately 1,140

- NONION S-6, product of NOF Corp.

Polyoxyethylene monostearate, approximately 7 repeating units, weight-average molecular weight: approximately 880

- UNILUBE 5TP-300KB

Polyoxyethylenepolyoxypropylene pentaerythritol ether, produced by addition of 5 mol of ethylene oxide and 65 mol of propylene oxide to 1 mol of pentaerythritol, weight-average molecular weight: 4,130

- WILBRITE s753, product of NOF Corp.

Polyoxyethylene polyoxypropylene polyoxybutylene glycerin, weight-average molecular weight: approximately 960

- UNIOL TG-330, product of NOF Corp.

Glyceryl ether of polypropylene glycol, approximately 6 repeating units, weight-average molecular weight: approximately 330

- UNIOL TG-1000, product of NOF Corp.

Glyceryl ether of polypropylene glycol, approximately 16 repeating units, weight-average molecular weight: approximately 1,000

- UNIOL TG-3000, product of NOF Corp.

Glyceryl ether of polypropylene glycol, approximately 16 repeating units, weight-average molecular weight: approximately 3,000

- UNIOL TG-4000, product of NOF Corp.

Glyceryl ether of polypropylene glycol, approximately 16 repeating units, weight-average molecular weight: approximately 4,000

- UNILUBE DGP-700, product of NOF Corp.

Diglyceryl ether of polypropylene glycol, approximately 9 repeating units, weight-average molecular weight: approximately 700

- UNIOX HC60, product of NOF Corp.

Polyoxyethylene hydrogenated castor oil, weight-average molecular weight: approximately 3,570

- Vaseline, product of Cognis Japan

Petroleum-derived hydrocarbon, semi-solid

[Example 1]

[Menstrual blood surface residue rate A, with absorption of large amount of blood]

A test was conducted to evaluate the absorption property of a sanitary napkin after one-time absorption of a large amount of blood.

There were prepared a top sheet, formed of a hydrophilic agent-treated air-through nonwoven fabric (composite fiber composed of polyester and polyethylene terephthalate, basis weight: 35 g/m$^2$), a second sheet, formed of an air-through nonwoven fabric (composite fiber composed of polyester and polyethylene terephthalate, basis weight: 30 g/m$^2$), an absorbent body comprising pulp (basis weight: 150 to 450 g/m$^2$, increased at the center section), an acrylic super-absorbent polymer (basis weight: 15 g/m$^2$) and tissue as a core wrap, a water-repellent agent-treated side sheet, and a back sheet composed of a polyethylene film.

The top sheet was a top sheet produced by the method described in Japanese Unexamined Patent Publication No. 2008-2034, having a ridge-furrow structure, with a ridge thickness of approximately 1.5 mm and a furrow thickness of approximately 0.4 mm, and the pitch of the ridge-furrow structure (ridge width + furrow width) was approximately 4 mm and open holes were formed in the furrows at an open area of approximately 15%.

UNISTAR H-408BRS (product of NOF Corp., tetraester of pentaerythritol and fatty acid) was selected as the blood slipping agent, and it was coated onto the skin contact surface (ridge-furrow side) of the top sheet from a control seam HMA gun at room temperature, to a basis weight of 5.0 g/$m^2$. With an electron microscope it was confirmed that the H-408BRS was adhering onto the fiber surfaces as fine particulates.

A back sheet, an absorbent body, a second sheet, and a top sheet with the ridge-furrow side facing upward, were stacked in that order to form sanitary napkin No.1-1.

Sanitary napkins No.1-2 to No.1-49 were produced, changing the blood slipping agent from UNISTAR H-408BRS to the ones listed in Table 2. Each blood slipping agent was used directly, when it was liquid at room temperature, or when the blood slipping agent was solid at room temperature it was heated to its melting point +20°C, and then a control seam HMA gun was used for atomization of the blood slipping agent and coating onto the skin contact surface of the top sheet to a basis weight of about 5 g/$m^2$.

The blood slipping agent was coated onto essentially the entire skin contact surface of the top sheet, and on both the ridges and furrows.

[Test methods]

After measuring the mass $W_2$ (g) of the top sheet (the mass of the top sheet before the test), an acrylic board with an opened hole (200 mm × 100 mm, 125 g, with a 40 mm × 10 mm hole opened at the center) was placed on the top sheet, at the center section in the lengthwise direction and widthwise direction of the absorbent article, and 4.0 g of horse EDTA blood at 37±1°C (obtained by adding ethylenediaminetetraacetic acid (hereunder, "EDTA") to horse blood to prevent coagulation) was dropped through the hole using a pipette.

After dropping the horse EDTA blood, the acrylic board was immediately removed, the top sheet was taken off, the mass $W_3$ (g) (mass of the top sheet after the test) was measured and the "surface residue rate A (mass%)" was calculated by the following formula.

$$\text{Surface residue rate A (mass\%)} = 100 \times [W_3\ (g) - W_2\ (g)]/4.0\ (g)$$

The tack on the skin contact surface of the top sheet was measured at 35°C, and evaluated on the following scale.

G: No tack
F: Slight tack
P: Tack

The surface residue rate A and tack of each absorbent article, and the properties of each blood slipping agent, are shown below in Table 2. Fig. 8 is an electron micrograph of the skin contact surface of a top sheet in a sanitary napkin wherein the top sheet comprises tri-C2L oil fatty acid glycerides.

Table 2

| No. | Blood slipping agent | Kinematic viscosity (mm$^2$/s, 40°C) | Water holding percentage (wt%) | Weight-average molecular weight | IOB | Melting point (°C) | Surface residue rate A (wt%) | Tack |
|---|---|---|---|---|---|---|---|---|
| 1-1 | H-408 BRS | 45 | 0.7 | 640 | 0.13 | <-5 | 0.8 | G |
| 1-2 | H-2408 BRS-22 | 22 | 0.8 | 520 | 0.18 | <-5 | 0.8 | G |
| 1-3 | Tri-C2L oil fatty acid glyceride | 20 | <1.0 | 570 | 0.27 | 37 | | G |
| 1-4 | Tri-CL oil fatty acid glyceride | 15 | <1.0 | 570 | 0.28 | 38 | | G |
| 1-5 | PANACET 810s | 9 | 0.3 | 480 | 0.32 | -5 | 0.8 | G |

(continued)

| No. | Blood slipping agent | Kinematic viscosity ($mm^2/s$, 40°C) | Water holding percentage (wt%) | Weight-average molecular weight | IOB | Melting point (°C) | Surface residue rate A (wt%) | Tack |
|---|---|---|---|---|---|---|---|---|
| 1-6 | PANACET 800 | 15 | 0.5 | 470 | 0.33 | -5 | 1.8 | G |
| 1-7 | PANACET 800B | 20 | <1.0 | 470 | 0.33 | -5 | | G |
| 1-8 | NA36 | 40 | <1.0 | 880 | 0.16 | 37 | | G |
| 1-9 | Tri-coconut oil fatty acid glyceride | 25 | <1.0 | 670 | 0.28 | 30 | | G |
| 1-10 | Caprylic acid diglyceride | 25 | 2.7 | 340 | 0.58 | <45 | 1.0 | G |
| 1-11 | UNISTAR H-208BRS | 8 | 0.7 | 360 | 0.24 | <-5 | 0.5 | G |
| 1-12 | COMPOL BL | 10 | 1.6 | 270 | 0.50 | 2 | 1.3 | G |
| 1-13 | COMPOL BS | 35 | 0.3 | 350 | 0.36 | 37 | 2.5 | G |
| 1-14 | Tributyl O-acetylcitrate | 15 | 0.9 | 400 | 0.60 | <45 | 0.5 | G |
| 1-15 | Tributyl citrate | 12 | 0.6 | 360 | 0.78 | <45 | 1.8 | G |
| 1-16 | Dioctyl adipate | 7 | 0.4 | 380 | 0.27 | <45 | 1.5 | G |
| 1-17 | ELECTOL WE20 | 10 | 0.3 | 360 | 0.13 | 29 | 0.5 | G |
| 1-18 | ELECTOL WE40 | 15 | 0.5 | 390 | 0.12 | 37 | 2.3 | G |
| 1-19 | UNIOL PB500 | 40 | 3.6 | 500 | 0.44 | <45 | 2.5 | G |
| 1-20 | UNIOL PB700 | 50 | 2.3 | 700 | 0.49 | -5 | 1.3 | G |
| 1-21 | PARLEAM 6 | 5 | 0.06 | 330 | 0.00 | -5 | 2.0 | G |
| No. | Blood slipping agent | Kinematic viscosity ($mm2/^s$, 40°C) | Water holding percentage (mass%) | Weight-average molecular weight | IOB | Melting point (°C) | Surface residue rate A (mass%) | Tack |
| 1-22 | NA50 | 80<< | -* | 880 | 0.18 | 52 | 4.3 | G |
| 1-23 | (Caprylic acid/ capric acid) monoglyceride | 70 | 4.0<< | 220 | 1.15 | <45 | 5.0 | G |
| 1-24 | 90-L2 lauric acid monoglyceride | 80<< | 4.0<< | <1,000 | 0.87 | 58 | 5.0 | G |
| 1-25 | Isopropyl citrate | 120 | 4.0<< | 230 | 1.56 | <45 | 4.8 | F |
| 1-26 | Diisostearyl malate | 450 | 4.0<< | 640 | 0.28 | <45 | 3.3 | F |
| 1-27 | UNIOL PB1000R | 70 | 5.5 | 1000 | 0.40 | <45 | 2.5 | F |
| 1-28 | UNIOL D-250 | 20 | 4.0<< | 250 | | <45 | 3.8 | G |
| 1-29 | UNIOL D-400 | 30 | 4.0<< | 400 | 0.76 | <45 | 4.8 | G |
| 1-30 | UNIOL D-700 | 50 | 34.6 | 700 | 0.58 | <45 | 4.8 | G |
| 1-31 | UNIOL D-1000 | 70 | 26.7 | 1,000 | 0.51 | <45 | 3.8 | F |

(continued)

| No. | Blood slipping agent | Kinematic viscosity (mm2/s, 40°C) | Water holding percentage (mass%) | Weight-average molecular weight | IOB | Melting point (°C) | Surface residue rate A (mass%) | Tack |
|---|---|---|---|---|---|---|---|---|
| 1-32 | UNIOL D-1200 | 90 | 16.2 | 1,160 | 0.48 | <45 | 3.0 | F |
| 1-33 | UNIOL D-2000 | 160 | | 2,030 | | <45 | | P |
| 1-34 | UNIOL D-3000 | | 0.6 | 3,000 | 0.39 | <45 | 3.0 | P |
| 1-35 | UNIOL D-4000 | 450 | 0.5 | 4,000 | 0.38 | <45 | 2.5 | P |
| 1-36 | PEG 1500 | 120 | 4.0<< | 1,500-1,600 | 0.78 | 40 | 5.5 | P |
| 1-37 | WILBRITE CP9 | 120 | 0.6 | 1,150 | 0.21 | 35 | 6.8 | P |
| 1-38 | UNILUBE MS-70K | 50 | 2.8 | 1,140 | 0.30 | <-10 | 1.5 | F |
| 1-39 | NONION S-6 | 65 | 4.0<< | 880 | 0.44 | 37 | | G |
| 1-40 | UNILUBE 5TP-300KB | 310 | 3.9 | 4,130 | 0.39 | <45 | 2.0 | P |
| 1-41 | WILBRITE s753 | 120 | 27.3 | 960 | 0.67 | -5 | 3.5 | F |
| 1-42 | UNIOL TG-330 | 30 | | 330 | 1.27 | <45 | | G |
| 1-43 | UNIOL TG-1000 | 100 | 21.2 | 1,000 | 0.61 | <45 | 3.5 | G |
| 1-44 | UNIOL TG-3000 | 230 | 4.3 | 3,000 | 0.42 | <45 | 1.0 | P |
| 1-45 | UNIOL TG-4000 | 300 | 2.4 | 4,000 | 0.40 | <45 | 2.0 | P |
| 1-46 | UNILUBE DGP-700 | 200 | 4.0<< | 700 | 0.91 | <0 | 3.5 | F |
| 1-47 | UNIOX HC60 | 1150 | | 3,570 | 0.46 | 33 | | P |
| 1-48 | Vaseline | 80<< | 0.0 | <1,000 | 0.00 | 55 | 4.0 | P |
| 1-49 | None | - | - | - | - | - | 7.5 | G |
| *High viscosity, unmeasurable. | | | | | | | | |

With sanitary napkin No.1-49, which had no blood slipping agent, the surface residue rate A was 7.5 mass%, but with sanitary napkins No.1-1 to No.1-21 wherein the kinematic viscosity and water holding percentage were within the prescribed ranges, the surface residue rate A was 2.5 mass% or lower.

With sanitary napkins No.1-1 to No.1-21, it was observed that the horse EDTA blood that was dropped onto the ridges of the top sheet slid down from the ridges into the furrows, and was rapidly absorbed from the furrows into the absorbent body. However, with sanitary napkin No.1-49 which had no blood slipping agent, the dropped horse EDTA blood did not slip down into the furrows but slowly dripped down into the furrows, most of it remaining on the ridges of the top sheet. Also, with the absorbent articles with high a water holding percentage, as with No.1-30, for example, the horse EDTA blood that was dropped onto the ridges of the top sheet did not slip down into the furrows but slowly dripped while partially remaining on the top sheet, and a portion thereof remained on the ridges.

This suggests that sanitary napkins No.1-1 to No.1-21 allow rapid migration of menstrual blood from the top sheet into the absorbent body, when a large amount of menstrual blood has reached the top sheet at once.

Next, several volunteer subjects were asked to wear sanitary napkins Nos. 1-1 to 1-49, and most of the obtained responses indicated that with the sanitary napkins comprising blood slipping agents Nos. 1-1 to 1-21, the top sheets had no sticky feel and the top sheets were smooth, even after absorption of menstrual blood.

[Example 2]

[Menstrual blood surface residue rate B, with absorption of small amount of blood]

A test was conducted to evaluate the absorption property of a sanitary napkin after absorption of a small amount of blood.

There were prepared a top sheet, formed of a hydrophilic agent-treated air-through nonwoven fabric (composite fiber composed of polyester and polyethylene terephthalate, basis weight: 35 g/$m^2$) (hereunder also referred to as "top sheet with ridge-furrows"), a second sheet formed of an air-through nonwoven fabric (composite fibers composed of polyester and polyethylene terephthalate, basis weight: 30 g/$m^2$), an absorbent body comprising pulp (basis weight: 150 to 450 g/$m^2$, increased at the center section), an acrylic super-absorbent polymer (basis weight: 15 g/$m^2$) and tissue as a core wrap, a water-repellent agent-treated side sheet, and a back sheet composed of a polyethylene film.

The top sheet was a top sheet produced by the method described in Japanese Unexamined Patent Publication No. 2008-2034, having a ridge-furrow structure, with a ridge thickness of approximately 1.5 mm and a furrow thickness of approximately 0.4 mm, and the pitch of the ridge-furrow structure (ridge width + furrow width) was approximately 4 mm and open holes were formed in the furrows at an open area of approximately 15%.

UNISTAR H-408BRS (product of NOF Corp., tetraester of pentaerythritol and fatty acid) was selected as the blood slipping agent, and it was coated onto the skin contact surface (ridge-furrow side) of the top sheet from a control seam HMA gun at room temperature, to a basis weight of 5.0 g/$m^2$. With an electron microscope it was confirmed that the H-408BRS was adhering onto the fiber surfaces as fine particulates.

A back sheet, an absorbent body, a second sheet, and a top sheet with the ridge-furrow side facing upward, were stacked in that order to form sanitary napkin No.2-1(i).

A sanitary napkin No.2-1(ii) was formed in the same manner as the sanitary napkin No.2-1(i), except that the top sheet was changed to a top sheet formed of a flat hydrophilic agent-treated air-through nonwoven fabric (composite fiber composed of polyester and polyethylene terephthalate, basis weight: 35 g/$m^2$), without a ridge-furrow structure (hereunder also referred to as "flat top sheet").

Sanitary napkins No.2-2(i) to No.2-11(i) and No.2-2(ii) to No.2-11(ii) were produced, changing the blood slipping agent from UNISTAR H-408BRS to the ones listed in Table 3. Each blood slipping agent was used directly, when it was liquid at room temperature, or when the blood slipping agent was solid at room temperature it was heated to its melting point +20°C, and then a control seam HMA gun was used for atomization of the blood slipping agent and coating onto the skin contact surface of the top sheet to a basis weight of about 5 g/$m^2$.

The blood slipping agent was coated over essentially the entire skin contact surface of the top sheet, and on both the ridges and furrows of the top sheets with a ridge-furrow structure.

[Test methods]

After measuring the mass $W_4$ (g) of the top sheet (the mass of the top sheet before the test), approximately 0.25 g (2 drops) of horse EDTA blood at 37±1°C was added dropwise through a pipette, on the top sheet at the center in the lengthwise direction and widthwise direction of the absorbent article. The horse EDTA blood was dropped onto the top parts of the ridges, in the top sheets with ridge-furrows.

At 30 seconds after dropping, the top sheet was taken off, the mass $W_5$ (g) (mass of top sheet after the test) was measured and the "surface residue rate B (mass%)" was calculated by the following formula.

$$\text{Surface residue rate B (mass\%)} = 100 \times (W_5\ (g) - W_4\ (g))/W_6\ (g)$$

$W_6$ (g) is the mass of the dropped horse EDTA blood, calculated from the mass of the pipette before and after dropping.

The results are shown in Table 3 below.

Table 3

| No. | Blood slipping agent | Surface residue rate B (mass%) | |
|---|---|---|---|
| | | Top sheet with ridge-furrows | Flat top sheet |
| 2-1 | H-408 BRS | 4% | 32% |
| 2-2 | PANACET 810S | 8% | 40% |

(continued)

| No. | Blood slipping agent | Surface residue rate B (mass%) | |
|---|---|---|---|
| | | Top sheet with ridge-furrows | Flat top sheet |
| 2-3 | Capric acid diglyceride | 8% | 24% |
| 2-4 | COMPOL BL | 4% | 32% |
| 2-5 | Tributyl O-acetylcitrate | 8% | 44% |
| 2-6 | Dioctyl adipate | 8 % | 32% |
| 2-7 | ELECTOL WE40 | 8% | 24% |
| 2-8 | UNIOL PB500 | 4% | 68% |
| 2-9 | PARLEAM 6 | 4% | 100% |
| 2-10 | UNIOL D-250 | 16% | 48% |
| 2-11 | None | 28% | 28% |

Table 3 shows that when the blood slipping agent was H-408BRS, PANACET 810S, capric acid diglyceride, COMPOL BL, tributyl O-acetylcitrate, dioctyl adipate, ELECTOL WE40, UNIOL PB500 or PARLEAM 6, the surface residue rate B of the top sheet with ridge-furrows was low. This suggests that blood slipping agents having the prescribed properties cause rapid migration of small amounts of blood from the ridges to the furrows and into the absorbent body.

[Example 3]

[Viscosity of blood containing blood slipping agent]

The viscosity of the blood slipping agent-containing blood was measured using a Rheometric Expansion System ARES (Rheometric Scientific, Inc.). After adding 2 mass% of PANACET 810s to horse defibrinated blood, the mixture was gently agitated to form a sample, the sample was placed on a 50 mm-diameter parallel plate, with a gap of 100 $\mu$m, and the viscosity was measured at 37$\pm$0.5°C. The sample was not subjected to a uniform shear rate, due to the parallel plate, but the average shear rate indicated by the device was 10 $s^{-1}$.

The viscosity of the horse defibrinated blood containing 2 mass% PANACET 810s was 5.9 mPa·s, while the viscosity of the horse defibrinated blood containing no blood slipping agent was 50.4 mPa·s. Thus, the horse defibrinated blood containing 2 mass% PANACET 810s clearly had an approximately 90% lower viscosity than the blood containing no blood slipping agent.

It is known that blood contains components, such as blood cells and has a thixotropic nature, and it is believed that the blood slipping agent of the present disclosure has an effect of lowering the viscosity of blood, such as menstrual blood in the low viscosity range. Lowering the blood viscosity presumably allows absorbed menstrual blood to more easily migrate rapidly from the top sheet to the absorbent body.

[Example 4]

[Photomicrograph of blood slipping agent-containing blood]

Menstrual blood was sampled from healthy volunteers onto food storage wrap film, and PANACET 810s dispersed in a 10-fold mass of phosphate-buffered saline was added to a portion thereof to a PANACET 810s concentration of 1 mass%. The menstrual blood was dropped onto a slide glass, a cover glass was placed thereover, and the state of the erythrocytes was observed with an optical microscope. A photomicrograph of menstrual blood containing no blood slipping agent is shown in Fig. 9(a), and a photomicrograph of menstrual blood containing PANACET 810s is shown in Fig. 9(b).

As shown in Fig. 9, the erythrocytes formed aggregates, including a rouleaux structure, in the menstrual blood containing no blood slipping agent, while the erythrocytes were stably dispersed in the menstrual blood containing PANACET 810s. This suggests that the blood slipping agent has the function of stabilizing erythrocytes in blood.

[Example 5]

[Surface tension of blood containing blood slipping agent]

The surface tension of blood containing a blood slipping agent was measured by the pendant drop method, using a Drop Master500 contact angle meter by Kyowa Interface Science Co., Ltd. The surface tension was measured after adding a prescribed amount of blood slipping agent to sheep defibrinated blood, and thoroughly shaking.

The measurement was accomplished automatically with the apparatus, and the surface tension γ was determined by the following formula (see Fig. 10).

$$\gamma = g \times \rho \times (de)^2 \times 1/H$$

g: Gravitational constant
1/H: Correction factor determined from ds/de
p: Density
de: Maximum diameter
ds: Diameter at location of increase by de from dropping edge

The density ρ was measured at the temperatures listed in Table 4, according to JIS K 2249-1995, "Density test methods and density/mass/volume conversion tables", 5. Vibrating density test method.

The measurement was accomplished using a DA-505 by Kyoto Electronics Co., Ltd.

The results are shown in Table 4 below.

Table 4

| No. | Blood slipping agent | | Measuring temperature (°C) | Surface tension (mN/m) |
|---|---|---|---|---|
| | Type | Amount (mass%) | | |
| 1 | - | - | 35 | 62.1 |
| 2 | PANACET 810s | 0.01 | 35 | 61.5 |
| 3 | | 0.05 | 35 | 58.2 |
| 4 | | 0.10 | 35 | 51.2 |
| 5 | ELECTOL WE20 | 0.10 | 35 | 58.8 |
| 6 | PARLEAM 6 | 0.10 | 35 | 57.5 |
| 7 | - | - | 50 | 56.3 |
| 8 | WILBRITE cp9 | 0.10 | 50 | 49.1 |

Based on Table 4 it is seen that the blood slipping agent has an effect of lowering the surface tension of blood.

Lowering the surface tension of blood presumably allows absorbed blood to rapidly migrate from the top sheet to the absorbent body, without being retained between the top sheet fibers.

Reference Signs List

1, 1A Absorbent articles
2, 2A Top sheets
3, 3A Back sheets
4, 4A Absorbent bodies
5, 5A Diffusion sheets
6, 6A Cover sheets
7 Side sheet
8 Pressure-sensitive adhesive section
11 Body section

12 Wing section
13A Compressed section
14A Domed section
15A Perimeter section
16A Cushion section
17A Elastic member
141A Center section of domed section
142A Peripheral section of domed section

## Claims

1. An absorbent article (1A) comprising:

   a liquid-permeable top sheet (2A) provided on the skin side,
   a liquid-impermeable back sheet (3A) provided on the clothing side,
   a liquid-retaining absorbent body (4A) provided between the top sheet and the back sheet,
   a first sheet (5A) provided between the top sheet and the absorbent body and provided in the region in which the absorbent body is provided, and
   a second sheet (6A) provided between the first sheet and the absorbent body,
   wherein the first sheet (5A) has a liquid-permeable as well as a liquid-retaining property,
   the second sheet (6A) has a liquid-permeable property but no liquid-retaining property, and conceals the redness of menstrual blood absorbed into the absorbent body (4A),
   the absorbent article has a domed section (14A) that protrudes in a thickness direction of the absorbent article,
   the domed section (14A) comprises a cushion section (16A) between the first sheet (5A) and the second sheet (6A), the cushion section has a maximum thickness of 3 to 30 mm, the cushion section contains multiple fibers, and the intersections between the multiple fibers are heat-fused, and
   the domed section (14A) contains a blood slipping agent having a kinematic viscosity of 0.01 to 80 $mm^2/s$ at 40°C as described in the description, a water holding percentage of 0.01 to 4.0 mass% as described in the description, and a weight-average molecular weight of less than 1,000 as described in the description.

2. The absorbent article according to claim 1, wherein the region in which the first sheet (5A) is provided includes an excretory opening contact region.

3. The absorbent article according to claim 1 or 2, wherein the first sheet (5A) contains cellulose-based fibers at 50 mass% or greater.

4. The absorbent article according to any one of claims 1 to 3, wherein the second sheet (6A) contains thermoplastic chemical fibers at 50 mass% or greater.

5. The absorbent article according to any one of claims 1 to 4, wherein the proportion of the area of the first sheet (5A) with respect to the area of the absorbent body (4A) is 10% to 90%.

6. The absorbent article according to any one of claims 1 to 5, wherein the region in which the second sheet (6A) is provided includes the region in which the first sheet (5A) is provided,
   the ratio of the area of the second sheet (6A) with respect to the area of the first sheet (5A) being 110% or greater and the ratio of the area of the second sheet (6A) with respect to the area of the absorbent body (4A) being 150% or less.

7. The absorbent article according to any one of claims 1 to 6, wherein the blood slipping agent also has an IOB of 0.00 to 0.60 as described in the description.

8. The absorbent article according to any one of claims 1 to 7, wherein the blood slipping agent is selected from the group consisting of the following items (i)-(iii), and any combination thereof:

   (i) a hydrocarbon;
   (ii) a compound having (ii-1) a hydrocarbon moiety, and (ii-2) one or more, same or different groups selected from the group consisting of carbonyl group (-CO-) and oxy group (-0-) inserted between a C-C single bond of

the hydrocarbon moiety; and

(iii) a compound having (iii-1) a hydrocarbon moiety, (iii-2) one or more, same or different groups selected from the group consisting of carbonyl group (-CO-) and oxy group (-0-) inserted between a C-C single bond of the hydrocarbon moiety, and (iii-3) one or more, same or different groups selected from the group consisting of carboxyl group (-COOH) and hydroxyl group (-OH) substituting a hydrogen of the hydrocarbon moiety;

with the proviso that when 2 or more oxy groups are inserted in the compound of (ii) or (iii), the oxy groups are not adjacent.

**9.** The absorbent article according to any one of claims 1 to 8, wherein the blood slipping agent is selected from the group consisting of the following items (i')-(iii'), and any combination thereof:

(i') a hydrocarbon;

(ii') a compound having (ii'-1) a hydrocarbon moiety, and (ii'-2) one or more, same or different bonds selected from the group consisting of carbonyl bond (-CO-), ester bond (-COO-), carbonate bond (-OCOO-), and ether bond (-O-) inserted between a C-C single bond of the hydrocarbon moiety; and

(iii') a compound having (iii'-1) a hydrocarbon moiety, (iii'-2) one or more, same or different bonds selected from the group consisting of carbonyl bond (-CO-), ester bond (-COO-), carbonate bond (-OCOO-), and ether bond (-O-) inserted between a C-C single bond of the hydrocarbon moiety, and (iii'-3) one or more, same or different groups selected from the group consisting of carboxyl group (-COOH) and hydroxyl group (-OH) substituting a hydrogen on the hydrocarbon moiety;

with the proviso that when 2 or more same or different bonds are inserted in the compound of (ii') or (iii'), the bonds are not adjacent.

**10.** The absorbent article according to any one of claims 1 to 9, wherein the blood slipping agent is selected from the group consisting of the following items (A)-(F), and any combination thereof:

(A) an ester of (A1) a compound having a chain hydrocarbon moiety and 2-4 hydroxyl groups substituting hydrogens on the chain hydrocarbon moiety, and (A2) a compound having a chain hydrocarbon moiety and 1 carboxyl group substituting a hydrogen on the chain hydrocarbon moiety;

(B) an ether of (B1) a compound having a chain hydrocarbon moiety and 2-4 hydroxyl groups substituting hydrogens on the chain hydrocarbon moiety and (B2) a compound having a chain hydrocarbon moiety and 1 hydroxyl group substituting a hydrogen on the chain hydrocarbon moiety;

(C) an ester of (C1) a carboxylic acid, hydroxy acid, alkoxy acid or oxoacid comprising a chain hydrocarbon moiety and 2-4 carboxyl groups substituting hydrogens on the chain hydrocarbon moiety and (C2) a compound having a chain hydrocarbon moiety and 1 hydroxyl group substituting a hydrogen on the chain hydrocarbon moiety;

(D) a compound having a chain hydrocarbon moiety and one bond selected from the group consisting of an ether bond (-0-), carbonyl bond (-CO-), ester bond (-COO-) and carbonate bond (-OCOO-) inserted between a C-C single bond of the chain hydrocarbon moiety;

(E) a polyoxy $C_3$-$C_6$ alkylene glycol, or alkyl ester or alkyl ether thereof; and

(F) a chain hydrocarbon.

**11.** The absorbent article according to any one of claims 1 to 10, wherein the blood slipping agent is selected from the group consisting of ($a_1$) an ester of a chain hydrocarbon tetraol and at least one fatty acid, ($a_2$) an ester of a chain hydrocarbon triol and at least one fatty acid, ($a_3$) an ester of a chain hydrocarbon diol and at least one fatty acid, ($b_1$) an ether of a chain hydrocarbon tetraol and at least one aliphatic monohydric alcohol, ($b_2$) an ether of a chain hydrocarbon triol and at least one aliphatic monohydric alcohol, ($b_3$) an ether of a chain hydrocarbon diol and at least one aliphatic monohydric alcohol, ($c_1$) an ester of a chain hydrocarbon tetracarboxylic acid, hydroxy acid, alkoxy acid or oxoacid with 4 carboxyl groups, and at least one aliphatic monohydric alcohol, ($c_2$) an ester of a chain hydrocarbon tricarboxylic acid, hydroxy acid, alkoxy acid or oxoacid with 3 carboxyl groups, and at least one aliphatic monohydric alcohol, ($c_3$) an ester of a chain hydrocarbon dicarboxylic acid, hydroxy acid, alkoxy acid or oxoacid with 2 carboxyl groups, and at least one aliphatic monohydric alcohol, ($d_1$) an ether of an aliphatic monohydric alcohol and an aliphatic monohydric alcohol, ($d_2$) a dialkyl ketone, ($d_3$) an ester of a fatty acid and an aliphatic monohydric alcohol, ($d_4$) a dialkyl carbonate, ($e_1$) a polyoxy $C_3$-$C_6$ alkylene glycol, ($e_2$) an ester of a polyoxy $C_3$-$C_6$ alkylene glycol and at least one fatty acid, ($e_3$) an ether of a polyoxy $C_3$-$C_6$ alkylene glycol and at least one aliphatic monohydric alcohol, and ($f_1$) a chain alkane, and any combination thereof.

12. The absorbent article according to any one of claims 1 to 11, wherein the blood slipping agent has a vapor pressure of 0.00 to 0.01 Pa at 1 atmosphere, 40°C.

## Patentansprüche

1. Absorbierender Artikel (1A), der Folgendes umfasst:

   eine flüssigkeitsdurchlässige obere Lage (2A), die auf der Hautseite bereitgestellt ist,
   eine flüssigkeitsundurchlässige hintere Lage (3A), die auf der Kleidungsseite bereitgestellt ist,
   einen Flüssigkeit zurückhaltenden Saugkörper (4A), der zwischen der oberen Lage und der hinteren Lage bereitgestellt ist,
   eine erste Lage (5A), die zwischen der oberen Lage und dem Saugkörper bereitgestellt ist und in dem Bereich bereitgestellt ist, in dem der Saugkörper bereitgestellt ist, und
   eine zweite Lage (6A), die zwischen der ersten Lage und dem Saugkörper bereitgestellt ist,
   wobei die erste Lage (5A) eine flüssigkeitsdurchlässige sowie eine Flüssigkeit zurückhaltende Eigenschaft aufweist,
   die zweite Lage (6A) eine flüssigkeitsdurchlässige Eigenschaft, doch keine Flüssigkeit zurückhaltende Eigenschaft aufweist und die Rötung von Menstruationsblut verbirgt, das in den Saugkörper (4A) absorbiert wurde,
   der absorbierende Artikel einen gewölbten Abschnitt (14A) aufweist, der in einer Dickenrichtung des absorbierenden Artikels hervorsteht, wobei der gewölbte Abschnitt (14A) einen gepolsterten Abschnitt (16A) zwischen der ersten Lage (5A) und der zweiten Lage (6A) umfasst, der gepolsterte Abschnitt eine maximale Dicke von 3 bis 30 mm aufweist, der gepolsterte Abschnitt zahlreiche Fasern enthält und die Kreuzungspunkte zwischen den zahlreichen Fasern wärmegeschmolzen sind, und
   der gewölbte Abschnitt (14A) ein Blut-Gleitmittel enthält, das Folgendes aufweist: eine kinematische Viskosität von 0,01 bis 80 $mm^2/s$ bei 40 °C, wie in der Beschreibung beschrieben, einen Wasseraufnahme-Prozentsatz von 0,01 bis 4,0 Masse-%, wie in der Beschreibung beschrieben, und ein gewichtsmittleres Molekulargewicht von weniger als 1 000, wie in der Beschreibung beschrieben.

2. Absorbierender Artikel nach Anspruch 1, wobei der Bereich, in dem die erste Lage (5A) bereitgestellt ist, einen Ausscheidungsöffnungskontaktbereich einschließt.

3. Absorbierender Artikel nach Anspruch 1 oder 2, wobei die erste Lage (5A) Cellulose-basierte Fasern zu 50 Masse-% oder größer enthält.

4. Absorbierender Artikel nach einem der Ansprüche 1 bis 3, wobei die zweite Lage (6A) thermoplastische Chemiefasern zu 50 Masse-% oder größer enthält.

5. Absorbierender Artikel nach einem der Ansprüche 1 bis 4, wobei der Anteil der Fläche der ersten Lage (5A) in Bezug auf die Fläche des Saugkörpers (4A) 10 % bis 90 % beträgt.

6. Absorbierender Artikel nach einem der Ansprüche 1 bis 5, wobei der Bereich, in dem die zweite Lage (6A) bereitgestellt ist, den Bereich einschließt, in dem die erste Lage (5A) bereitgestellt ist,
   wobei das Verhältnis der Fläche der zweiten Lage (6A) in Bezug auf die Fläche der ersten Lage (5A) 110 % oder mehr beträgt und das Verhältnis der Fläche der zweiten Lage (6A) in Bezug auf die Fläche des Saugkörpers (4A) 150 % oder weniger beträgt.

7. Absorbierender Artikel nach einem der Ansprüche 1 bis 6, wobei das Blut-Gleitmittel auch eine IOB von 0,00 bis 0,60 aufweist, wie in der Beschreibung beschrieben.

8. Absorbierender Artikel nach einem der Ansprüche 1 bis 7, wobei das Blut-Gleitmittel aus der Gruppe, die aus den folgenden Punkten (i)-(iii) besteht, und jedweder Kombination davon ausgewählt ist:

   (i) einem Kohlenwasserstoff;
   (ii) einer Verbindung, die Folgendes aufweist: (ii-1) eine Kohlenwasserstoffeinheit und (ii-2) eine oder mehrere, gleiche oder unterschiedliche Gruppen, die aus der Gruppe ausgewählt sind, die aus Carbonylgruppe (-CO-) und Oxygruppe (-O-) besteht, die zwischen eine C-C-Einfachbindung der Kohlenwasserstoffeinheit eingefügt sind; und

(iii) einer Verbindung, die Folgendes aufweist: (iii-1) eine Kohlenwasserstoffeinheit, (iii-2) eine oder mehrere, gleiche oder unterschiedliche Gruppen, die aus der Gruppe ausgewählt sind, die aus Carbonylgruppe (-CO-) und Oxygruppe (-O-) besteht, die zwischen eine C-C-Einfachbindung der Kohlenwasserstoffeinheit eingefügt sind, und (iii-3) eine oder mehrere, gleiche oder unterschiedliche Gruppen, die aus der Gruppe ausgewählt sind, die aus Carboxylgruppe (-COOH) und Hydroxylgruppe (-OH) besteht, die einen Wasserstoff der Kohlenwasserstoffeinheit substituieren;

mit der Maßgabe, dass bei der Einfügung von 2 oder mehr Oxygruppen in die Verbindung von (ii) oder (iii) die Oxygruppen nicht benachbart sind.

**9.** Absorbierender Artikel nach einem der Ansprüche 1 bis 8, wobei das Blut-Gleitmittel aus der Gruppe, die aus den folgenden Punkten (i')-(iii') besteht, und jedweder Kombination davon ausgewählt ist:

(i') einem Kohlenwasserstoff;
(ii') einer Verbindung, die Folgendes aufweist: (ii'-1) eine Kohlenwasserstoffeinheit und (ii'-2) eine oder mehrere, gleiche oder unterschiedliche Bindungen, die aus der Gruppe ausgewählt sind, die aus Folgenden besteht: Carbonylbindung (-CO-), Esterbindung (-COO-), Carbonatbindung (-OCOO-) und Etherbindung (-O-), die zwischen eine C-C-Einfachbindung der Kohlenwasserstoffeinheit eingefügt sind; und
(iii') einer Verbindung, die Folgendes aufweist: (iii'-1) eine Kohlenwasserstoffeinheit, (iii'-2) eine oder mehrere, gleiche oder unterschiedliche Bindungen, die aus der Gruppe ausgewählt sind, die aus Folgenden besteht: Carbonylbindung (-CO-), Esterbindung (-COO-), Carbonatbindung (-OCOO-) und Etherbindung (-O-), die zwischen eine C-C-Einfachbindung der Kohlenwasserstoffeinheit eingefügt sind, und (iii'-3) eine oder mehrere, gleiche oder unterschiedliche Gruppen, die aus der Gruppe ausgewählt sind, die aus Carboxylgruppe (-COOH) und Hydroxylgruppe (-OH) besteht, die einen Wasserstoff an der Kohlenwasserstoffeinheit substituieren;

mit der Maßgabe, dass bei der Einfügung von 2 oder mehr, gleichen oder unterschiedlichen Bindungen in die Verbindung von (ii') oder (iii') die Bindungen nicht benachbart sind.

**10.** Absorbierender Artikel nach einem der Ansprüche 1 bis 9, wobei das Blut-Gleitmittel aus der Gruppe, die aus den folgenden Punkten (A)-(F) besteht, und jedweder Kombination davon ausgewählt ist:

(A) einem Ester von (A1) einer Verbindung, die eine Kettenkohlenwasserstoffeinheit und 2-4 Hydroxylgruppen, die Wasserstoffe an der Kettenkohlenwasserstoffeinheit substituieren, aufweist, und (A2) einer Verbindung, die eine Kettenkohlenwasserstoffeinheit und eine Carboxylgruppe, die einen Wasserstoff an der Kettenkohlenwasserstoffeinheit substituiert, aufweist;
(B) einem Ether von (B1) einer Verbindung, die eine Kettenkohlenwasserstoffeinheit und 2-4 Hydroxylgruppen, die Wasserstoffe an der Kettenkohlenwasserstoffeinheit substituieren, aufweist, und (B2) einer Verbindung, die eine Kettenkohlenwasserstoffeinheit und eine Hydroxylgruppe, die einen Wasserstoff an der Kettenkohlenwasserstoffeinheit substituiert, aufweist;
(C) einem Ester von (C1) einer Carbonsäure, Hydroxysäure, Alkoxysäure oder Oxosäure, die eine Kettenkohlenwasserstoffeinheit und 2-4 Carboxylgruppen, die Wasserstoffe an der Kettenkohlenwasserstoffeinheit substituieren, umfassen, und (C2) einer Verbindung, die eine Kettenkohlenwasserstoffeinheit und eine Hydroxylgruppe, die einen Wasserstoff an der Kettenkohlenwasserstoffeinheit substituiert, aufweist;
(D) einer Verbindung, die Folgendes aufweist: eine Kettenkohlenwasserstoffeinheit und eine Bindung, die aus der Gruppe ausgewählt ist, die aus einer Etherbindung (-O-), Carbonylbindung (-CO-), Esterbindung (-COO-) und Carbonatbindung (-OCOO-) besteht, die zwischen eine C-C-Einfachbindung der Kettenkohlenwasserstoffeinheit eingefügt sind;
(E) einem Polyoxy-$C_3$-$C_6$-Alkylenglycol oder Alkylester oder Alkylether davon; und
(F) einem Kettenkohlenwasserstoff.

**11.** Absorbierender Artikel nach einem der Ansprüche 1 bis 10, wobei das Blut-Gleitmittel aus der Gruppe ausgewählt ist, die aus Folgenden besteht: ($a_1$) einem Ester eines Kettenkohlenwasserstofftetraols und mindestens einer Fettsäure, ($a_2$) einem Ester eines Kettenkohlenwasserstofftriols und mindestens einer Fettsäure, ($a_3$) einem Ester eines Kettenkohlenwasserstoffdiols und mindestens einer Fettsäure, ($b_1$) einem Ether eines Kettenkohlenwasserstofftetraols und mindestens eines aliphatischen einwertigen Alkohols, ($b_2$) einem Ether eines Kettenkohlenwasserstofftriols und mindestens eines aliphatischen einwertigen Alkohols, ($b_3$) einem Ether eines Kettenkohlenwasserstoffdiols und mindestens eines aliphatischen einwertigen Alkohols, ($c_1$) einem Ester einer Kettenkohlenwasserstofftetracarbonsäure, -hydroxysäure, -alkoxysäure oder -oxosäure mit 4 Carboxylgruppen und mindestens eines aliphatischen

einwertigen Alkohols, ($c_2$) einem Ester einer Kettenkohlenwasserstofftricarbonsäure, -hydroxysäure, -alkoxysäure oder -oxosäure mit 3 Carboxylgruppen und mindestens eines aliphatischen einwertigen Alkohols, ($c_3$) einem Ester einer Kettenkohlenwasserstoffdicarbonsäure, -hydroxysäure, -alkoxysäure oder -oxosäure mit 2 Carboxylgruppen und mindestens eines aliphatischen einwertigen Alkohols, ($d_1$) einem Ether eines aliphatischen einwertigen Alkohols und eines aliphatischen einwertigen Alkohols, ($d_2$) einem Dialkylketon, ($d_3$) einem Ester einer Fettsäure und eines aliphatischen einwertigen Alkohols, ($d_4$) einem Dialkylcarbonat, ($e_1$) einem Polyoxy-$C_3$-$C_6$-Alkylenglycol, ($e_2$) einem Ester eines Polyoxy-$C_3$-$C_6$-Alkylenglycols und mindestens einer Fettsäure, ($e_3$) einem Ether eines Polyoxy-$C_3$-$C_6$-Alkylenglycols und mindestens eines aliphatischen einwertigen Alkohols und ($f_1$) einem Kettenalkan, und jedweder Kombination davon.

**12.** Absorbierender Artikel nach einem der Ansprüche 1 bis 11, wobei das Blut-Gleitmittel einen Dampfdruck von 0,00 bis 0,01 Pa bei 1 Atmosphäre, 40 °C, aufweist.

## Revendications

**1.** Article absorbant (1A) comprenant :

une feuille supérieure perméable aux liquides (2A) pourvue du côté tourné vers la peau,
une feuille de support imperméable aux liquides (3A) pourvue du côté tourné vers les vêtements,
un corps absorbant de rétention des liquides (4A) pourvu entre la feuille supérieure et la feuille de support,
une première feuille (5A) pourvue entre la feuille supérieure et le corps absorbant et située dans la région dans laquelle le corps absorbant est pourvu, et
une deuxième feuille (6A) pourvue entre la première feuille et le corps absorbant,
dans lequel la première feuille (5A) possède une propriété perméable aux liquides ainsi qu'une propriété de rétention des liquides,
la deuxième feuille (6A) possède une propriété perméable aux liquides mais non pas une propriété de rétention de liquides, et elle dissimule la rougeur du sang menstruel absorbé dans le corps absorbant (4A),
l'article absorbant présente une section en dôme (14A) qui fait saillie dans une direction de l'épaisseur de l'article absorbant, la section en dôme (14A) comprenant une section de compression (16A) située entre la première feuille (5A) et la deuxième feuille (6A), la section de compression ayant une épaisseur maximale de 3 à 30 mm, la section de compression contenant des fibres multiples, et les intersections entre les fibres multiples sont fusionnées sous l'effet de la chaleur, et
la section en dôme (14A) contient un agent facilitant le glissement du sang ayant une viscosité cinématique dans la plage de 0,01 à 80 $mm^2/s$ à 40°C comme décrit dans la description, un pourcentage de rétention de l'eau dans la plage de 0,01 à 4,0 % en masse comme décrit dans la description et un poids moléculaire moyen en poids inférieur à 1.000 comme décrit dans la description.

**2.** Article absorbant selon la revendication 1, dans lequel la région dans laquelle la première feuille (5A) est pourvue comprend une région touchant l'orifice d'excrétion.

**3.** Article absorbant selon la revendication 1 ou la revendication 2, dans lequel la première feuille (5A) contient des fibres à base de cellulose d'une masse de 50 % ou supérieure.

**4.** Article absorbant selon l'une quelconque des revendications 1 à 3, dans lequel la deuxième feuille (6A) contient des fibres chimiques thermoplastiques d'une masse de 50 % ou supérieure.

**5.** Article absorbant selon l'une quelconque des revendications 1 à 4, dans lequel la proportion de la zone de la première feuille (5A) par rapport à la zone du corps absorbant (4A) est de 10 % à 90 %.

**6.** Article absorbant selon l'une quelconque des revendications 1 à 5, dans lequel la région dans laquelle la deuxième feuille (6A) est pourvue contient la région dans laquelle la première feuille (5A) est pourvue,
le rapport de la zone de la deuxième feuille (6A) par rapport à la zone de la première feuille (5A) étant de 110 % ou supérieur et le rapport de la zone de la deuxième feuille (6A) par rapport à la zone du corps absorbant (4A) étant de 150 % ou inférieur.

**7.** Article absorbant selon l'une quelconque des revendications 1 à 6, dans lequel l'agent facilitant le glissement du sang a en outre une valeur d'IOB qui va de 0,00 à 0,60 comme décrit dans la description.

8. Article absorbant selon l'une quelconque des revendications 1 à 7, dans lequel l'agent facilitant le glissement du sang est sélectionné dans le groupe constitué des composés (i)-(iii) suivants et de toute combinaison de ceux-ci :

(i) un hydrocarbure ;
(ii) un composé ayant (ii-1) un fragment hydrocarboné, et (ii-2) un ou plusieurs groupements, identiques ou différents, sélectionnés dans le groupe consistant en un groupement carbonyle (-CO-) et un groupement oxyle (-O-) insérés entre une simple liaison C-C du fragment hydrocarboné ; et
(iii) un composé ayant (iii-1) un fragment hydrocarboné, (iii-2) un ou plusieurs groupements, identiques ou différents, sélectionnés dans le groupe consistant en un groupement carbonyle (-CO-) et un groupement oxy (-O-) insérés entre une simple liaison C-C du fragment hydrocarboné, et (iii-3) un ou plusieurs groupements, identiques ou différents, sélectionnés dans le groupe consistant en un groupement carbonyle (-COOH) et un groupement hydroxyle (-OH) substituant un hydrogène du fragment hydrocarboné ;

à condition que quand un ou plusieurs groupements sont insérés dans le composé (ii) ou (iii), les groupements oxy ne sont pas adjacents.

9. Article absorbant selon l'une quelconque des revendications 1 à 8, où l'agent facilitant le glissement du sang est sélectionné dans le groupe consistant en les composés (i')-(iii') suivants et toute combinaison de ceux-ci ;

(i') un hydrocarbure ;
(ii') un composé ayant (ii'-1) un fragment hydrocarboné et (ii'-2) une ou plusieurs liaisons, identiques ou différentes, sélectionnées dans le groupe consistant en une liaison carbonyle (-CO-), une liaison ester (-COO-), une liaison carbonate (-OCOO-) et une liaison ester (-O-) insérées entre une simple liaison C-C du fragment hydrocarboné ; et
(iii') un composé ayant (iii'-1) un fragment hydrocarboné, (iii'-2) une ou plusieurs liaisons, identiques ou différentes, sélectionnées dans le groupe consistant en une liaison carbonyle (-CO-), une liaison ester (-COO-), une liaison carbonate (-OCOO-) et une liaison éther (-O-) insérées entre une simple liaison C-C du fragment hydrocarboné et (iii'-3) un ou plusieurs groupements, identiques ou différents, sélectionnés dans le groupe consistant en un groupement carboxyle (-COOH) et un groupement hydroxyle (-OH) substituant un hydrogène sur le fragment hydrocarboné ;

à condition que quand 2 ou plusieurs liaisons identiques ou différentes sont insérées dans le composé (ii') ou (iii'), les liaisons ne sont pas adjacentes.

10. Article absorbant selon l'une quelconque des revendications 1 à 9, où l'agent facilitant le glissement du sang est sélectionné dans le groupe consistant en les composés (A) à (F) suivants et toute combinaison de ceux-ci :

(A) un ester de (A1) un composé ayant un fragment à chaîne hydrocarbonée et 2-4 groupements hydroxyles substituant des hydrogènes sur le fragment à chaîne hydrocarbonée et de (A2) un composé ayant un fragment à chaîne hydrocarbonée et 1 groupement carboxyle substituant un hydrogène sur le fragment à chaîne hydrocarbonée ;
(B) un ester de (B1) un composé ayant un fragment à chaîne hydrocarbonée et 2-4 groupements hydroxyles substituant des hydrogènes sur le fragment à chaîne hydrocarbonée et de (B2) un composé ayant un fragment à chaîne hydrocarbonée et 1 groupement hydroxyle substituant un hydrogène sur le fragment à chaîne hydrocarbonée ;
(C) un ester de (C1) un acide carboxylique, un hydroxyacide, un alcoxyacide ou un oxoacide comprenant un fragment à chaîne hydrocarbonée et 2-4 groupements carboxyles substituant des hydrogènes sur le fragment à chaîne hydrocarbonée et de (C2) un composé ayant un fragment à chaîne hydrocarbonée et 1 groupement hydroxyle substituant un hydrogène sur le fragment à chaîne hydrocarbonée ;
(D) un composé ayant un fragment à chaîne hydrocarbonée et une liaison sélectionnée dans le groupe consistant en une liaison éther (-O-), une liaison carbonyle (-CO-), une liaison ester (-COO-) et une liaison carbonate (-OCOO-) insérées entre une simple liaison C-C du fragment à chaîne hydrocarbonée ;
(E) un poly(oxyalkylène en $C_3$-$C_6$ glycol) ou un ester alkylique ou éther alkylique de celui-ci ; et
(F) une chaîne hydrocarbonée.

11. Article absorbant selon l'une quelconque des revendications 1 à 10, où l'agent facilitant le glissement du sang est sélectionné dans le groupe consistant en ($a_1$) un ester d'un tétraol à chaîne hydrocarbonée et d'au moins un acide gras, ($a_2$) un ester d'un triol à chaîne hydrocarbonée et d'au moins un acide gras, ($a_3$) un ester d'un diol à chaîne

hydrocarbonée et d'au moins un acide gras, ($b_1$) un éther d'un tétraol à chaîne hydrocarbonée et d'au moins un alcool aliphatique monohydrique, ($b_2$) un éther d'un triol à chaîne hydrocarbonée et d'au moins un alcool aliphatique monohydrique, ($b_3$) un éther d'un diol à chaîne hydrocarbonée et d'au moins un alcool aliphatique monohydrique, ($c_1$) un ester d'un acide tétracarboxylique, hydroxyacide, alcoxyacide ou oxoacide à chaîne hydrocarbonée ayant 4 groupements carboxyles et d'au moins un alcool aliphatique monohydrique, ($c_2$) un ester d'un acide tricarboxylique, hydroxyacide, alcoxyacide ou oxoacide à chaîne hydrocarbonée ayant 3 groupements carboxyles et d'au moins un alcool aliphatique monohydrique, ($c_3$) un ester d'un acide dicarboxylique, hydroxyacide, alcoxyacide ou oxoacide à chaîne hydrocarbonée ayant 2 groupements carboxyles et d'au moins un alcool aliphatique monohydrique, ($d_1$) un éther d'un alcool aliphatique monohydrique et d'un alcool aliphatique monohydrique, ($d_2$) une dialkylcétone, ($d_3$) un ester d'un acide gras et d'un alcool aliphatique monohydrique, ($d_4$) un carbonate de dialkyle, ($e_1$) un poly(oxyalkylène en $C_3$-$C_6$ glycol), ($e_2$) un ester d'un poly(oxyalkylène en $C_3$-$C_6$ glycol) et d'au moins un acide gras, ($e_3$) un éther d'un poly(oxyalkylène en $C_3$-$C_6$ glycol) et d'au moins un alcool aliphatique monohydrique et ($f_1$) une chaîne alcane et toute combinaison de ceux-ci.

**12.** Article absorbant selon l'une quelconque des revendications 1 à 11, dans lequel l'agent facilitant le glissement du sang a une pression de vapeur de 0,00 à 0,01 Pa mesurée sous 1 atmosphère, 40 °C.

FIG. 1

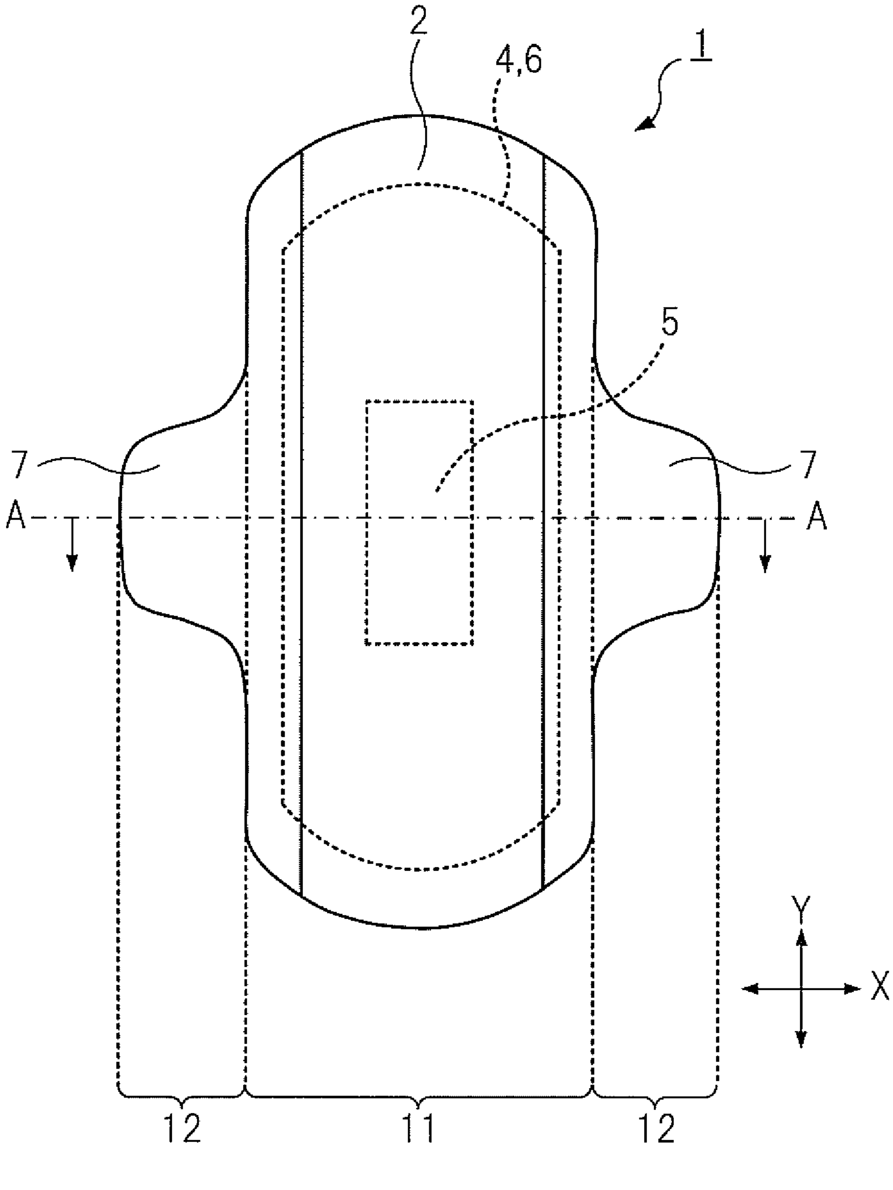

FIG. 2

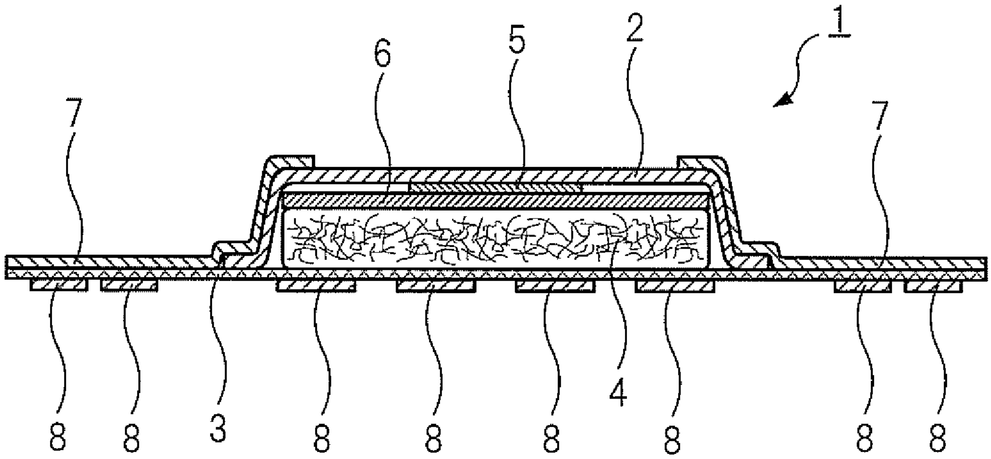

FIG. 3

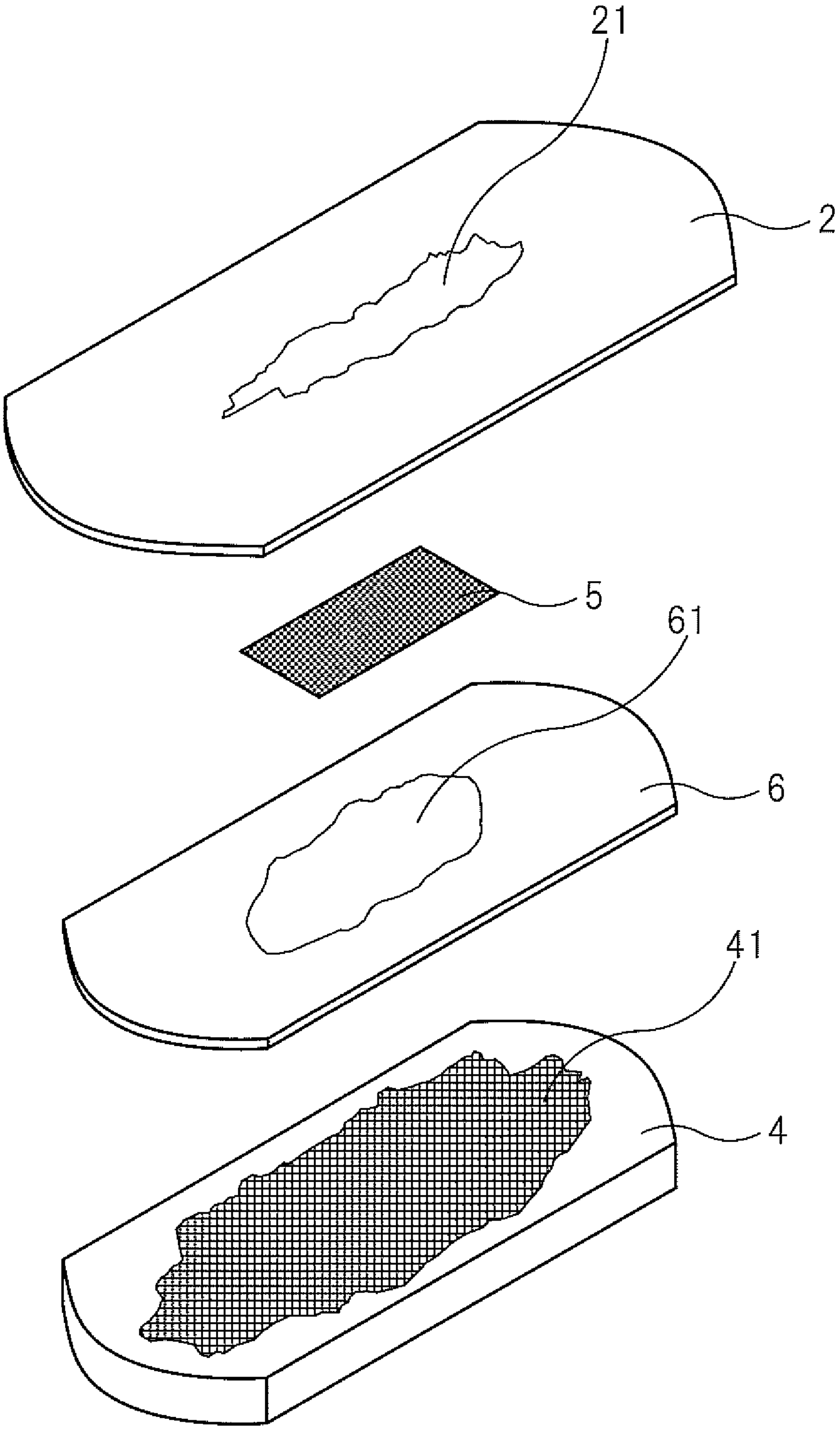

FIG. 4

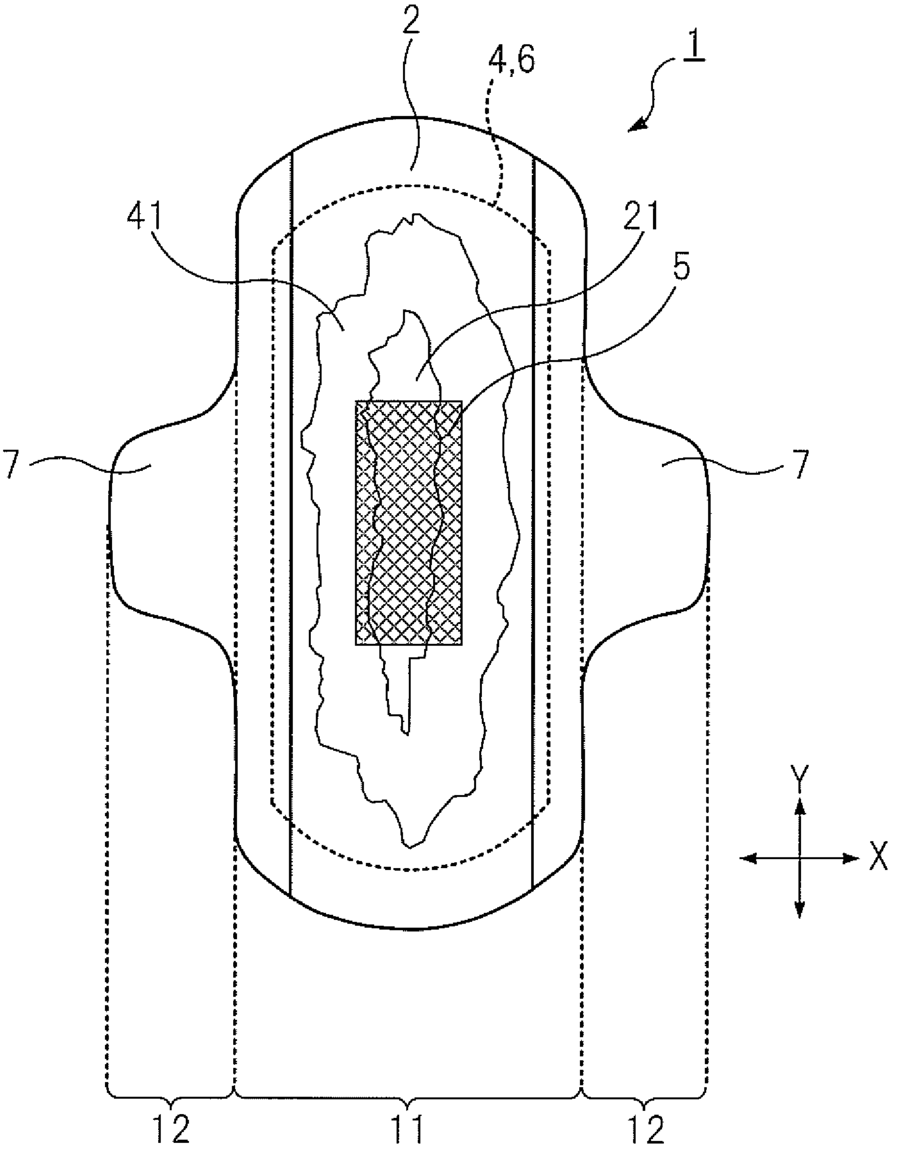

FIG. 5

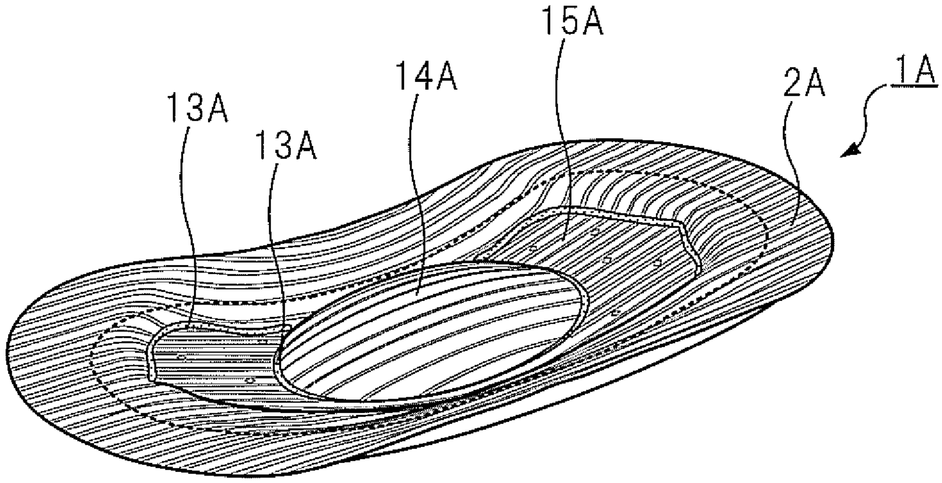


FIG. 6

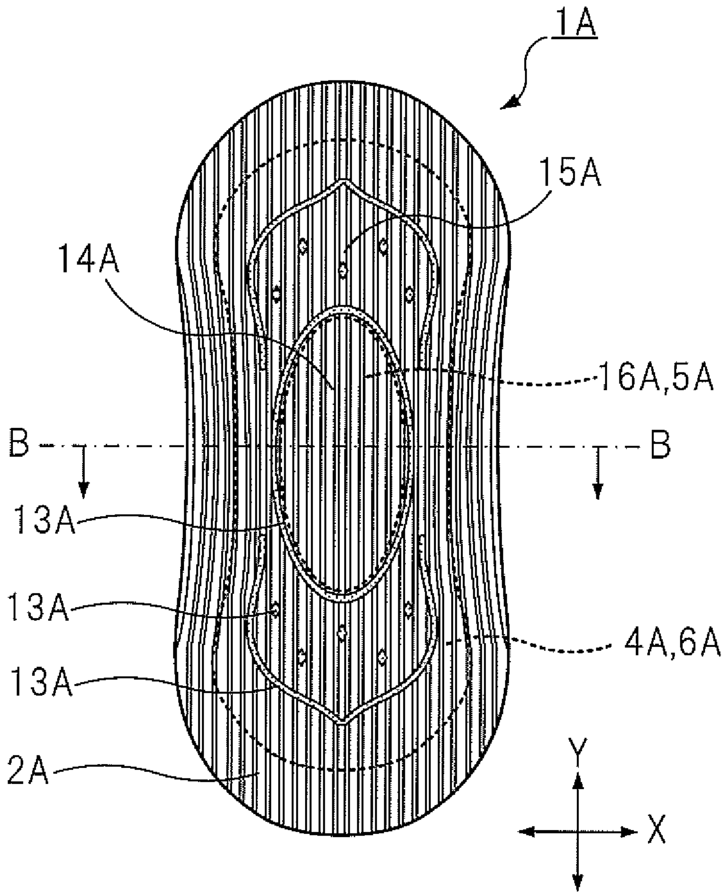

FIG. 7

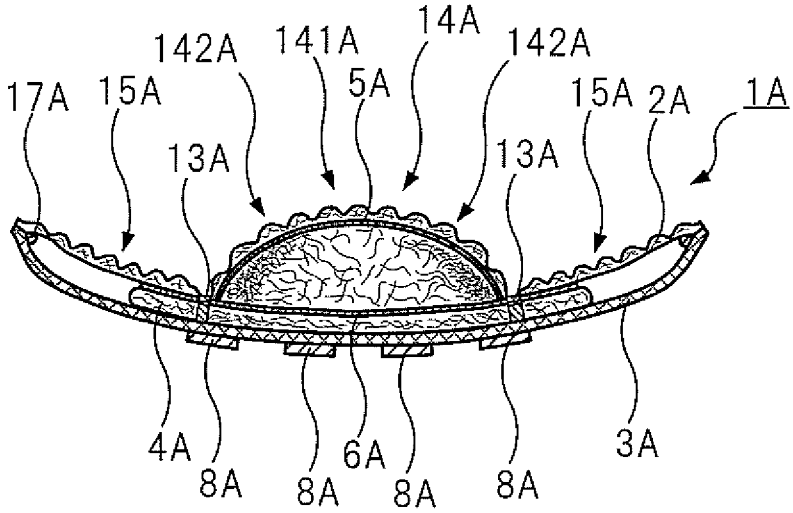
142A
141A
14A
142A
17A
15A
5A
15A
2A
1A
13A
13A
4A
8A
8A
6A
8A
8A
3A

FIG. 8

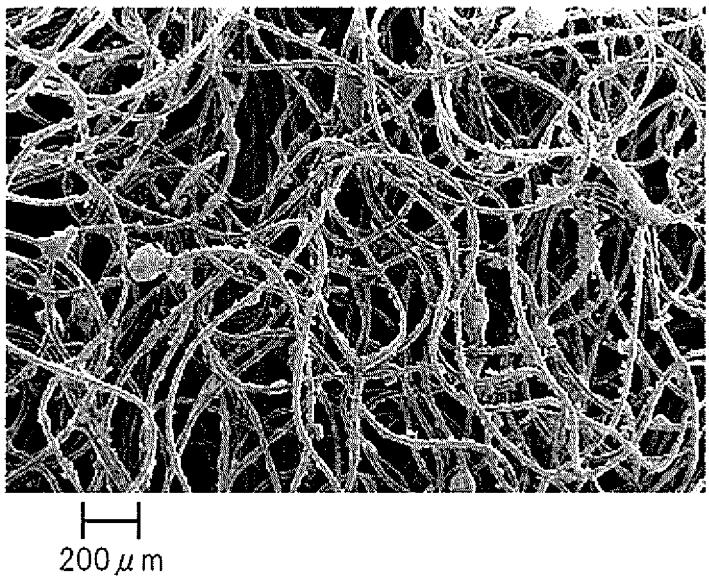
200 μm

FIG. 9

(a)

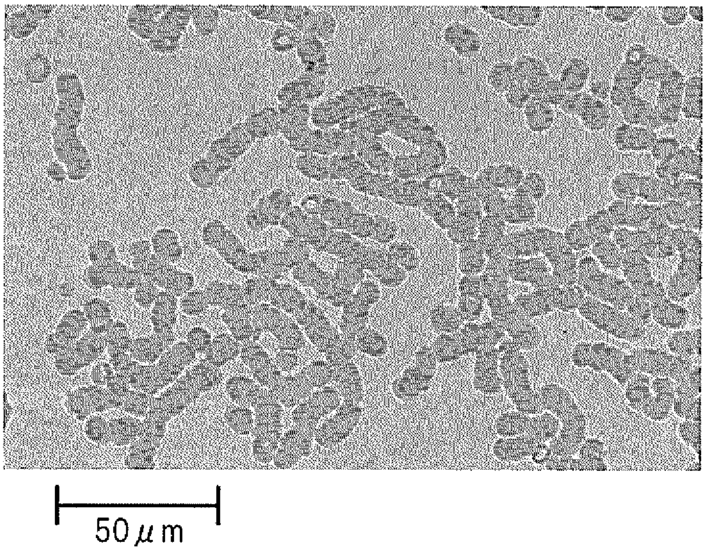



(b)

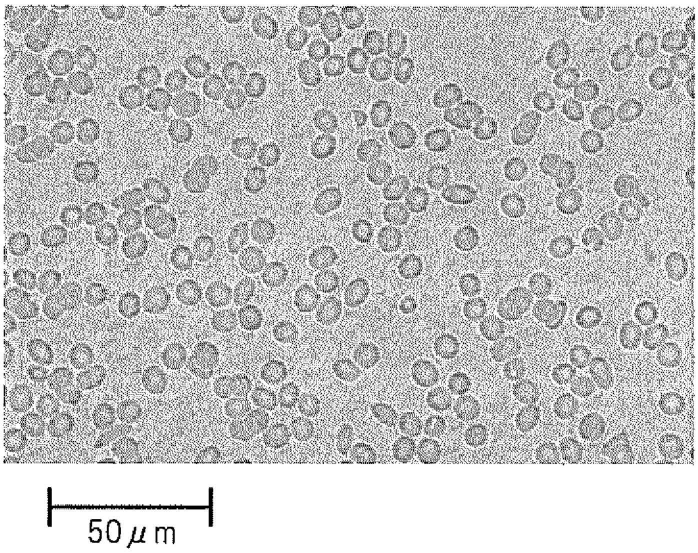

FIG. 10

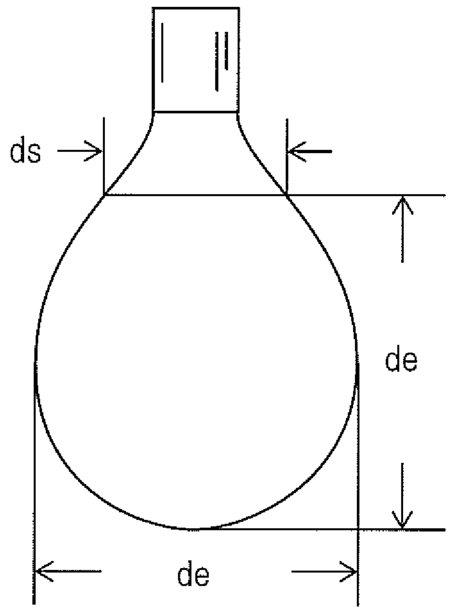

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4266685 B
- JP 2810772 B
- JP 2008025078 A
- JP 2008025079 A
- JP 2011226010 A
- JP 2011226011 A
- JP 2002528174 A
- JP 2008002034 A


### Non-patent literature cited in the description


- **FUJITA A.** Organic compound predictions and organic paradigms. *Kagaku no Ryoiki (Journal of Japanese Chemistry),* 1957, vol. 11 (10), 719-725